# EUROPEAN PATENT APPLICATION

(11) **EP 4 745 124 A1**
(43) Date of publication of application: **20.05.2026**
(21) Application number: 24906574.9
(22) Date of filing: 20.12.2024
(51) Int. Cl.: C07D 209/92, C07D 401/12, C07D 471/08, C07D 487/04, C07D 403/12, C07D 401/14, C07D 403/14, C07D 487/10, C07D 413/14, A61K 31/496

(54) **NAPHTHALIMIDE-BASED CORONAVIRUS PAPAIN-LIKE PROTEASE INHIBITOR**

(30) Priority: 22.12.2023 CN 202311794503
(71) Applicant: Guangzhou National Laboratory, Guangzhou, Guangdong 510005 (CN)
(72) Inventor: CHEN, Hongming, Guangzhou, Guangdong 510005 (CN); SHANG, Jinsai, Guangzhou, Guangdong 510005 (CN); YANG, Qi, Guangzhou, Guangdong 510005 (CN); ZHANG, Guihua, Guangzhou, Guangdong 510005 (CN); HE, Pan, Guangzhou, Guangdong 510005 (CN); ZHOU, Peiqi, Guangzhou, Guangdong 510005 (CN); CHEN, Hua, Guangzhou, Guangdong 510005 (CN); ZHONG, Jingpeng, Guangzhou, Guangdong 510005 (CN); RAN, Ting, Guangzhou, Guangdong 510005 (CN); LU, Yongzhi, Guangzhou, Guangdong 510005 (CN); DAI, Minxian, Guangzhou, Guangdong 510005 (CN); LI, Wenqi, Guangzhou, Guangdong 510005 (CN); CHEN, Xinwen, Guangzhou, Guangdong 510005 (CN)
(74) Representative: Schlich
(86) International application number: PCT/CN2024/141217
(87) International publication number: WO 2025/131104

(57) **Abstract**

The present application relates to the field of pharmaceutical chemistry, and in particular to a naphthalimide-based coronavirus papain-like protease inhibitor as represented by formula (I) and a use thereof.

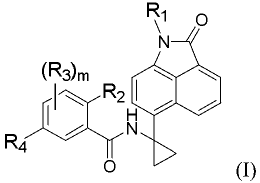

## Description

### TECHNICAL FIELD

The present application pertains to the field of pharmaceutical chemistry, specifically relating to a naphthalimide-based coronavirus papain-like protease inhibitor as shown in formula (I) and use thereof.

### BACKGROUND

Coronavirus, as a type of pathogen threatening human health, belongs to the family Coronaviridae within the order Nidovirales. It is an enveloped, single-stranded, positive-sense RNA virus, divided into three genera: α, β and γ. Coronavirus particles are spherical or irregular in shape, with an envelope, measuring 80-120 nm in size. The 5' end of its genome bears a cap structure and contains 6-10 open reading frames (ORFs) thereafter. The first reading frame, occupying 2/3 of the genome, encodes a replicase, while the other 1/3 of the genome primarily encodes structural proteins, generally including spike (S) protein, envelope (E) protein, membrane (M) protein and nucleocapsid (N) protein. E protein and M protein are primarily involved in the assembly process of the virus, while N protein encapsulates the genome to form a nucleocapsid complex. Coronaviruses primarily mediate viral invasion and determine viral tissue or host tropism by binding to host cell receptors through the Spike glycoprotein (S glycoprotein). They enter cells by recognizing the human host's ACE2 protein, and amino acid variations in the S protein domains can lead to changes in viral species tropism and infection characteristics.

3C-like protease (3CLpro or Mpro) and papain-like protease (PLpro) play crucial roles in coronavirus replication. Among them, PLpro is a key regulatory protein molecule for the formation of the SARS-CoV-2 replicase complex (RC), which is essential for viral genome transcription and replication. As one of the representative non-structural proteins, PLpro counteracts the host's natural immune response and has important biological functions in regulating the replication and proliferation of novel coronavirus and inhibiting the host's immune response. It is also highly conserved evolutionarily, possesses deubiquitination function, and is one of the ideal targets for the development of broad-spectrum anti-coronavirus drugs. Therefore, inhibiting the activity of the viral replication complex through small molecule inhibitors of PLpro protease may help treat novel coronavirus pneumonia.

### SUMMARY OF THE INVENTION

In a first aspect, the present application provides a compound represented by formula (I), or a stereoisomer thereof, a tautomer thereof, a solvate thereof, an isotopically labeled compound thereof, or a pharmaceutically acceptable salt thereof, 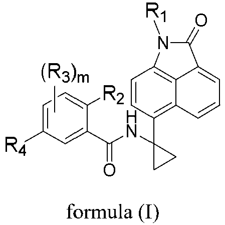 wherein,
R₁ is selected from the group consisting of hydrogen, C₁₋₆ alkyl, C₁₋₆ haloalkyl, -C(=O)-C₁₋₆ alkyl, -C(=O)-C₁₋₆ haloalkyl, -C(=O)-NH-C₁₋₆ alkyl, C₃₋₈ cycloalkyl and 4-8 membered heterocyclyl;
R₂ is selected from the group consisting of hydrogen, halogen, nitro, -CN, -OH, -COOH, -NH₂, -O-C₁₋₆ alkyl, -NH-C₁₋₆ alkyl, -N-(C₁₋₆ alkyl)₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl, -S(=O)₂NH₂, C₁₋₆ alkyl and C₁₋₆ haloalkyl;
R₃ is selected from the group consisting of halogen, nitro, -CN, -OH, -COOH, -NH₂, -O-C₁₋₆ alkyl, -NH-C₁₋₆ alkyl, -N-(C₁₋₆ alkyl)₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl, -S(=O)₂NH₂, C₁₋₆ alkyl and C₁₋₆ haloalkyl;
m is 0, 1, 2, or 3;
R₄ is selected from R₅ and 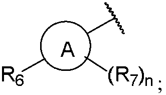
R₅ is selected from C₁₋₈ alkyl and C₁₋₈ haloalkyl, wherein the C₁₋₈ alkyl and C₁₋₈ haloalkyl are optionally substituted with one or more groups selected from the group consisting of -OH, -O-C₁₋₄ alkyl, -NH₂, -NH-C₁₋₄ alkyl and -N(C₁₋₄ alkyl)₂;
ring A is selected from the group consisting of C₃₋₁₂ cycloalkyl, C₄₋₁₂ cycloalkenyl, 4-12 membered heterocyclyl, C₆₋₁₂ aryl and 5-12 membered heteroaryl;
R₆ is selected from the group consisting of hydrogen, carbonyl, halogen, nitro, -CN, -OH, -COOH, -NH₂, C₁₋₆ alkyl, -O-C₁₋₆ alkyl, -NH-C₁₋₆ alkyl, -N-(C₁₋₆ alkyl)₂, -S(=O)₂NH₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl, -C(=NH)NH₂, -C(=NH)NHC(=NH)NH, 4-8 membered heterocyclyl and -NH-(4-8 membered heterocyclyl), wherein the C₁₋₆ alkyl and 4-8 membered heterocyclyl are optionally substituted with one or more groups selected from the group consisting of halogen, nitro, =O, -CN, -OH, -COOH, -NH₂, -C₁₋₆ alkyl, -O-C₁₋₆ alkyl, -NH-C₁₋₆ alkyl, -N-(C₁₋₆ alkyl)₂, -S(=O)₂OH, -S(=O)₂NH₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl and morpholinyl;
R₇ is selected from the group consisting of halogen, nitro, -CN, -OH, -COOH, -NH₂, -O-C₁₋₆ alkyl, -NH-C₁₋₆ alkyl, -N-(C₁₋₆ alkyl)₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl, -S(=O)₂NH₂, C₁₋₆ alkyl and C₁₋₆ haloalkyl, or two R₇ form =O;
or, two R₇, together with the connected atoms, form C₃₋₆ cycloalkyl or 4-7 membered heterocyclyl, wherein the C₃₋₆ cycloalkyl and the 4-7 membered heterocyclyl are optionally substituted with 1, 2, or 3 groups selected from OH and C₁₋₄ alkyl;
or, R₆ and R₇ are joined to form a 4-6 membered heterocyclyl, the 4-6 membered heterocyclyl is optionally substituted with 1, 2, or 3 groups selected from OH and C₁₋₄ alkyl; and
n is 0, 1, 2, 3, 4, 5, 6, or 7.

In some embodiments, the R₄ is

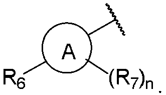

In some embodiments, the R₁ is selected from the group consisting of hydrogen, C₁₋₄ alkyl, C₁₋₄ haloalkyl, -C(=O)-C₁₋₄ alkyl, -C(=O)-C₁₋₄ haloalkyl, C₃₋₆ cycloalkyl and 4-6 membered heterocyclyl.

In some embodiments, the R₁ is selected from hydrogen, C₁₋₄ alkyl and C₃₋₆ cycloalkyl.

In some embodiments, the R₁ is selected from the group consisting of hydrogen, methyl, ethyl, n-propyl, isopropyl, cyclobutyl, cyclopentyl and cyclobutyl.

In some embodiments, the R₁ is selected from the group consisting of hydrogen, methyl, ethyl, isopropyl and cyclopropyl.

In some embodiments, the R₁ is selected from hydrogen and C₁₋₄ alkyl.

In some embodiments, the R₁ is selected from the group consisting of hydrogen, methyl, ethyl and isopropyl.

In some embodiments, the R₂ is selected from the group consisting of hydrogen, fluorine, chlorine, bromine, -CN, -OH, -COOH, -NH₂, -O-C₁₋₄ alkyl, -NH-C₁₋₄ alkyl, -N-(C₁₋₄ alkyl)₂, C₁₋₄ alkyl and C₁₋₄ haloalkyl.

In some embodiments, the R₂ is selected from the group consisting of hydrogen, fluorine, chlorine, -CN, -OH, -COOH, -NH₂, -O-methyl, -NH-methyl, methyl, ethyl, halogenated methyl and halogenated ethyl.

In some embodiments, the R₂ is methyl.

In some embodiments, R₃ is selected from the group consisting of halogen, -CN, -OH, -COOH, -NH₂, -O-C₁₋₄ alkyl, -NH-C₁₋₄ alkyl, -N-(C₁₋₄ alkyl)₂, C₁₋₆ alkyl and C₁₋₆ haloalkyl.

In some embodiments, the R₃ is selected from the group consisting of fluorine, chlorine, bromine, -CN, -OH, -COOH, -NH₂, -O-methyl, -NH-methyl, methyl, ethyl, halogenated methyl and halogenated ethyl.

In some embodiments, the R₅ is selected from C₁₋₆ alkyl and C₁₋₆ haloalkyl, wherein the C₁₋₆ alkyl and C₁₋₆ haloalkyl are optionally substituted with 1, 2, 3, 4, or 5 groups selected from -NH₂, -NH-C₁₋₄ alkyl and -N(C₁₋₄ alkyl)₂.

In some embodiments, the R₅ is selected from C₁₋₆ alkyl, and the C₁₋₆ alkyl is optionally substituted with 1, 2, or 3 groups selected from -NH₂, -NH-methyl and -NH(methyl)₂.

In some embodiments, the R₅ is selected from

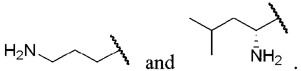

In some embodiments, the ring A is selected from the group consisting of C₄₋₁₀ cycloalkenyl and 4-10 membered heterocyclyl.

In some embodiments, the ring A is selected from the group consisting of C₄₋₁₀ cycloalkenyl and 4-10 membered heterocyclyl, wherein the 4-10 membered heterocyclyl contains 1, 2, or 3 heteroatoms selected from N, O, and S.

In some embodiments, the ring A is selected from the group consisting of C₄₋₈ cycloalkenyl, 4-7 membered nitrogen-containing monocyclic heterocyclyl, 7-10 membered nitrogen-containing bridged heterocyclyl, 7-10 membered nitrogen-containing fused heterocyclyl and 7-10 membered nitrogen-containing spiroheterocyclyl.

In some embodiments, the ring A is selected from the group consisting of 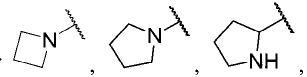 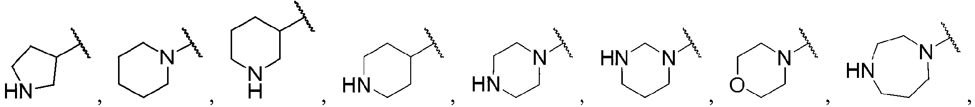 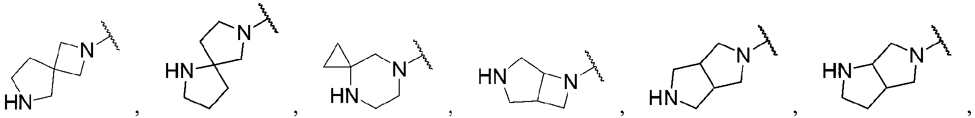 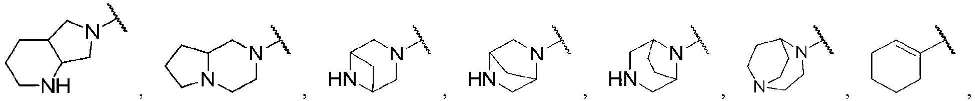 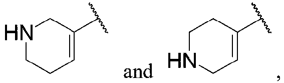 wherein 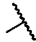 represents the connection site of ring A to phenyl in formula I.

In some embodiments, the ring A is selected from 4-10 membered heterocyclyl.

In some embodiments, the ring A is selected from 4-10 membered heterocyclyl, wherein the 4-10 membered heterocyclyl contains 1 or 2 nitrogen (N) atoms.

In some embodiments, the ring A is selected from 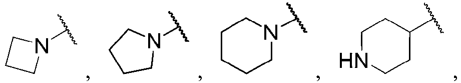 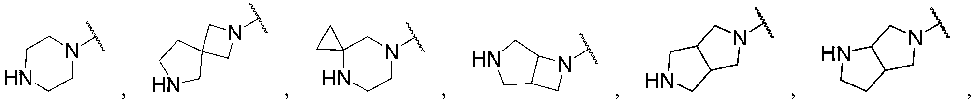 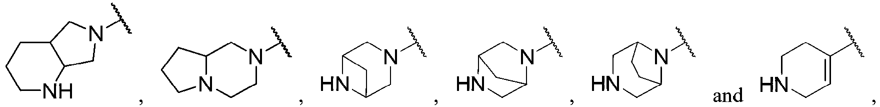 wherein 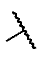 represents the connection site of ring A to phenyl in formula I.

In some embodiments, the R₆ is selected from the group consisting of hydrogen, carbonyl, halogen, nitro, -CN, -OH, -COOH, -NH₂, C₁₋₄ alkyl, -O-C₁₋₄ alkyl, -NH-C₁₋₄ alkyl, -N-(C₁₋₄ alkyl)₂, -S(=O)₂NH₂, -S(=O)₂-C₁₋₄ alkyl, -C(=O)-C₁₋₄ alkyl, -C(=O)-NH-C₁₋₄ alkyl, -NH-C(=O)-C₁₋₄ alkyl, -C(=NH)NH₂, -C(=NH)NHC(=NH)NH, 4-6 membered heterocyclyl and -NH-(4-6 membered heterocyclyl), wherein the C₁₋₄ alkyl and 4-6 membered heterocyclyl are optionally substituted with 1, 2, 3, 4, or 5 groups selected from the group consisting of fluorine, chlorine, bromine, =O, -CN, -OH, -COOH, -NH₂, -C₁₋₄ alkyl, -O-C₁₋₄ alkyl, -NH-C₁₋₄ alkyl, -N-(C₁₋₄ alkyl)₂, -S(=O)₂OH, -S(=O)₂NH₂, -S(=O)₂-C₁₋₄ alkyl and morpholinyl.

In some embodiments, the R₆ is selected from the group consisting of hydrogen, fluorine, chlorine, bromine, -CN, -OH, -NH₂, methyl, ethyl, propyl, butyl, -NH-methyl, -NH-ethyl, -NH-propyl, -NH-butyl, -N(methyl)₂, -N(methyl)-ethyl, -N(methyl)-propyl, -N(ethyl)₂, -N(ethyl)-propyl, -S(=O)₂NH₂, -S(=O)₂-methyl, -S(=O)₂-ethyl, -S(=O)₂-propyl, -C(=NH)NH₂, -C(=NH)NHC(=NH)NH₂, azetidinyl, pyrrolidinyl, piperidinyl, piperazinyl, morpholinyl, thiomorpholinyl, -NH-oxetanyl, -NH-tetrahydrofuranyl and -NH-tetrahydropyranyl, wherein the methyl, ethyl, propyl, butyl, azetidinyl, pyrrolidinyl, piperidinyl, piperazinyl, morpholinyl, thiomorpholinyl, oxetanyl, tetrahydrofuranyl and tetrahydropyranyl groups are optionally substituted with 1, 2, 3, 4, or 5 groups selected from the group consisting of fluorine, chlorine, bromine, =O, -OH, -COOH, -NH₂, methyl, ethyl, propyl, -O-methyl, -O-ethyl, -NH-methyl, -NH-ethyl, -N(methyl)₂, -N(methyl)-ethyl, -N(ethyl)₂, -S(=O)₂OH, -S(=O)₂NH₂, -S(=O)₂-methyl and morpholinyl.

In some embodiments, the R₆ is selected from the group consisting of hydrogen, fluorine, -CN, -OH, -NH₂, methyl, ethyl, propyl, butyl 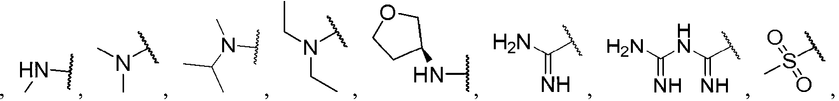 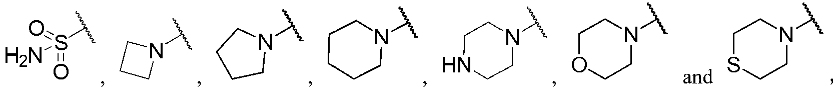 , wherein the methyl, ethyl, propyl, butyl, 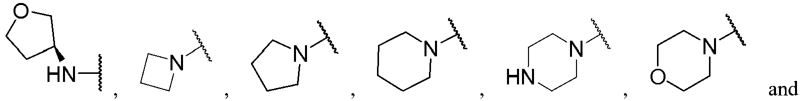 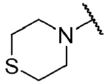 are optionally substituted with 1, 2, or 3 groups selected from the group consisting of fluorine, =O, -OH, -COOH, methyl, -N(CH₃)₂, -S(=O)₂-methyl and morpholinyl.

In some embodiments, the R₆ is selected from the group consisting of hydrogen, fluorine, -CN, -OH, -NH₂, -CH₃, -CH₂CH₃,

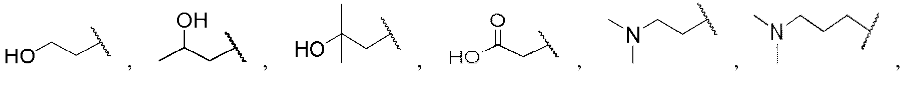

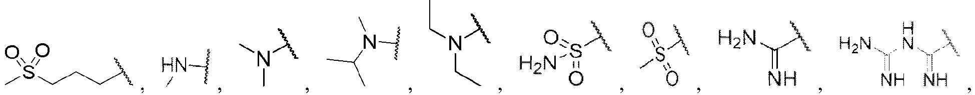

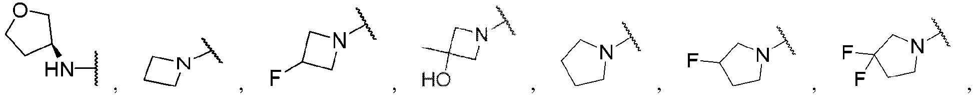

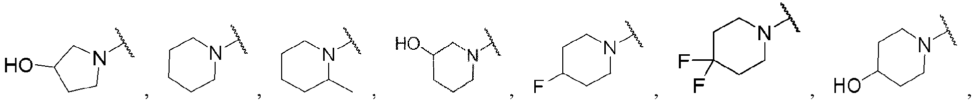

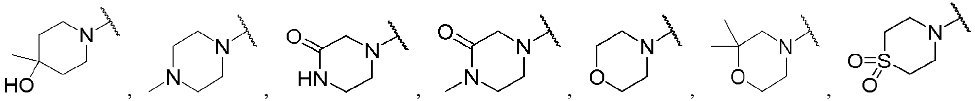

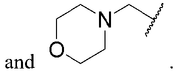

In some embodiments, the R₆ is selected from the group consisting of hydrogen, C₁₋₄ alkyl, -N-(C₁₋₄ alkyl)₂ and 4-6 membered heterocyclyl, wherein the C₁₋₄ alkyl and 4-6 membered heterocyclyl are optionally substituted with 1, 2, or 3 groups selected from halogen, =O, -CN, -OH, -COOH, -NH₂, -N-(C₁₋₄ alkyl)₂, -S(=O)₂-methyl, C₁₋₄ alkyl and morpholinyl.

In some embodiments, the R₆ is selected from the group consisting of hydrogen, C₁₋₄ alkyl, -N-(C₁₋₄ alkyl)₂ and 4-6 membered heterocyclyl, wherein the 4-6 membered heterocyclyl contains 1 or 2 heteroatoms selected from N, O and S, the C₁₋₄ alkyl and 4-6 membered heterocyclyl are optionally substituted with 1, 2, or 3 groups selected from the group consisting of fluorine, =O, -OH, -N(CH₃)₂, -S(=O)₂-CH₃, -CH₃ and morpholinyl.

In some embodiments, the R₆ is selected from the group consisting of hydrogen, -CH₃, -CH₂CH₃,

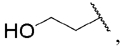

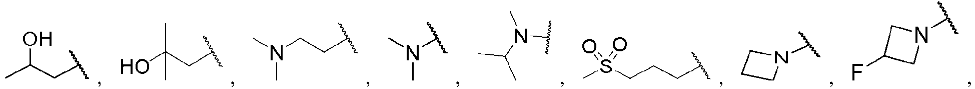

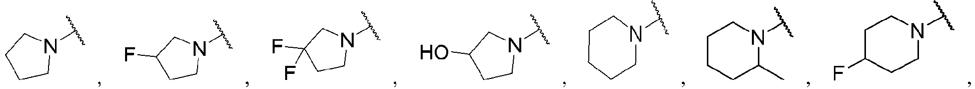

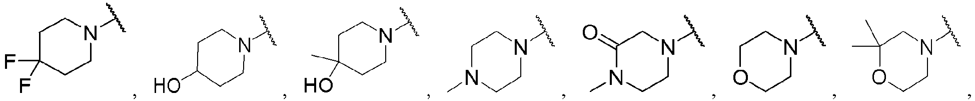

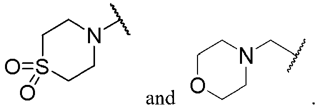

In some embodiments, each of the R₇ is independently selected from the group consisting of halogen, nitro, -CN, -OH, -COOH, -NH₂, -O-C₁₋₄ alkyl, -NH-C₁₋₄ alkyl, -N-(C₁₋₄ alkyl)₂, -C(=O)-C₁₋₄ alkyl, -C(=O)-NH-C₁₋₄ alkyl, -NH-C(=O)-C₁₋₄ alkyl, C₁₋₆ alkyl and C₁₋₄ haloalkyl.

In some embodiments, each of the R₇ is independently selected from the group consisting of fluorine, chlorine, bromine, -CN, -OH, -COOH, -NH₂, -O-methyl, -O-ethyl, -O-propyl, -NH-methyl, -NH-ethyl, -NH-propyl, -N(methyl)₂, -N(methyl)-ethyl, -N(methyl)-propyl, -N(ethyl)₂, methyl, ethyl, propyl, halogenated methyl, halogenated ethyl and halogenated propyl.

In some embodiments, each of the R₇ is independently selected from the group consisting of fluorine, -OH, -NH₂, -COOH and methyl.

In some embodiments, each of the R₇ is independently selected from fluorine and methyl.

In some embodiments, two R₇ form =O.

In some embodiments, the compound is selected from compounds represented by formula (1-1), 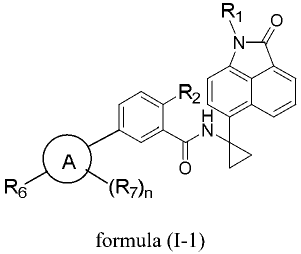 wherein, R₁, R₂, ring A, R₆, R₇ and n are defined as described in any embodiment of the present application.

In some embodiments, the compound is selected from compounds represented by formula (I-1A), 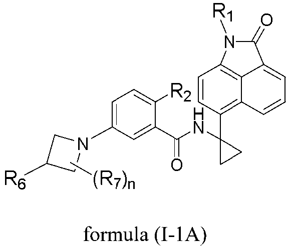 wherein, R₁, R₂, R₆, R₇, and n are defined as described in any embodiment of the present application.

In some embodiments, the R₁ is selected from methyl and ethyl.

In some embodiments, the R₆ is selected from the group consisting of -NH-C₁₋₄ alkyl, -N-(C₁₋₄ alkyl)₂, 4-6 membered heterocyclyl and -NH-(4-6 membered heterocyclyl), wherein the -NH-C₁₋₄ alkyl, -N-(C₁₋₄ alkyl)₂, 4-6 membered heterocyclyl and -NH-(4-6 membered heterocyclyl) are optionally substituted with 1, 2, or 3 groups selected from the group consisting of fluorine, chlorine, =O, -OH, -NH₂, methyl and -N(CH₃)₂.

In some embodiments, the R₆ is selected from the group consisting of -NH-methyl, -NH-ethyl, -NH-propyl, -NH-butyl, -N(methyl)₂, -N(methyl)-ethyl, -N(methyl)-propyl, -N(ethyl)₂, -N(ethyl)-propyl, azetidinyl, pyrrolidinyl, piperidinyl, piperazinyl, morpholinyl, thiomorpholinyl, -NH-oxetanyl, -NH-tetrahydrofuranyl and -NH-tetrahydropyranyl, wherein the methyl, ethyl, propyl, butyl, azetidinyl, pyrrolidinyl, piperidinyl, piperazinyl, morpholinyl, thiomorpholinyl, oxetanyl, tetrahydrofuranyl and tetrahydropyranyl are optionally substituted with 1, 2, or 3 groups selected from the group consisting of fluorine, chlorine, =O, -OH, -NH₂ and methyl.

In some embodiments, the R₆ is selected from the group consisting of

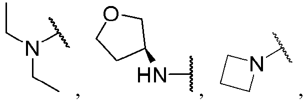

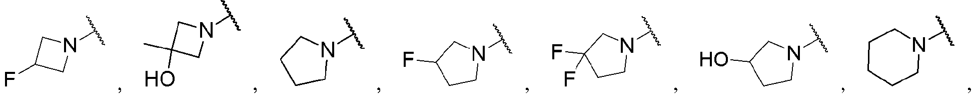

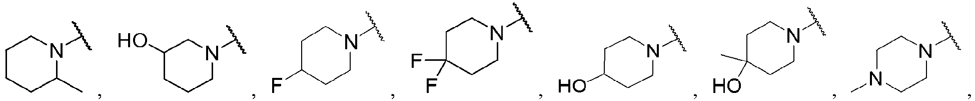

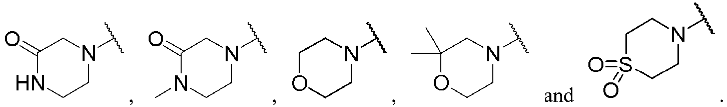

In some embodiments, R₆ and R₇ are joined to form a pyrrolidinyl, and the pyrrolidinyl is optionally substituted with one methyl group.

In some embodiments, the n is 0, 1, 2, or 3.

In some embodiments, the R₆ is selected from 4-6 membered heterocyclyl, and the 4-6 membered heterocyclyl are optionally substituted with 1, 2, or 3 groups selected from the group consisting of halogen, =O, -OH and C₁₋₄ alkyl.

In some embodiments, the R₆ is selected from 4-6 membered heterocyclyl, and the 4-6 membered heterocyclyl are monocyclic ring, the 4-6 membered heterocyclyl contain 1 or 2 heteroatoms selected from N, O and S, and the 4-6 membered heterocyclyl are optionally substituted with 1, 2 or 3 groups selected from the group consisting of fluorine, =O, -OH and methyl.

In some embodiments, the R₆ is selected from the group consisting of

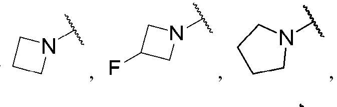

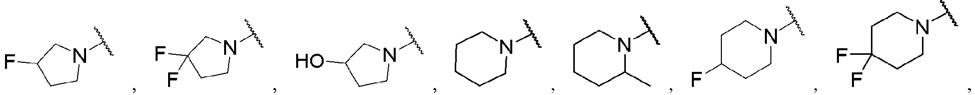

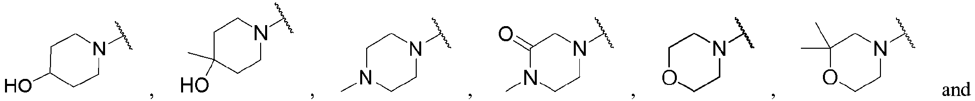

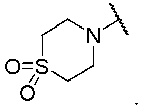

In some embodiments, the compound is selected from compounds represented by formula (I-1A-1), 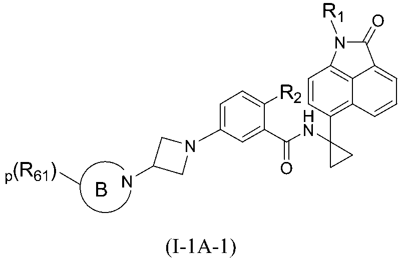 wherein,
R₁ and R₂ are defined as described in any embodiment of the present application;
ring B is selected from 4-6 membered heterocyclyl;
R₆₁ is selected from the group consisting of halogen, =O, -CN, -OH, -COOH, -NH₂, -C₁₋₄ alkyl, -NH-C₁₋₄ alkyl and -N-(C₁₋₄ alkyl)₂; and
p is 0, 1, 2, or 3.

In some embodiments, the R₁ is selected from methyl and ethyl.

In some embodiments, the ring B is selected from the group consisting of azetidinyl, pyrrolidinyl, piperidinyl, piperazinyl, morpholinyl and thiomorpholinyl.

In some embodiments, the R₆₁ is selected from the group consisting of fluorine, chlorine, bromine, =O, -CN, -OH, -COOH, -NH₂, methyl, ethyl and propyl.

In some embodiments, the R₆₁ is selected from the group consisting of fluorine, =O, -OH and methyl.

In some embodiments, the compound is selected from compounds represented by formula (I-1A-2), 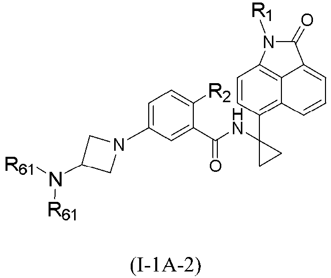 wherein,
R₁ and R₂ are defined as described in any embodiment of the present application, and
R₆₁ is selected from the group consisting of hydrogen, C₁₋₄ alkyl and 4-6 membered heterocyclyl.

In some embodiments, the R₁ is methyl.

In some embodiments, the R₆₁ is selected from the group consisting of hydrogen, methyl, ethyl, propyl, butyl, oxetanyl, tetrahydrofuranyl and tetrahydropyranyl.

In some embodiments, the R₆₁ is selected from the group consisting of hydrogen, -CH₂CH₃ and

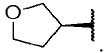

In some embodiments, the compound is selected from compounds represented by formula (I-1B), 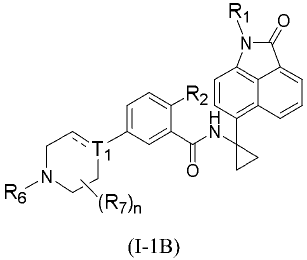 wherein,
T₁ is C, CH or N, 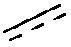 is a single bond or a double bond; and
R₁, R₂, R₆, R₇ and n are defined as described in any embodiment of the present application.

In some embodiments, the T₁ is N, and 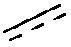 is a single bond.

In some embodiments, the R₆ is selected from the group consisting of hydrogen, -CN, -NH₂, C₁₋₄ alkyl, -C(=NH)NH₂, -C(=NH)NHC(=NH)NH, -S(=O)₂-C₁₋₄ alkyl and -S(=O)₂NH₂, wherein the C₁₋₄ alkyl is optionally substituted with 1, 2, or 3 groups selected from the group consisting of halogen, -OH, -COOH, methyl, -NH(CH₃), -N(CH₃)₂, -N(CH₃)(CH₂CH₃), -N(CH₂CH₃)₂ and -S(=O)₂-CH₃.

In some embodiments, the R₆ is selected from the group consisting of hydrogen, -CN, -NH₂, methyl, ethyl, propyl, butyl, -C(=NH)NH₂, -C(=NH)NHC(=NH)NH, -S(=O)₂-C₁₋₄ alkyl and -S(=O)₂NH₂, wherein the methyl, ethyl, propyl and butyl are optionally substituted with 1, 2, or 3 groups selected from the group consisting of fluorine, -OH, -COOH, methyl, -N(CH₃)₂ and -S(=O)₂-CH₃.

In some embodiments, the R₆ is selected from the group consisting of hydrogen, -CN, -NH₂, -CH₃, -CH₂CH₃,

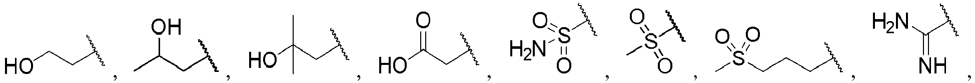

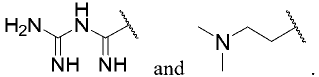

In some embodiments, each of the R₇ is independently selected from -COOH and C₁₋₄ alkyl.

In some embodiments, each of the R₇ is independently selected from -COOH and methyl.

In some embodiments, two R₇ form =O.

In some embodiments, R₆ and R₇ are joined to form a pyrrolidinyl, and the pyrrolidinyl is optionally substituted with one OH.

In some embodiments, the n is 0, 1, 2, 3, or 4.

In some embodiments, the R₆ is selected from hydrogen and C₁₋₄ alkyl, and the C₁₋₄ alkyl is optionally substituted with 1, 2, or 3 groups selected from the group consisting of -OH, -NH(CH₃), -N(CH₃)₂ and -S(=O)₂-CH₃.

In some embodiments, the R₆ is selected from the group consisting of hydrogen, -CH₃, -CH₂CH₃,

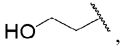

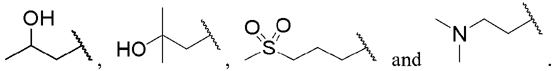

In some embodiments, each of the R₇ is independently selected from C₁₋₄ alkyl.

In some embodiments, each of the R₇ is an independent methyl.

In some embodiments, two R₇, together with the connecting atoms, form cyclopropyl, azetidinyl, pyrrolidinyl, piperidinyl, or homopiperidinyl.

In some embodiments, R₆ and R₇ are joined to form a pyrrolidinyl.

In some embodiments, the n is 0, 1, or 2.

In some embodiments, the compound is selected from compounds represented by formula (I-1C), 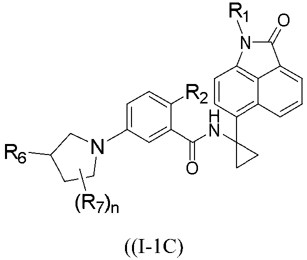 wherein, R₁, R₂, R₆, R₇ and n are defined as described in any embodiment of the present application.

In some embodiments, the R₆ is selected from the group consisting of hydrogen, C₁₋₄ alkyl, -N-(C₁₋₄ alkyl)₂ and 4-6 membered heterocyclyl, wherein the C₁₋₄ alkyl and 4-6 membered heterocyclyl are optionally substituted with 1, 2, or 3 groups selected from the group consisting of halogen, -OH, -COOH and methyl.

In some embodiments, the R₆ is selected from the group consisting of hydrogen, -CH₃,

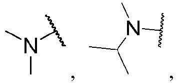

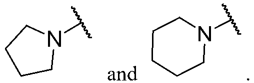

In some embodiments, R₆ and R₇ are joined to form a 5-6 membered heterocyclyl, the 5-6 membered heterocyclyl is optionally substituted with one OH or methyl.

In some embodiments, R₆ and R₇ are joined to form a pyrrolidinyl or a piperidinyl, wherein the pyrrolidinyl or piperidinyl is optionally substituted with one methyl.

In some embodiments, the n is 0, 1, or 2.

In some embodiments, the compound is selected from:

| | | |
|---|---|---|
| 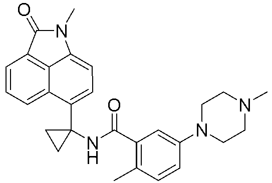 | 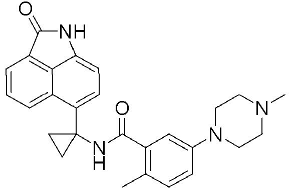 | 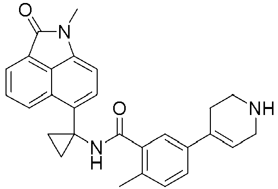 |
| P1 | P2 | P3 |
| 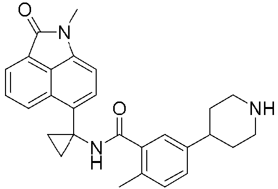 | 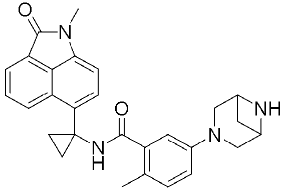 | 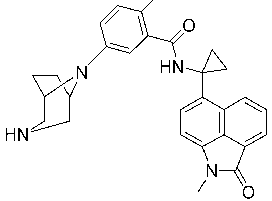 |
| P4 | P5 | P6 |
| 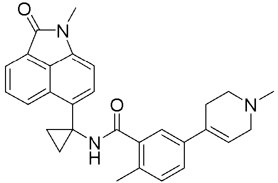 | 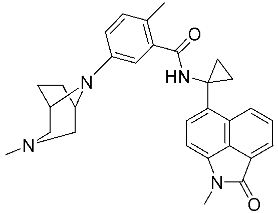 | 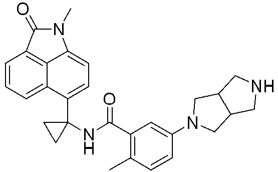 |
| P7 | P8 | P9 |
| 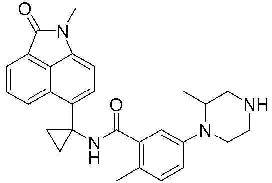 | 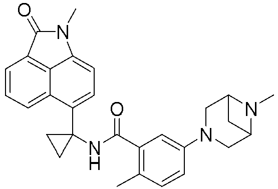 | 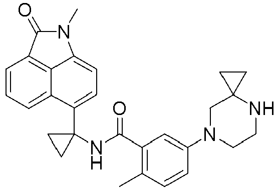 |
| P10 | P11 | P12 |
| 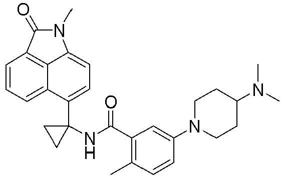 | 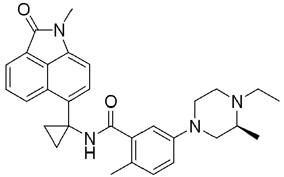 | 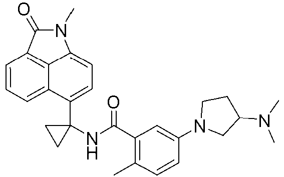 |
| P13 | P14 | P15 |
| 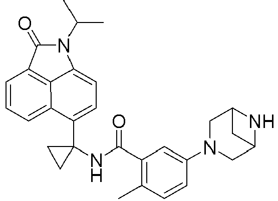 | 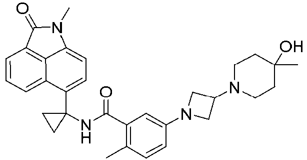 | 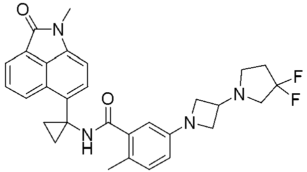 |
| P16 | P17 | P18 |
| 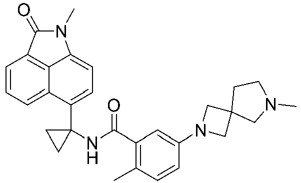 | 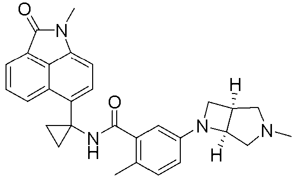 | 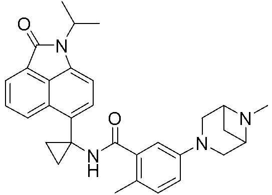 |
| P19 | P20 | P21 |
| 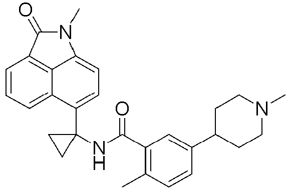 | 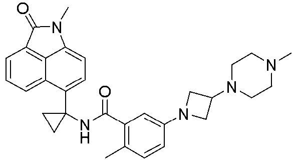 | 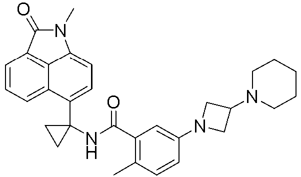 |
| P22 | P23 | P24 |
| 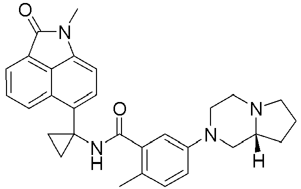 | 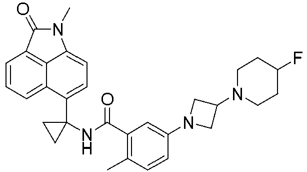 | 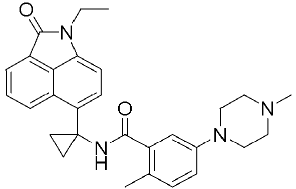 |
| P25 | P26 | P27 |
| 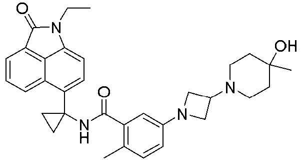 | 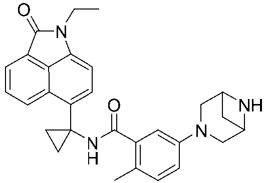 | 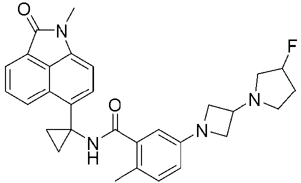 |
| P28 | P29 | P30 |
| 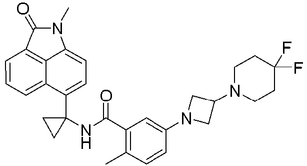 | 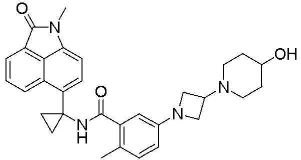 | 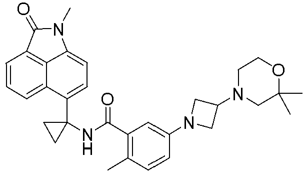 |
| P31 | P32 | P33 |
| 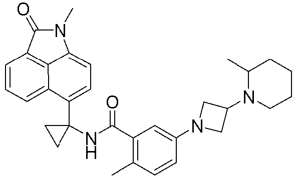 | 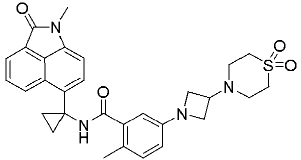 | 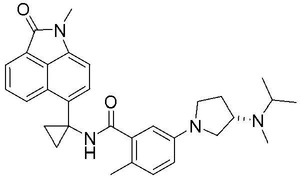 |
| P34 | P35 | P36 |
| 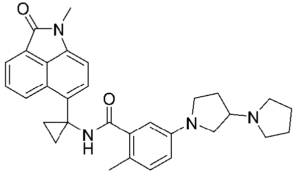 | 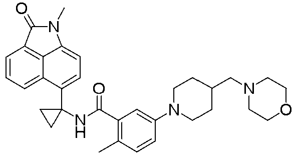 | 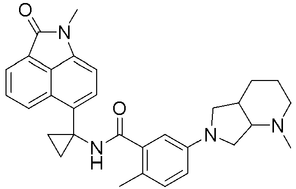 |
| P37 | P38 | P39 |
| 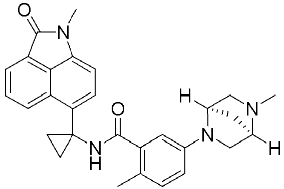 | 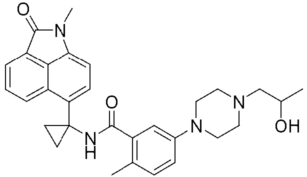 | 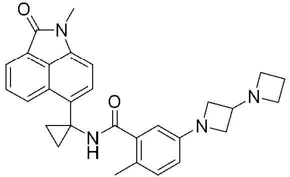 |
| P40 | P41 | P42 |
| 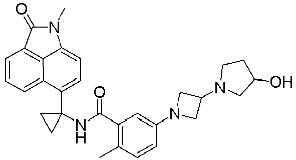 | 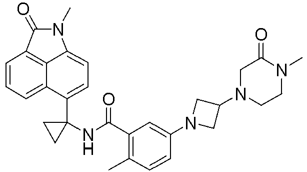 | 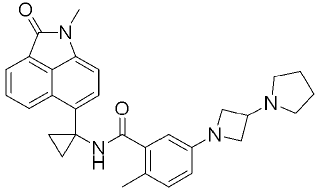 |
| P43 | P44 | P45 |
| 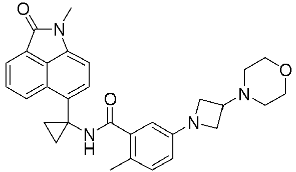 | 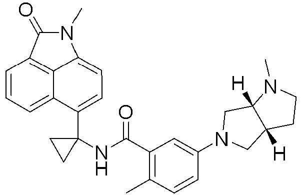 | 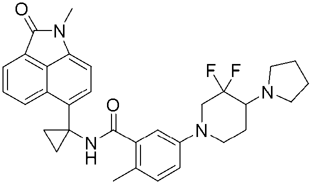 |
| P46 | P47 | P48 |
| 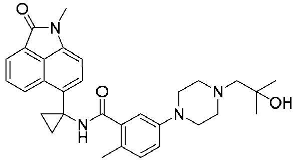 | 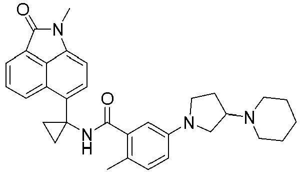 | 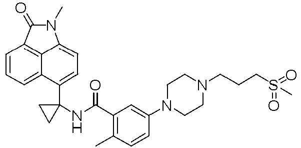 |
| P49 | P50 | P51 |
| 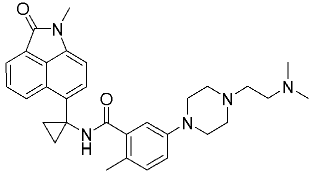 | 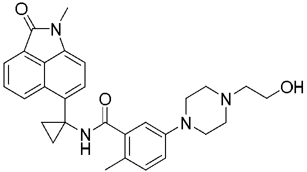 | 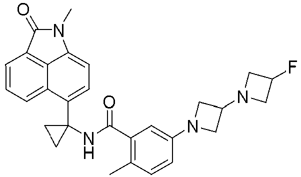 |
| P52 | P53 | P54 |
| 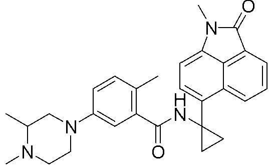 | 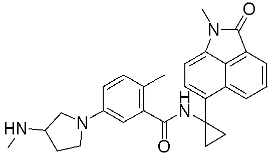 | 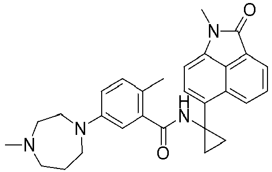 |
| P55 | P56 | P57 |
| 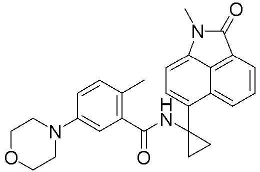 | 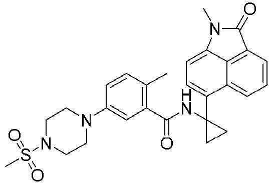 | 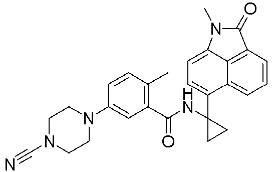 |
| P58 | P59 | P60 |
| 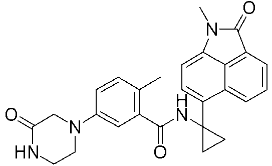 | 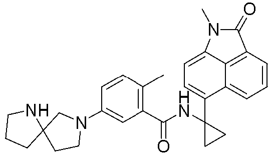 | 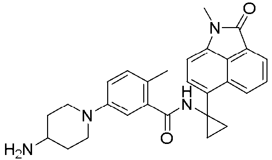 |
| P61 | P62 | P63 |
| 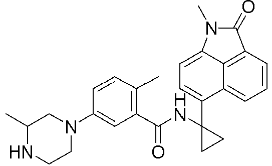 | 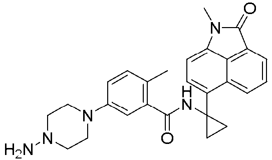 | 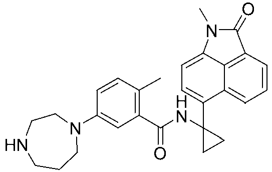 |
| P64 | P65 | P66 |
| 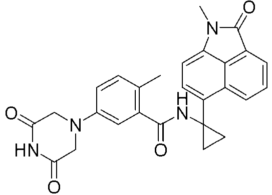 | 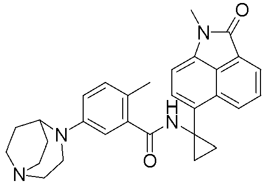 | 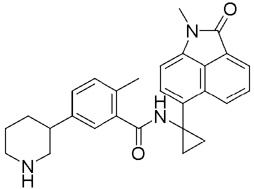 |
| P67 | P68 | P69 |
| 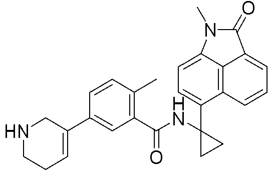 | 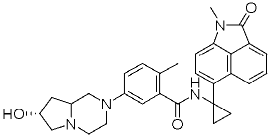 | 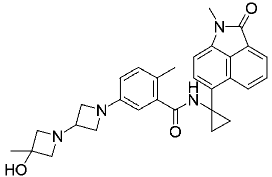 |
| P70 | P71 | P72 |
| 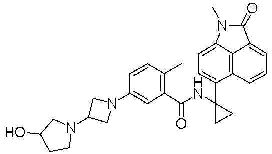 | 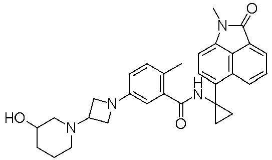 | 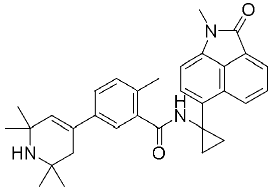 |
| P73 | P74 | P75 |
| 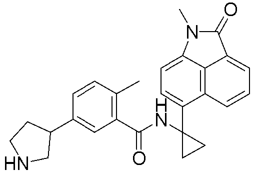 | 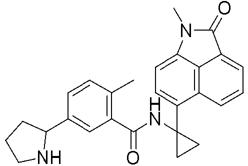 | 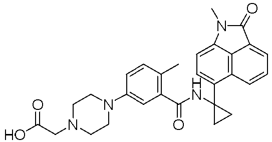 |
| P76 | P77 | P78 |
| 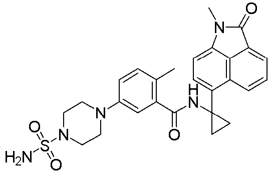 | 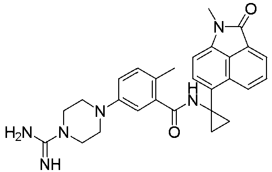 | 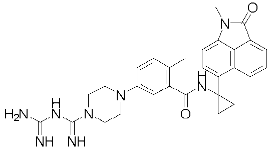 |
| P79 | P80 | P81 |
| 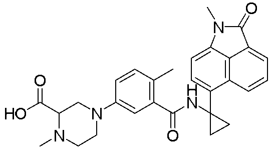 | 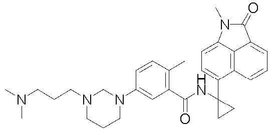 | 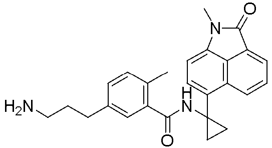 |
| P82 | P83 | P84 |
| 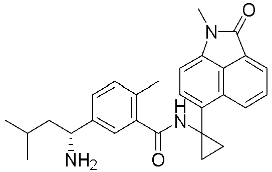 | 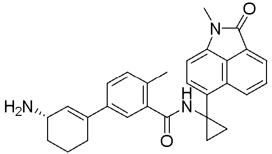 | 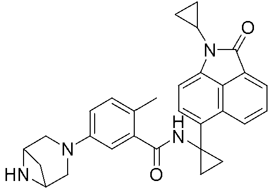 |
| P85 | P86 | P87 |
| 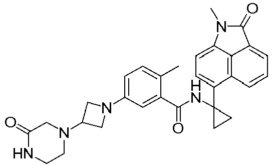 | 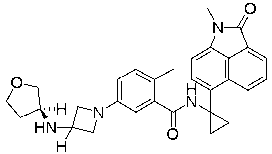 | 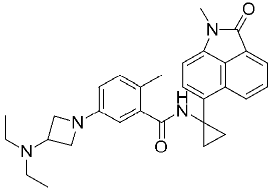 |
| P88 | P89 | P90 |

In a second aspect, the present application provides a pharmaceutical composition comprising at least one compound of the general formula according to the first aspect of the present application, or a stereoisomer thereof, a tautomer thereof, a solvate thereof, an isotopically labeled compound thereof, or a pharmaceutically acceptable salt thereof, as well as one or more pharmaceutical carriers and/or excipients.

In a third aspect, the present application provides a use of the compound according to the first aspect of the present application, or the pharmaceutical composition according to the second aspect of the present application, in the preparation of a medicament for the treatment and/or prevention of a disease or condition, or for the alleviation of the severity of the disease or condition, wherein the disease or condition is caused by a coronavirus, and wherein the medicament comprises a human medicament and a veterinary medicament.

In some embodiments, the disease or condition is selected from the group consisting of respiratory diseases (such as simple infections like fever, cough and sore throat, pneumonia, acute respiratory infection, severe acute respiratory infection (SARI), hypoxic respiratory failure and acute respiratory distress syndrome, COVID-19, severe acute respiratory syndrome (SARS), Middle East respiratory syndrome (MERS)), sepsis, septic shock and feline infectious peritonitis.

In a fourth aspect, the present application provides the compound according to the first aspect of the present application, or the pharmaceutical composition according to the second aspect of the present application, for use in the treatment and/or prevention of a disease or condition, or in the alleviation of the severity of the disease or condition, wherein the disease or condition is caused by a coronavirus.

In some embodiments, the disease or condition is selected from the group consisting of respiratory diseases (such as simple infections like fever, cough and sore throat, pneumonia, acute respiratory infection, severe acute respiratory infection (SARI), hypoxic respiratory failure and acute respiratory distress syndrome, COVID-19, severe acute respiratory syndrome (SARS), Middle East respiratory syndrome (MERS)), sepsis, septic shock and feline infectious peritonitis.

In the fifth aspect, the present application provides a method for treating and/or preventing a disease or condition, or alleviating the severity of the disease or condition, comprising administering to an individual in need an effective amount of the compound according to the first aspect of the present application, or the pharmaceutical composition according to the second aspect of the present application, wherein the disease or condition is caused by a coronavirus, and wherein the medicament comprises a human medicament and a veterinary medicament.

In some embodiments, the disease or condition is selected from the group consisting of respiratory diseases (such as simple infections like fever, cough and sore throat, pneumonia, acute respiratory infection, severe acute respiratory infection (SARI), hypoxic respiratory failure and acute respiratory distress syndrome, COVID-19, severe acute respiratory syndrome (SARS), Middle East respiratory syndrome (MERS)), sepsis, septic shock, and feline infectious peritonitis.

### Term definition

In the present application, unless otherwise stated, the scientific and technical terms used herein have the meanings commonly understood by those skilled in the art. Meanwhile, to facilitate a better understanding of the present application, definitions and explanations of relevant terms are provided below.

In case of any inconsistency between the compound name and its chemical structural formula used in the present application, the chemical structural formula shall prevail.

As used in the present application, the term "stereoisomer" refers to an isomer formed by at least one asymmetric center. In compounds with one or more (e.g., 1, 2, 3, or 4) asymmetric centers, racemic mixtures, single enantiomers, diastereomeric mixtures, and individual diastereomers can be produced. Specific individual molecules can also exist as geometric isomers (cis/trans).

Similarly, the compounds of the present application may exist as a mixture of two or more structurally distinct forms in rapid equilibrium (commonly referred to as tautomers). Representative examples of tautomers include keto-enol tautomers, phenol-ketone tautomers, nitroso-oxime tautomers, imine-enamine tautomers, etc. It should be understood that the scope of the present application encompasses all such isomers or mixtures thereof in any proportion (e.g., 60%, 65%, 70%, 75%, 80%, 85%, 90%, 95%, 96%, 97%, 98%, 99%).

Unless otherwise stated, the compounds of the present application may exist in the form of stereoisomers, including cis- and trans-isomers, optical isomers (such as R and S enantiomers), diastereomers, geometric isomers, rotational isomers, conformational isomers, atropisomers, and mixtures thereof. The compounds of the present application may exhibit more than one type of isomerism and optionally consist of mixtures thereof, such as racemic mixtures and diastereomer pairs.

The compounds of the present application may exist in the form of solvates (such as hydrates), wherein the compounds of the present application comprise solvents, such as water, methanol, or ethanol, as structural elements of the crystal lattice of the compounds. The amount of the solvent may exist in a stoichiometric or non-stoichiometric ratio.

As used in the present application, the term "isotopically labeled compound" refers to a compound in which one or more atoms are replaced with atoms having the same atomic number but a different atomic mass or mass number than the predominant atomic mass or mass number in nature. Examples of isotopes suitable for incorporation into the compounds of the present application include, but are not limited to, hydrogen isotopes such as ²H and ³H; carbon isotopes such as ¹¹C, ¹³C and ¹⁴C; chlorine isotopes such as ³⁶Cl; fluorine isotopes such as ¹⁸F; iodine isotopes such as ¹²³I and ¹²⁵I; nitrogen isotopes such as ¹³N and ¹⁵N; oxygen isotopes such as ¹⁵O, ¹⁷O and ¹⁸O; and sulfur isotopes such as ³⁵S.

As used in the present application, the term "pharmaceutically acceptable salt" refers to a salt of the compound of the present application that is pharmaceutically acceptable and possesses the pharmacological activity of the parent compound. Such kind of salts include: acid addition salts formed with inorganic acids or organic acids, or coordinated compounds wherein the acidic proton of the parent compound is replaced by a metal ion or coordinated with an organic base.

The pharmaceutically acceptable salts of the compounds of the present application include those derived from suitable inorganic and organic acids and bases. Examples of pharmaceutically acceptable non-toxic acid addition salts are salts formed between amino groups and inorganic acids (such as hydrochloric acid, hydrobromic acid, phosphoric acid, sulfuric acid, and perchloric acid) or organic acids (such as acetic acid, oxalic acid, maleic acid, tartaric acid, citric acid, succinic acid, or malonic acid), or salts formed by other methods used in the art (such as ion exchange). Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecyl sulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methane sulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate, and their analogous salts.

Salts derived from suitable bases include alkali metals, alkaline earth metals, ammonium (for example, N⁺(C1-4 alkyl)₄ salts). Representative alkali metal or alkaline earth metal salts include sodium salts, lithium salts, potassium salts, calcium salts, magnesium salts, and their analogous salts. When appropriate, other pharmaceutically acceptable salts include non-toxic ammonium, quaternary ammonium, and amine cations formed with counter ions such as halide, hydroxide, carboxylate, sulfate, phosphate, nitrate, lower alkyl sulfonate, and aryl sulfonate.

Pharmaceutically acceptable salts are also intended to encompass half-salts, where the ratio of compound to acid is 2:1. Exemplary half-salts are those derived from acids containing two carboxylic acid groups, such as malic acid, fumaric acid, maleic acid, succinic acid, tartaric acid, glutaric acid, oxalic acid, adipic acid, and citric acid. Other exemplary half-salts are those salts derived from diprotonic mineral acids (such as sulfuric acid). Preferred exemplary half-salts include, but are not limited to, hemimaleates, hemifumarates and hemisuccinates.

As used in the present application, the term "optionally substituted" means that the group may be either unsubstituted or substituted by a substituent; for example, "C₁₋₆ alkyl optionally substituted with halogen" indicates that C₁₋₆ alkyl can be unsubstituted or substituted with halogen to yield a haloalkyl group. It should be understood that when the statement "R is selected from C₁₋₆ alkyl, -O-C₁₋₆ alkyl and -NH-C₁₋₆ alkyl, wherein the C₁₋₆ alkyl is optionally substituted with halogen" is mentioned, it indicates that the C₁₋₆ alkyl in C₁₋₆ alkyl, -O-C₁₋₆ alkyl, and -NH-C₁₋₆ alkyl is all optionally substituted with halogen. It should also be understood that -N-(C₁₋₆ alkyl)₂ indicates that the nitrogen atom is attached to two C₁₋₆ alkyl groups, which can be the same or different.

As used in the present application, unless otherwise explicitly stated, the description "each independently selected from" adopted throughout the present application can refer to either the mutual non-influence between specific options expressed by the same or different symbols in different groups, or the mutual non-influence between specific options expressed by the same or different symbols in the same group.

As used in the present application, the term "halogen" refers to fluorine, chlorine, bromine and iodine.

As used in the present application, the term "alkyl" refers to a straight or branched monovalent saturated hydrocarbon group. For example, C₁₋₈ alkyl refers to a group having 1 to 8 carbon atoms, such as 1, 2, 3, 4, 5, 6, 7 or 8 carbon atoms; C₁₋₆ alkyl refers to a group having 1 to 6 carbon atoms, such as 1, 2, 3, 4, 5 or 6 carbon atoms. The C₁₋₈ alkyl includes C₁₋₆ alkyl, C₁₋₄ alkyl, etc. Examples of alkyl include, but are not limited to, methyl, ethyl, propyl, butyl, etc. The propyl includes n-propyl and isopropyl, and the butyl includes n-butyl, isobutyl and tert-butyl.

As used in the present application, the term "halo (halogenated)" means that the group it modifies is substituted by one or more halogen atoms, for example, by 1, 2, 3, 4, 5 or 6 halogen atoms. For example, "C₁-₆ haloalkyl" refers to C₁-₆ alkyl as previously defined, which is substituted by one or more halogen atoms, examples include, but are not limited to, CF₃, CHF₂ or CF₂CF₃.

As used in the present application, the term "cycloalkyl" refers to a saturated cyclic hydrocarbon group consisting of carbon and hydrogen atoms. For example, C₃₋₁₂ cycloalkyl has 3 to 12 carbon atoms, such as 3, 4, 5, 6, 7, 8, 9, 10, 11 or 12 carbon atoms. The C₃₋₁₂ cycloalkyl includes C₃₋₈ cycloalkyl, C₃₋₆ cycloalkyl, C₆₋₁₂ cycloalkyl, C₆₋₁₀ cycloalkyl, C₆₋₈ cycloalkyl, etc. The cycloalkyl includes monocyclic, bicyclic, or polycyclic rings, including spirocyclic, fused cyclic, or bridged rings. Examples include, but are not limited to, cyclohexyl, cycloheptyl, adamantyl, etc.

As used in the present application, the term "cycloalkenyl" refers to a partially unsaturated cyclic hydrocarbon group consisting of carbon and hydrogen atoms. For example, C₄₋₁₂ cycloalkenyl has 4 to 12 carbon atoms, such as 4, 5, 6, 7, 8, 9, 10, 11 or 12 carbon atoms, and the C₄₋₁₂ cycloalkenyl includes C₄₋₈ cycloalkenyl, C₆₋₁₂ cycloalkenyl, C₆₋₁₀ cycloalkenyl, C₆₋₈ cycloalkenyl, etc. The cycloalkenyl includes monocyclic, bicyclic, or polycyclic rings, including spirocyclic, fused cyclic, or bridged rings. Examples include, but are not limited to, cyclohexenyl, hexahydronaphthyl, etc.

As used in the present application, the term "heterocyclyl" refers to a saturated or partially unsaturated monovalent cyclic group containing heteroatoms, wherein 1, 2, 3, or 4 ring atoms are heteroatoms and the rest are carbon atoms; preferably, the heteroatoms are selected from N, O or S, wherein nitrogen atoms are optionally quaternized, and nitrogen and sulfur heteroatoms may be optionally oxidized. For example, a 5-12 membered heterocyclyl refers to a group consisting of 5-12 ring atoms, the 5-12 membered heterocyclyl includes 5-10 membered heterocyclyl, 5-7 membered heterocyclyl, 4-8 membered heterocyclyl, 4-6 membered heterocyclyl, etc. The heterocyclyl include monocyclic, bicyclic, or polycyclic rings, including spirocyclic, fused or bridged rings. Examples include, but are not limited to, oxetanyl, azetanyl, pyrrolidinyl, piperidinyl, piperazinyl, or morpholinyl. The term "nitrogen-containing monocyclic heterocyclyl" refers to a heterocyclyl in which at least one heteroatom is a nitrogen atom and the heterocyclyl is monocyclic. Examples include, but are not limited to, piperidinyl. The term "nitrogen-containing bridged heterocyclyl" refers to a heterocyclyl in which at least one heteroatom is a nitrogen atom and the heterocyclyl is bridged. Examples include, but are not limited to 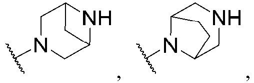 etc. The term "nitrogen-containing fused heterocyclyl" refers to a heterocyclyl in which at least one heteroatom is a nitrogen atom and the heterocyclyl is fused. Examples include, but are not limited to 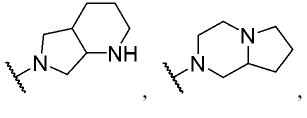 etc. The term "nitrogen-containing spiroheterocyclyl" refers to a heterocyclyl in which at least one heteroatom is a nitrogen atom and the heterocyclyl is fused. Examples include, but are not limited to 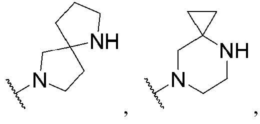 etc.

As used in the present application, the term "partially unsaturated" refers to a ring system that is neither saturated (i.e., does not contain double bonds) nor fully unsaturated (i.e., contains the maximum possible number of double bonds). In other words, a partially unsaturated ring system contains at least one double bond, but not the maximum possible number of double bonds.

As used in the present application, the term "heteroaryl" refers to unsaturated cyclic groups with a conjugated π-electron system, wherein 1, 2, 3 or 4 ring atoms are heteroatoms and the rest are carbon atoms; preferably, the heteroatoms are selected from N, O or S, wherein nitrogen atoms are optionally quaternized, and nitrogen and sulfur heteroatoms may be optionally oxidized. For example, 5-10 membered heteroaryl consists of 5 to 10 (such as 5, 6, 7, 8, 9 or 10) ring atoms, including 5-9 membered heteroaryl, 9-10 membered heteroaryl, and 5-6 membered heteroaryl, etc. The heteroaryl includes monocyclic and polycyclic rings, examples of which include but are not limited to imidazolyl, pyridinyl, quinolinyl, or isoquinolinyl.

As used in the present application, the term "pharmaceutical carrier and/or excipient" refers to a carrier and/or excipient that is pharmacologically and/or physiologically compatible with the subject and the active ingredient, and is well-known in the art (see, for example, Remington's Pharmaceutical Sciences. Edited by Gennaro AR, 19th ed. Pennsylvania: Mack Publishing Company, 1995). Pharmaceutical carriers and/or excipients include, but are not limited to: pH adjusters, surfactants, ionic strength enhancers, diluents, agents maintaining osmotic pressure, agents delaying absorption, preservatives, and stabilizers. For example, pH adjusters include, but are not limited to, phosphate buffer solutions. Surfactants include, but are not limited to, cationic, anionic, or nonionic surfactants, such as Tween-80. Ionic strength enhancers include, but are not limited to, sodium chloride. Preservatives include, but are not limited to, various antibacterial and antifungal agents, such as parabens, chlorobutanol, phenol, sorbic acid, etc. Agents maintaining osmotic pressure include, but are not limited to, sugars, NaCl, and their analogs. Agents delaying absorption include, but are not limited to, monostearate salt and gelatin. Diluents include, but are not limited to, water, aqueous buffers (such as buffered saline), alcohols, and polyols (such as glycerol), etc. Preservatives include, but are not limited to, various antibacterial and antifungal agents, such as thimerosal, 2-phenoxyethanol, parabens, chlorobutanol, phenol, sorbic acid, etc. Stabilizers have meanings commonly understood by those skilled in the art, and are capable of stabilizing the desired activity of the active ingredient in the drug, including but not limited to sodium glutamate, gelatin, SPGA, saccharides (such as sorbitol, mannitol, starch, sucrose, lactose, dextran, or glucose), amino acids (such as glutamic acid, glycine), proteins (such as dried whey, albumin, or casein) or their degradation products (such as lactalbumin hydrolysate), etc.

As used in the present application, the term "effective amount" refers to the quantity of a compound that, when administered, can alleviate one or more symptoms of the condition being treated to some extent.

As used in the present application, the term "treatment" refers to the purpose of alleviating, reducing, improving, or eliminating the targeted disease state or condition. If a subject receives a therapeutic amount of the papain-like protease inhibitor or its racemate, enantiomer, diastereomer, pharmaceutically acceptable salt, or mixture of the aforementioned forms according to the methods described herein, and one or more signs and symptoms of the subject exhibit observable and/or detectable reduction or improvement, the subject is successfully "treated". It should also be understood that the treatment of the disease state or condition mentioned not only includes complete treatment but also includes incomplete treatment that has achieved some biological or medically relevant improvement results.

As used in the present application, the term "prevention" aims to avoid, reduce, prevent, or delay the occurrence of a disease or disease-related symptoms, and such disease or disease-related symptoms have not yet appeared before the administration of the relevant drug. "Prevention" does not necessarily require completely preventing the occurrence of a disease or disease-related symptoms. For example, after the administration of the relevant drug, the risk of a subject developing a specific disease or disease-related symptoms can be reduced, or the severity of later-occurring related symptoms can be alleviated, which can be considered as "preventing" the occurrence or progression of the disease.

### DETAILED DESCRIPTION

The embodiments of the present application will be described in detail below in conjunction with examples. However, those skilled in the art will understand that the following examples are only used to illustrate the present application and should not be considered as limiting the scope of the present application. If specific conditions are not indicated in the examples, conventional conditions or conditions recommended by the manufacturer shall be followed. If the manufacturer of the reagents or instruments used is not indicated, they are all conventional products that can be obtained commercially or prepared by methods known to those skilled in the art. The obtained compounds are as follows:

### Example 1

### Preparation of Compound P1: 2-Methyl-N-(1-(1-methyl-2-oxo-1,2-dihydrobenzo[cd]indol-6-yl)cyclopropyl)-5-(4-methylpiperazin-1-yl)benzamide

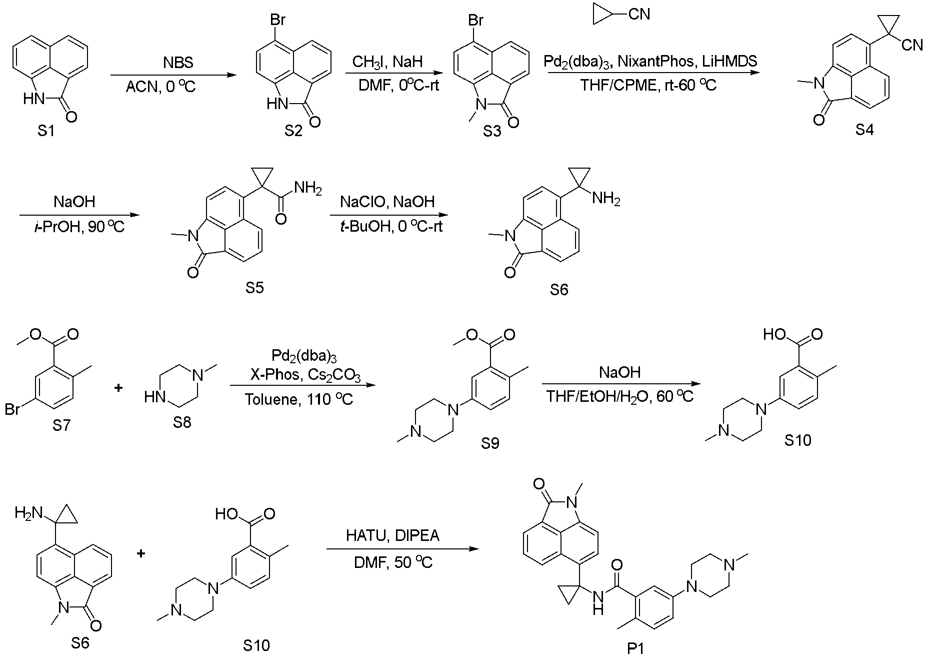

### (1) Preparation of Intermediate S2: 6-bromobenzo[cd]indol-2(1H)-one

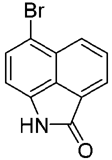

S1 (2000 mg, 11.82 mmol) was placed in a single-necked flask, acetonitrile (ACN, 30 mL) was added, and the mixture was cooled to 0 °C. N-Bromosuccinimide (NBS, 2746 mg, 15.42 mmol) was then added in three portions at 5-minute intervals. After the addition was complete, the mixture was stirred at this temperature for 3.0 hours. After the starting materials had completely reacted, the reaction solution was filtered, the filter cake was washed with water and then dried under vacuum to obtain a yellow solid S2 (2613 mg, 89% yield). MS: m/z [M+1]⁺ = 249.56.

### (2) Preparation of Intermediate S3: 6-bromo-1-methylbenzo[cd]indol-2(1H)-one

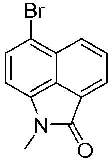

S2 (2613 mg, 10.53 mmol) was placed in a single-necked flask. *N,N*-Dimethylformamide (DMF, 40 mL) was added, and the mixture was cooled to 0 °C. NaH (629 mg, 15.73 mmol) was then added, and the mixture was stirred for 10 minutes. CH₃I (1771 mg, 12.48 mmol) was then added dropwise. The reaction mixture was heated to room temperature and stirred for 1.0 hour. After the starting materials had completely reacted, water was added to the reaction solution, resulting in the precipitation of a yellow solid. The mixture was stirred at room temperature for 20 minutes and then filtered, the filter cake was washed with water, then washed with petroleum ether (PE), and dried under vacuum to obtain a yellow solid S3 (2101 mg, 76% yield). MS: m/z [M+1]⁺ = 263.52.

### (3) Preparation of Intermediate S4: 1-(1-methyl-2-oxo-1,2-dihydrobenzo[cd]indol-6-yl)cyclopropyl-1-carbonitrile

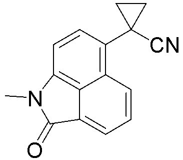

Tris(dibenzylideneacetone)dipalladium (Pd₂(dba)₃, 316 mg, 0.35 mmol) and 4,6-bis(diphenylphosphino)phenoxazine (NixantPhos, 358 mg, 0.65 mmol) were placed in a three-necked flask, followed by the addition of THF (20 mL). After bubbling with nitrogen (N₂) for 30 seconds, the mixture was stirred at room temperature for 5 minutes. Cyclopentyl methyl ether (20 mL), S3 (1628 mg, 6.56 mmol) and cyclopropyl cyanide (662 mg, 9.86 mmol) were added sequentially. Then, lithium bis(trimethylsilyl)amide (LiHMDS, 13 mL, 13.12 mmol, 1.0 M (mol/L) in THF (tetrahydrofuran)) was added dropwise. The mixture was heated to 60 °C and stirred for 1.0 hour. After the starting materials had completely reacted, the reaction solution was cooled to room temperature. A saturated ammonium chloride solution (50 mL) was added. The mixture was extracted three times with ethyl acetate (EA), and the organic phases were combined. The combined organic phases were concentrated under reduced pressure and purified by column chromatography (PE:EA=5:1, 4:1, 3:1) to obtain a yellow solid S4 (788 mg, 51% yield). MS: m/z [M+1]⁺ = 249.36.

### (4) Preparation of Intermediate S5: 1-(1-methyl-2-oxo-1,2-dihydrobenzo[cd]indol-6-yl)cyclopropyl-1-carboxamide

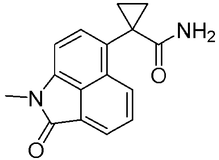

S4 (788 mg, 3.18 mmol) was placed in a single-necked flask. Isopropanol (*i*-PrOH, 8 mL) and NaOH (382 mg, 9.54 mmol) were added. The mixture was heated to 90 °C and stirred overnight. After the starting materials had completely reacted, the reaction solution was cooled to room temperature. Water (10 mL) was added, and the mixture was extracted with dichloromethane (DCM) three times, and the organic phases were combined. The combined organic phases were concentrated under reduced pressure and purified by column chromatography (PE:EA = 1:1, DCM:MeOH = 20:1) to obtain a yellow solid S5 (113 mg, 13% yield). MS: m/z [M+1]⁺ = 267.43.

### (5) Preparation of Intermediate S6: 6-(1-amino-cyclopropyl)-1-methylbenzo[cd]indol-2(1H)-one

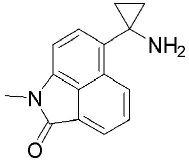

S5 (94 mg, 0.35 mmol) was placed in a single-necked flask. *tert*-Butanol (t-BuOH, 2 mL) was added, and the mixture was cooled to 0 °C. NaClO (0.41 mL, 0.98 mmol) solution and NaOH (3 N, 0.33 mL, 0.98 mmol) solution were then added. The mixture was heated to room temperature and stirred for 3 hours. After the starting materials had completely reacted, the reaction solution was concentrated under reduced pressure to remove the solvent. The resulting mixture was dissolved in DCM, filtered, and the filter cake was washed with DCM. The filtrate was dried over anhydrous sodium sulfate, filtered, and the filter cake was washed with DCM. The filtrate was concentrated under reduced pressure to obtain a pale yellow solid S6 (76 mg, 91% yield). MS: m/z [M+1]⁺ = 239.26.

### (6) Preparation of Intermediate S9: methyl 2-ethyl-5-(4-methylpiperazin-1-yl)benzoate

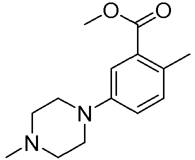

S7 (5000 mg, 21.83 mmol) and S8 (2420 mg, 24.10 mmol) were placed in a sealed tube. Toluene (100 mL), X-Phos (370 mg, 0.78 mmol), Cs₂CO₃ (13100 mg, 40.02 mmol) and Pd₂(dba)₃ (370 mg, 0.40 mmol) were added. The mixture was bubbled with N₂ for 30 seconds, then heated to 110 °C and stirred overnight. After the starting materials had completely reacted, the reaction solution was cooled to room temperature and filtered through diatomite, the filter cake was washed with EA. The resulting filtrate was concentrated under reduced pressure and then purified by column chromatography (DCM:MeOH = 30:1, 20:1) to obtain a reddish-brown oil S9 (5416 mg, 100% yield). MS: m/z [M+1]⁺ = 249.33.

### (7) Preparation of Intermediate S10: 2-methyl-5-(4-methylpiperazin-1-yl)benzoic acid

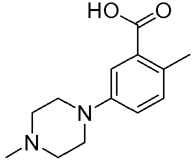

S9 (5416 mg, 21.83 mmol) was placed in a single-necked flask. THF (25 mL) and EtOH (25 mL) were added, followed by the addition of a solution of NaOH (4366 mg, 109.15 mmol) in H₂O (25 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was filtered, and the filter cake was washed with water. The filtrate was extracted with EA (50 mL). The aqueous phase was collected, and water was removed under reduced pressure. The resulting solid mixture was dissolved in a mixture of DCM and MeOH (DCM:MeOH = 10:1, 500 mL), concentrated under reduced pressure, and then re-dissolved in a mixture of DCM and MeOH (DCM:MeOH = 20:1, 500 mL). The resulting solution was dried over anhydrous sodium sulfate, filtered, the filter cake was washed with DCM. The resulting filtrate was concentrated under reduced pressure to obtain a pale yellow solid S10 (5110 mg, 100% yield). MS: m/z [M+1]⁺ = 235.28.

### (8) Preparation of P1: 2-methyl-N-(1-(1-methyl-2-oxo-1,2-dihydrobenzo[cd]indol-6-yl)cyclopropyl)-5-(4-methylpiperazin-1-yl)benzamide

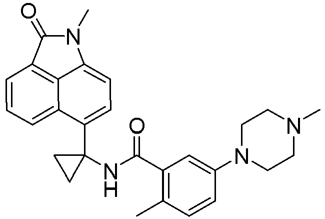

S6 (40 mg, 0.17 mmol) and S10 (47 mg, 0.20 mmol) were placed in a single-necked flask. N,N-Dimethylformamide (DMF, 1.5 mL), N,N-diisopropylethylamine (DIPEA, 71 mg, 0.55 mmol) and 2-(7-azabenzotriazol-1-yl)-N,N,N',N'-tetramethyluronium hexafluorophosphate (HATU, 86 mg, 0.23 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted with DCM three times. The organic phases were combined. The combined organic phases were concentrated under reduced pressure and then purified by column chromatography (PE:EA = 1:1, DCM:MeOH = 20:1) to obtain a bright yellow solid P1 (62 mg, 20% yield). ¹H NMR (600 MHz, DMSO-*d₆*) δ 9.06 (s, 1H), 8.81 (d, *J =* 8.2 Hz, 1H), 8.04 (d, *J =* 6.9 Hz, 1H), 7.83 (dd, *J =* 8.3, 7.0 Hz, 1H), 7.73 (d, *J =* 7.3 Hz, 1H), 7.08 (d, *J* = 7.3 Hz, 1H), 6.96 (d, *J =* 8.4 Hz, 1H), 6.84 (dd, *J =* 8.4, 2.7 Hz, 1H), 6.59 (d, *J =* 2.7 Hz, 1H), 3.36 (s, 3H), 3.03 (t, *J =* 5.0 Hz, 4H), 2.27 (s, 3H), 1.93 (s, 3H), 1.36-1.28 (m, 3H), 1.24 (d, *J =* 9.3 Hz, 3H), 1.20-1.17 (m, 2H). MS: m/z [M+1]⁺ = 454.37.

### Example 2

### Preparation of Compound P2: 2-Methyl-5-(4-methylpiperazin-1-yl)-N-(1-(2-oxo-1,2-dihydrobenzo[cd]indol-6-yl)cyclopropyl)benzamide

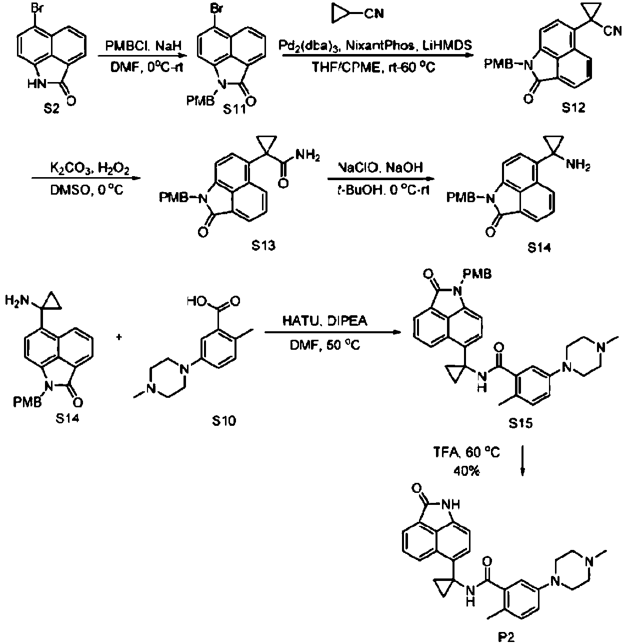

### (1) Preparation of Intermediate S11: 6-bromo-1-(4-methoxybenzyl)benzo[cd]indol-2(1H)-one

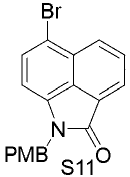

S2 (898 mg, 3.62 mmol) was placed in a single-necked flask, DMF (10 mL) was added, and the mixture was cooled to 0 °C. NaH (290 mg, 7.24 mmol) was added. The mixture was stirred for 10 min. p-Methoxybenzyl chloride (850 mg, 5.43 mmol) was added dropwise. The mixture was heated to room temperature and stirred for 1.0 h. After the starting materials had completely reacted, water was added to the reaction solution, resulting in the precipitation of a yellow solid. The mixture was stirred at room temperature for 20 min, filtered, and the filter cake was washed with water, then washed with PE, and dried to obtain a yellow solid S11 (905 mg, 68% yield). MS: m/z [M+1]⁺ = 369.27.

### (2) Preparation of Intermediate S12: 1-(1-(4-methoxybenzyl)-2-oxo-1,2-dihydrobenzo[cd]indol-6-yl)cyclopropyl-1-carbonitrile

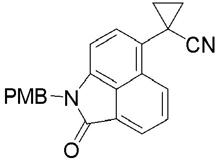

Pd₂(dba)₃ (117 mg, 0.13 mmol) and NixantPhos (133 mg, 0.25 mmol) were placed in a three-necked flask, followed by the addition of tetrahydrofuran (7 mL). The mixture was bubbled with nitrogen for 30 seconds and stirred at room temperature for 5 minutes. Cyclopentyl methyl ether (7 mL), S11 (905 mg, 2.46 mmol), and cyclopropyl cyanide (248 mg, 3.70 mmol) were added sequentially. Then, LiHMDS (5 mL, 4.92 mmol, 1.0 M in THF) was added dropwise. The mixture was heated to 60 °C and stirred for 1.0 hour. After the starting materials had completely reacted, the reaction solution was cooled to room temperature. A saturated ammonium chloride solution (30 mL) was added. The mixture was extracted three times with EA, and the organic phases were combined. The combined organic phases were concentrated under reduced pressure and then purified by column chromatography (PE:EA=8:1, 4:1, 2:1) to obtain a yellow solid S12 (386 mg, 44% yield). MS: m/z [M+1]⁺ = 355.36.

### (3) Preparation of Intermediate S13: 1-(1-(4-methoxybenzyl)-2-oxo-1,2-dihydrobenzo[cd]indol-6-yl)cyclopropyl-1-carboxamide

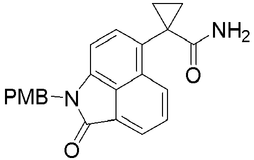

S12 (286 mg, 0.81 mmol) was placed in a single-necked flask, dimethyl sulfoxide (DMSO, 3 mL) and K₂CO₃ (56 mg, 0.41) were added, and the mixture was cooled to 0 °C, H₂O₂ (0.14 mL, 1.22 mmol, 30% in water) was added dropwise. The mixture was stirred for 2.5 h. After the starting materials had completely reacted, water (6 mL) was added to the reaction solution, resulting in the precipitation of a yellow solid. The mixture was filtered, the filter cake was washed with water (2 mL) and dried under vacuum, then purified by column chromatography (PE:EA=1:1, DCM:MeOH=30:1) to obtain a yellow solid S13 (62 mg, 21% yield). MS: m/z [M+1]⁺ = 373.43.

### (4) Preparation of Intermediate S14: 6-(1-amino-cyclopropyl)-1-(4-methoxybenzyl)benzo[cd]indol-2(1H)-one

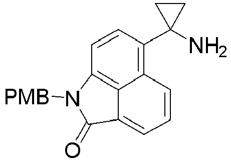

S13 (62 mg, 0.17 mmol) was placed in a single-necked flask, *t*-BuOH (2 mL) was added. The mixture was cooled to 0 °C. NaClO (0.2 mL, 0.48 mmol) solution and NaOH (3 N, 0.16 mL, 0.48 mmol) solution were added. The mixture was heated to room temperature and stirred for 3 hours. After the starting materials had completely reacted, the reaction solution was concentrated under reduced pressure to remove the solvent. DCM (300 mL) was added to the resulting mixture, dried over anhydrous sodium sulfate, filtered, and the filter cake was washed with DCM. The resulting filtrate was concentrated under reduced pressure to obtain a yellow solid S14 (37 mg, 64% yield). MS: m/z [M+1]⁺ = 345.29.

### (5) Preparation of Intermediate S15: N-(1-(1-(4-methoxybenzyl)-2-oxo-1,2-dihydrobenzo[cd]indol-6-yl)cyclopropyl-2-methyl-5-(4-methylpiperazin-1-yl)be nzamide

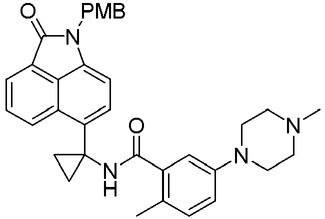

S14 (37 mg, 0.11 mmol) and S10 (31 mg, 0.13 mmol) were placed in a single-necked flask. DMF (1.5 mL), DIPEA (46 mg, 0.36 mmol) and HATU (57 mg, 0.15 mmol) were added. The mixture was heated to 50 °C and stirred for 2 hours. After the starting materials had completely reacted, the reaction solution was cooled to room temperature. Water was added, resulting in the precipitation of a yellow solid. The resulting mixture was stirred at room temperature for 1.0 hour, filtered, the filter cake was washed with water, and dried to obtain a yellow solid S15, which was directly used for the next reaction. MS: m/z [M+1]⁺ = 561.38.

### (6) Preparation of P2: 2-methyl-N-(1-(1-methyl-2-oxo-1,2-dihydrobenzo[cd]indol-6-yl)cyclopropyl)-5-(4-methylpiperazin-1-yl)benzamide

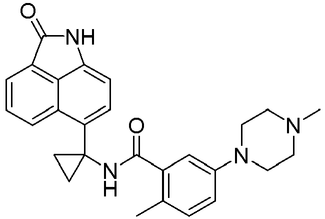

S15 (30 mg, 0.054 mmol) was placed in a single-necked flask. Trifluoroacetic acid (TFA, 1.5 mL) was added. The mixture was heated to 60 °C and stirred overnight. After the starting materials had completely reacted, the reaction solution was concentrated under reduced pressure to remove TFA. A saturated sodium bicarbonate solution (8 mL) was added to the obtained mixture, and the mixture was extracted three times with DCM, the organic phase was collected and concentrated under reduced pressure, and then purified by column chromatography (PE:EA=1:1, DCM:MeOH=20:1) to obtain a bright yellow solid P2 (14 mg, 55% yield). ¹HNMR (600 MHz, DMSO-*d₆*) δ 10.73 (s, 1H), 9.05 (s, 1H), 8.80 (d, J = 8.2 Hz, 1H), 8.00 (d, J = 6.8 Hz, 1H), 7.83 (t, J = 7.4 Hz, 1H), 7.68 (d, J = 7.1 Hz, 1H), 6.99 (d, J = 8.0 Hz, 1H), 6.91 (d, J = 7.2 Hz, 1H), 6.87 (d, J = 7.9 Hz, 1H), 6.62 (s, 1H), 3.12 (s, 3H), 2.83 (s, 3H), 1.95 (s, 3H), 1.34 (d, J = 9.3 Hz, 1H), 1.30 (s, 2H), 1.23 (s, 4H), 1.18 (s, 2H). MS: m/z [M+1]⁺ = 441.22.

### Example 3

### Preparation of Compound P3: 2-Methyl-N-(1-(1-methyl-2-oxo-1,2-dihydrobenzo[cd]indol-6-yl)cyclopropyl)-5-(1,2,3,6-tetrahydropiperidin -4-yl)benzamide

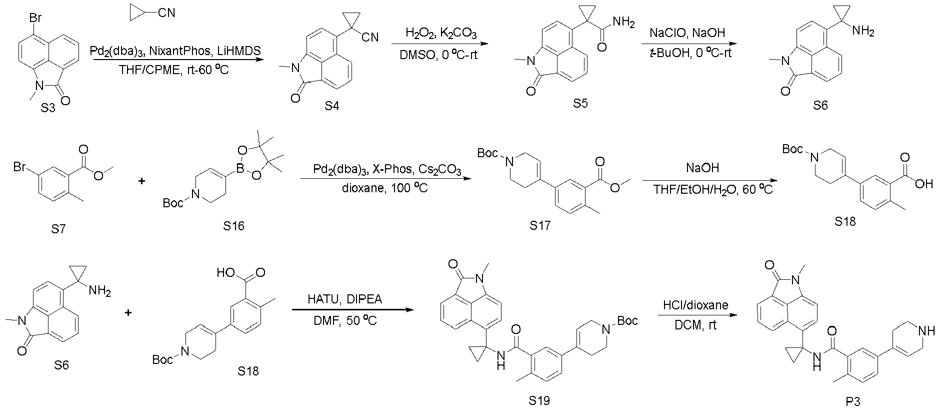

### (1) Preparation of Intermediate S4: 1-(1-methyl-2-oxo-1,2-dihydrobenzo[cd]indol-6-yl)cyclopropyl-1-carbonitrile

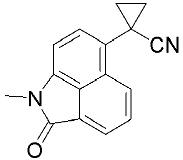

Pd₂(dba)₃ (1102 mg, 1.20 mmol) and NixantPhos (1253 mg, 2.27 mmol) were placed in a three-necked flask, followed by the addition of THF (70 mL). The mixture was bubbled with nitrogen for 30 seconds and stirred at room temperature for 5 minutes. Cyclopentyl methyl ether (70 mL), S3 (6000 mg, 22.89 mmol) and cyclopropyl cyanide (2307 mg, 34.39 mmol) were added sequentially. Then, LiHMDS (46 mL, 45.78 mmol, 1.0 M in THF) was added dropwise. The mixture was heated to 60 °C and stirred overnight. After the starting materials had completely reacted, the reaction solution was cooled to room temperature. A saturated ammonium chloride solution (100 mL) was added. The mixture was extracted three times with EA, and the organic phases were combined. The combined organic phases were concentrated under reduced pressure and then purified by column chromatography (PE:EA=5:1, 4:1, 3:1, 2:1) to obtain a yellow solid S4 (2424 mg, 43% yield). MS: m/z [M+1]⁺ = 249.26.

### (2) Preparation of Intermediate S5: 1-(1-methyl-2-oxo-1,2-dihydrobenzo[cd]indol-6-yl)cyclopropyl-1-carboxamide

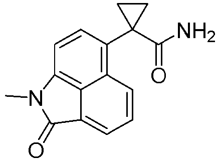

S4 (2424 mg, 9.77 mmol) was placed in a single-necked flask, DMSO (8 mL) and K₂CO₃ (675 mg, 4.89 mmol) were added, and the mixture was cooled to 0 °C, H₂O₂ (4.8 mL, 44.16 mmol, 30% in water) was added dropwise, then heated to room temperature and stirred for 6 hours. After the starting materials had completely reacted, water (30 mL) was added to the reaction solution, resulting in the precipitation of a yellow solid. The mixture was stirred at room temperature for 20 minutes, filtered, and the filter cake was washed with water and then purified by column chromatography (PE:EA=1:1, DCM:MeOH=30:1) to obtain a yellow solid S5 (1124 mg, 43% yield). MS: m/z [M+1]⁺ = 267.43.

### (3) Preparation of Intermediate S6: 6-(1-amino-cyclopropyl)-1-methylbenzo[cd]indol-2(1H)-one

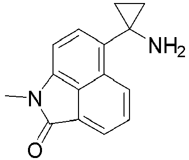

S5 (1124 mg, 4.22 mmol) was placed in a single-necked flask, t-BuOH (15 mL) was added. The mixture was cooled to 0 °C. NaClO (4.90 mL, 11.84 mmol) solution and NaOH (3 N, 3.90 mL, 11.84 mmol) solution were added. The mixture was heated to room temperature and stirred for 3 h. After the starting materials had completely reacted, the reaction solution was concentrated under reduced pressure to remove the solvent. The resulting solid mixture was purified by column chromatography (PE:EA=1:1, DCM:MeOH=30:1) to obtain a yellow solid S6 (590 mg, 60% yield). MS: m/z [M+1]⁺ = 239.26.

### (4) Preparation of Intermediate S17: tert-butyl 4-(3-methoxycarbonyl)-4-methylphenyl)-3,6-dihydropiperidin-1(2H)-carboxylate

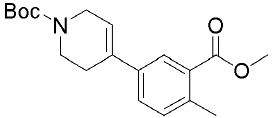

S7 (500 mg, 2.18 mmol) and S16 (675 mg, 2.18 mmol) were placed in a sealed tube, 1,4-dioxane (10 mL), 2-dicyclohexylphosphino-2',4',6'-triisopropylbiphenyl (X-Phos, 42 mg, 0.087 mmol), Cs₂CO₃ (1421 mg, 4.36 mmol) and Pd₂(dba)₃ (40 mg, 0.044 mmol) were added. The mixture was bubbled with nitrogen for 30 seconds, then heated to 100°C and stirred overnight. After the starting materials had completely reacted, the reaction solution was cooled to room temperature and filtered through diatomite, and the filter cake was washed with EA. The resulting filtrate was concentrated under reduced pressure and then purified by column chromatography (PE:EA=30:1) to obtain a pale yellow oil S17 (717 mg, 100% yield). MS: m/z [M+1]⁺ = 332.38.

### (5) Preparation of Intermediate S18: 5-(1-(tert-butoxycarbonyl)-1,2,3,6-tetrahydropiperidin -4-yl)-2-methylbenzoic acid

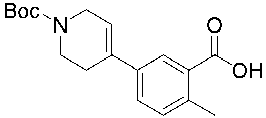

S17 (335 mg, 1.01 mmol) was placed in a single-necked flask. THF (2 mL) and EtOH (2 mL) were added, followed by the addition of a solution of NaOH (202 mg, 5.05 mmol) in H₂O (2 mL). The mixture was heated to 60 °C and stirred for 2 hours. After the starting materials had completely reacted, the reaction solution was concentrated under reduced pressure to remove THF and EtOH, and the pH was then adjusted to 4-5 with a saturated citric acid solution, resulting in the precipitation of a white solid. Water (3 mL) was added, and the mixture was filtered, the filter cake was washed with a small amount of water and dissolved with DCM, dried with anhydrous sodium sulfate, and then concentrated under reduced pressure to obtain a white solid S18 (319 mg, 100% yield). MS: m/z [M+1]⁺ = 318.28.

### (6) Preparation of Intermediate S19: tert-butyl 4-(4-methyl-3-((1-(1-methyl-2-oxo-1,2-dihydrobenzo[cd]indol-6-yl)cyclopropyl)carbamoyl)phenyl)-3,6-dihydropiperid in-1(2H)-carboxylate

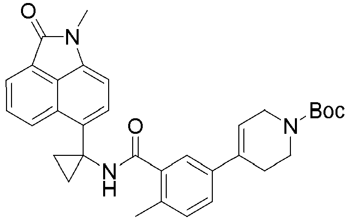

S6 (35 mg, 0.15 mmol) and S18 (56 mg, 0.18 mmol) were placed in a single-necked flask. DMF (1.5 mL), DIPEA (63 mg, 0.49 mmol) and HATU (78 mg, 0.20 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was stirred at room temperature for 0.5 h, filtered, the filter cake was washed with water and purified by column chromatography (PE:EA=2:1) to obtain a yellow solid S19 (64 mg, 80% yield). MS: m/z [M+1]⁺ = 538.38.

### (7) Preparation of P3: 2-methyl-N-(1-(1-methyl-2-oxo-1,2-dihydrobenzo[cd]indol-6-yl)cyclopropyl)-5-(1,2,3,4-tetrahydropiperidin-4-yl)benza mide

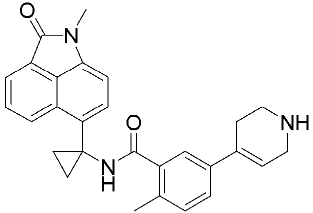

S19 (61 mg, 0.11 mmol) was placed in a single-necked flask. DCM (2.0 mL) and hydrochloric acid (1.0 mL, 4.0 M in 1,4-dioxane) were added. The mixture was stirred at room temperature for 1.0 h. After the starting materials had completely reacted, the reaction solution was concentrated under reduced pressure to remove solvent and obtain a yellow solid mixture. A saturated sodium bicarbonate solution (4 mL) was added to the obtained mixture, extracted three times with DCM, the organic phase was collected and dried with anhydrous sodium sulfate, and then concentrated under reduced pressure to obtain a bright yellow solid P3 (48 mg, 100% yield). ¹ H NMR (600 MHz, DMSO-*d₆*) δ 9.15 (s, 1H), 8.80 (d, J = 8.3 Hz, 1H), 8.04 (d, J = 6.9 Hz, 1H), 7.83 (t, J = 7.6 Hz, 1H), 7.74 (d, J = 7.3 Hz, 1H), 7.29 (d, J = 7.8 Hz, 1H), 7.09 (d, J = 7.8 Hz, 3H), 6.09 (s, 1H), 3.36 (s, 3H), 2.86 (t, J = 5.4 Hz, 2H), 2.22 (s, 2H), 2.03 (s, 3H), 1.33 (d, J = 7.0 Hz, 2H), 1.23 (s, 2H), 1.20 (d, J = 5.4 Hz, 2H). MS: m/z [M+1]⁺ = 438.28.

### Example 4

### Preparation of Compound P4: 2-Methyl-N-(1-(1-methyl-2-oxo-1,2-dihydrobenzo[cd]indol-6-yl)cyclopropyl)-5-(piperidin-4-yl)benzamide

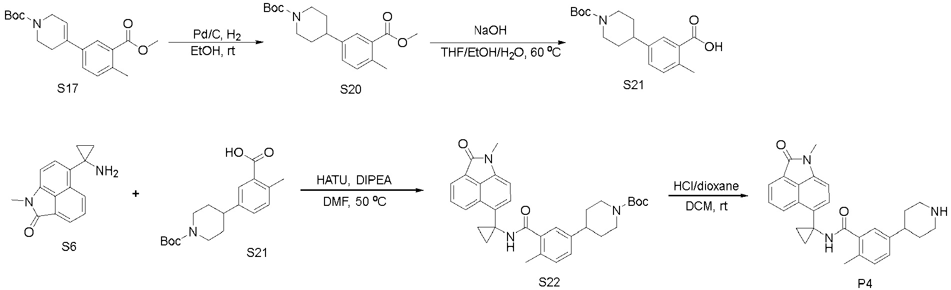

### (1) Preparation of Intermediate S20: tert-butyl 4-(3-(methoxycarbonyl)-4-methylphenyl)piperidin-1-carboxylate

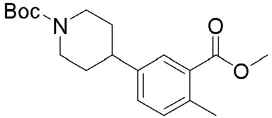

S17 (382 mg, 1.15 mmol) was placed in a single-necked flask. Ethanol (5.0 mL) and palladium-carbon (191 mg, 10%) were added. The mixture was stirred at room temperature under a hydrogen balloon overnight. After the starting materials had completely reacted, the reaction solution was filtered through diatomite, the filter cake was washed with dichloromethane (20 mL). The resulting filtrate was concentrated under reduced pressure and then purified by column chromatography (PE:EA=6:1, 4:1) to obtain a white solid S20 (360 mg, 94% yield). MS: m/z [M+1]⁺ = 334.36.

### (2) Preparation of Intermediate S21: 5-(1-(tert-butoxycarbonyl)piperidin-4-yl)-2-methylbenzoic acid

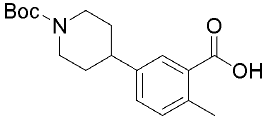

S20 (360 mg, 1.08 mmol) was placed in a single-necked flask. THF (2 mL) and EtOH (2 mL) were added, followed by the addition of a solution of NaOH (216 mg, 5.40 mmol) in H₂O (2 mL). The mixture was heated to 60 °C and stirred for 2 hours. After the starting materials had completely reacted, the reaction solution was concentrated under reduced pressure to remove THF and EtOH, and the pH was then adjusted to 4-5 with a saturated citric acid solution, resulting in the precipitation of a white solid. Water (3 mL) was added, and the mixture was filtered, the filter cake was washed with a small amount of water and dissolved with DCM, dried with anhydrous sodium sulfate, and then concentrated under reduced pressure to obtain a white solid S21 (325 mg, 94% yield). MS: m/z [M+1]⁺ = 320.26.

### (3) Preparation of Intermediate S22: tert-butyl 4-(4-methyl-3-((1-(1-methyl-2-oxo-1,2-dihydrobenzo[cd]indol-6-yl)cyclopropyl)carbamoyl)phenyl)piperidin-1-carboxy late

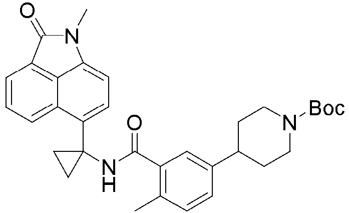

S6 (35 mg, 0.15 mmol) and S21 (57 mg, 0.18 mmol) were placed in a single-necked flask. DMF (1.5 mL), DIPEA (63 mg, 0.49 mmol) and HATU (78 mg, 0.20 mmol) were added. The mixture was heated to 50 °C and stirred for 1.0 h. After the starting materials had completely reacted, the reaction solution was cooled to room temperature. Water was added, resulting in the precipitation of a yellow solid. The resulting mixture was stirred at room temperature for 0.5 h, filtered, the filter cake was washed with water and then purified by column chromatography (PE:EA=2:1) to obtain a yellow solid S22 (61 mg, 75% yield). MS: m/z [M+1]⁺ = 540.32.

### (4) Preparation of P4: 2-methyl-N-(1-(1-methyl-2-oxo-1,2-dihydrobenzo[cd]indol-6-yl)cyclopropyl)-5-(piperidin-4-yl)benzamide

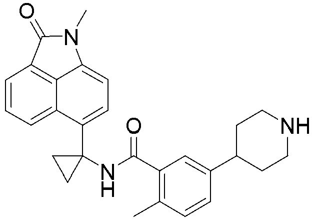

S22 (61 mg, 0.11 mmol) was placed in a single-necked flask. DCM (2.0 mL) and hydrochloric acid (1.0 mL, 4.0 M in 1,4-dioxane) were added. The mixture was stirred at room temperature for 1.0 h. After the starting materials had completely reacted, the reaction solution was concentrated under reduced pressure to remove the solvent and obtain a yellow solid mixture. A saturated sodium bicarbonate solution (4 mL) was added to the obtained mixture, and the mixture was extracted three times with DCM, the organic phase was collected, dried with anhydrous sodium sulfate, and then concentrated under reduced pressure to obtain a bright yellow solid P4 (48 mg, 100% yield). ¹ H NMR (600 MHz, DMSO-*d₆*) δ 9.10 (s, 1H), 8.80 (d, J = 8.2 Hz, 1H), 8.04 (d, J = 6.9 Hz, 1H), 7.84 (t, J = 7.6 Hz, 1H), 7.75 (d, J = 7.3 Hz, 1H), 7.10 (t, J = 6.7 Hz, 2H), 7.05 (d, J = 7.8 Hz, 1H), 6.90 (s, 1H), 3.36 (s, 3H), 2.97 (d, J = 11.8 Hz, 2H), 2.46 (d, J = 11.9 Hz, 1H), 1.99 (s, 3H), 1.58 (d, J = 11.9 Hz, 2H), 1.42-1.39 (m, 2H), 1.33-1.31 (m, 2H), 1.24-1.22 (m, 2H), 1.20 (d, J = 5.5 Hz, 2H). MS: m/z [M+1]⁺ = 440.31.

### Example 5

### Preparation of Compound P5: 5-(3,6-diazabicyclo[3.1.1]heptan-3-yl)-2-methyl-N-(1-(1-methyl-2-oxo-1,2-dihydrobenzo[cd]indol-6-yl)cyclopropyl)be nzamide

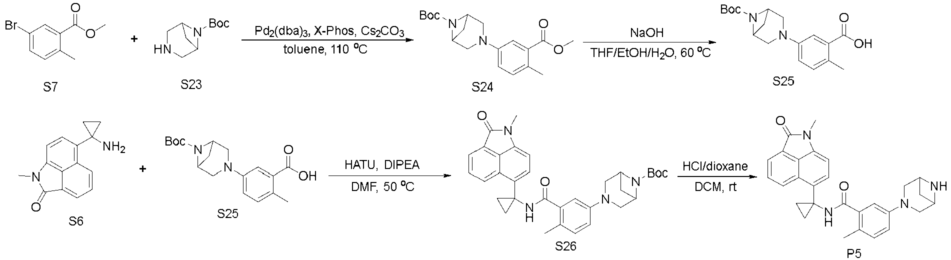

### (1) Preparation of Intermediate S24: tert-butyl 3-(3-(methoxycarbonyl)-4-methylphenyl) -6-azabicyclo[3.1.1]heptan-6-carboxylate

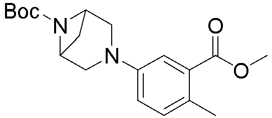

S7 (150 mg, 0.65 mmol) and S23 (154 mg, 0.78 mmol) were placed in a sealed tube. Toluene (3 mL), X-Phos (12 mg, 0.025 mmol), Cs₂CO₃ (423 mg, 1.30 mmol) and Pd₂(dba)₃ (12 mg, 0.013 mmol) were added. The mixture was bubbled with N₂ for 30 seconds, then heated to 110°C and stirred overnight. After the starting materials had completely reacted, the reaction solution was cooled to room temperature, concentrated under reduced pressure and then purified by column chromatography (PE:EA=10:1) to obtain a pale yellow oil S24 (121 mg, 54% yield). MS: m/z [M+1]⁺ = 347.25.

### (2) Preparation of Intermediate S25: 5-6-(tert-butoxycarbonyl)-3,6-diazabicyclo[3.1.1]heptan-3-yl)-2-methylbenzoic acid

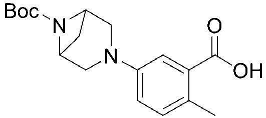

S24 (121 mg, 0.35 mmol) was placed in a single-necked flask. THF (1 mL) and EtOH (1 mL) were added, followed by the addition of a solution of NaOH (70 mg, 1.75 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with a saturated citric acid solution, resulting in the precipitation of a pale yellow solid. Water (3 mL) was added, and the mixture was filtered, the filter cake was washed with a small amount of water and dissolved with DCM, dried with anhydrous sodium sulfate, and then concentrated under reduced pressure to obtain a pale yellow solid S25 (116 mg, 100% yield). MS: m/z [M+1]⁺ = 333.23.

### (3) Preparation of Intermediate S26: tert-butyl 3-(4-methyl-3-((1-(1-methyl-2-oxo-1,2-dihydrobenzo[cd]indol-6-yl)cyclopropyl)carbamoyl)phenyl)-3,6-diazabicyclo[3. 1.1]heptan-6-carboxylate

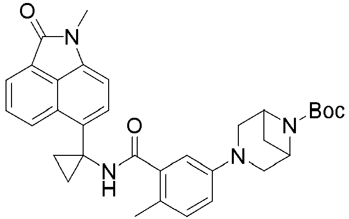

S6 (30 mg, 0.13 mmol) and S25 (50 mg, 0.15 mmol) were placed in a single-necked flask. DMF (1.5 mL), DIPEA (55 mg, 0.42 mmol) and HATU (66 mg, 0.17 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was stirred at room temperature for 0.5 h, filtered, the filter cake was washed with water and purified by column chromatography (PE:EA=3:1, 1:1, 1:2) to obtain a yellow solid S26 (57 mg, 79% yield). MS: m/z [M+1]⁺ = 553.25.

### (4) Preparation of P5: 5-(3,6-diazabicyclo[3.1.1]heptan-3-yl)-2-methyl-N-(1-(1-methyl-2-oxo-1,2-dihydrobenzo[cd]indol-6-yl)cyclopropyl)be nzamide

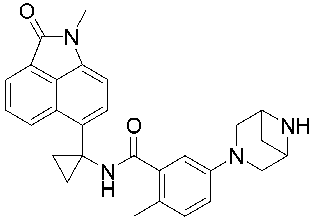

S26 (57 mg, 0.10 mmol) was placed in a single-necked flask. DCM (2.0 mL) and hydrochloric acid (1.0 mL, 4.0 M in 1,4-dioxane) were added. The mixture was stirred at room temperature for 1.0 h. After the starting materials had completely reacted, the reaction solution was concentrated under reduced pressure to remove solvent and obtain a yellow solid mixture. A saturated sodium bicarbonate solution (4 mL) was added to the obtained mixture, extracted three times with DCM, the organic phase was collected and concentrated under reduced pressure, and then purified by column chromatography (DCM:MeOH=20:1, 10:1, 8:1) to obtain a bright yellow solid P5 (37 mg, 82% yield). ¹H NMR (600 MHz, DMSO-*d₆*) δ 9.05 (s, 1H), 8.82 (d, J = 8.2 Hz, 1H), 8.04 (d, J = 6.9 Hz, 1H), 7.83 (dd, J = 8.2, 7.0 Hz, 1H), 7.73 (d, J = 7.3 Hz, 1H), 7.09 (d, J = 7.3 Hz, 1H), 6.98 (d, J = 8.5 Hz, 1H), 6.62 (dd, J = 8.4, 2.7 Hz, 1H), 6.39 (d, J = 2.7 Hz, 1H), 3.99 (d, J = 5.4 Hz, 2H), 3.49 (d, J = 11.0 Hz, 2H), 3.36 (s, 3H), 2.65-2.61 (m, 1H), 1.95 (s, 3H), 1.57 (d, J = 9.1 Hz, 1H), 1.32-1.30 (m, 2H), 1.23 (s, 2H), 1.21-1.19 (m, 2H). MS: m/z [M+1]⁺ = 453.39.

### Example 6

### Preparation of Compound P6: 5-(3,8-diazabicyclo[3.2.1]octan-8-yl)-2-methyl-N-(1-(1-methyl-2-oxo-1,2-dihydrobenzo[cd]indol-6-yl)cyclopropyl)ben zamide

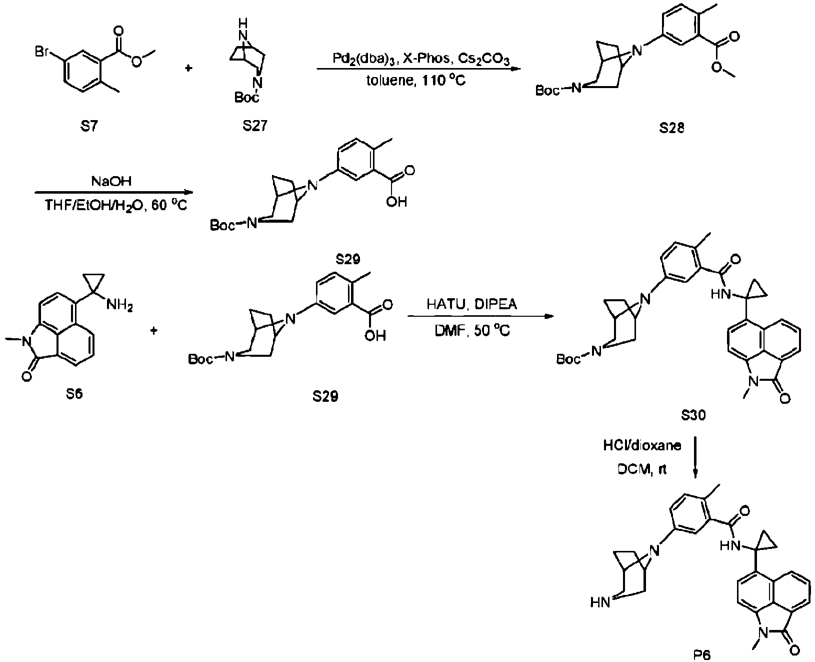

### (1) Preparation of Intermediate S28: tert-butyl 8-(3-(methoxycarbonyl)-4-methylphenyl) -3,8-diazabicyclo[3.2.1]octan-3-benzoate

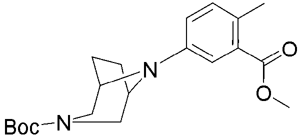

S7 (150 mg, 0.65 mmol) and S27 (166 mg, 0.78 mmol) were placed in a sealed tube. Toluene (3 mL), X-Phos (12 mg, 0.025 mmol), Cs₂CO₃ (423 mg, 1.30 mmol) and Pd₂(dba)₃ (12 mg, 0.013 mmol) were added. The mixture was bubbled with N₂ for 30 seconds, then heated to 110°C and stirred overnight. After the starting materials had completely reacted, the reaction solution was cooled to room temperature and concentrated under reduced pressure, and then purified by column chromatography (PE:EA=10:1) to obtain a pale yellow oil S28 (155 mg, 66% yield). MS: m/z [M+1]⁺ = 360.16.

### (2) Preparation of Intermediate S29: 5-(3-(tert-butoxycarbonyl)-3,8-diazabicyclo[3.2.1]octan-8-yl)-2-methylbenzoic acid

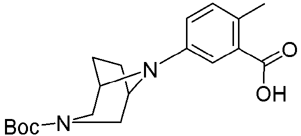

S28 (121 mg, 0.35 mmol) was placed in a single-necked flask. THF (1 mL) and EtOH (1 mL) were added, followed by the addition of a solution of NaOH (70 mg, 1.75 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with a saturated citric acid solution, resulting in the precipitation of a pale yellow solid. Water (3 mL) was added, and the mixture was filtered. The filter cake was washed with a small amount of water and dissolved with DCM, dried with anhydrous sodium sulfate, and then concentrated under reduced pressure to obtain a pale yellow solid S29 (149 mg, 100% yield). MS: m/z [M+1]⁺ = 347.23.

### (3) Preparation of Intermediate S30: tert-butyl 8-(4-methyl-3-((1-(1-methyl-2-oxo-1,2-dihydrobenzo[cd]indol-6-yl)cyclopropyl)carbamoyl)phenyl)-3,8-diazabicyclo[3. 2.1]octan-3-carboxylate

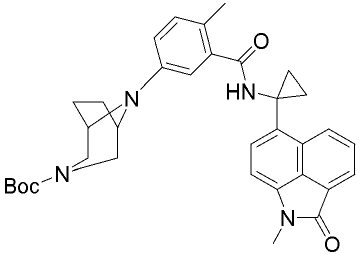

S6 (30 mg, 0.13 mmol) and S29 (52 mg, 0.15 mmol) were placed in a single-necked flask. DMF (1.5 mL), DIPEA (55 mg, 0.42 mmol) and HATU (66 mg, 0.17 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was stirred at room temperature for 0.5 h, filtered, the filter cake was washed with water and then purified by column chromatography (PE:EA=3:1, 1:1) to obtain a yellow solid S30 (61 mg, 82% yield). MS: m/z [M+1]⁺ = 567.23.

### (4) Preparation of P6: 5-(3,8-diazabicyclo[3.2.1]octan-8-yl)-2-methyl-N-(1-(1-methyl-2-oxo-1,2-dihydrobenzo[cd]indol-6-yl)cyclopropyl)ben zamide

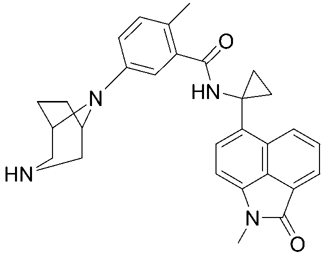

S30 (60 mg, 0.10 mmol) was placed in a single-necked flask. DCM (2.0 mL) and hydrochloric acid (1.0 mL, 4.0 M in 1,4-dioxane) were added. The mixture was stirred at room temperature for 1.0 h. After the starting materials had completely reacted, the reaction solution was concentrated under reduced pressure to remove solvent and obtain a yellow solid mixture. A saturated sodium bicarbonate solution (4 mL) was added to the obtained mixture, extracted three times with DCM, the organic phase was collected and concentrated under reduced pressure, and then purified by column chromatography (DCM:MeOH=20:1, 10:1, 8:1) to obtain a bright yellow solid P6 (46 mg, 98% yield). ¹ H NMR (600 MHz, DMSO-*d₆*) δ 9.06 (s, 1H), 8.80 (d, J = 8.2 Hz, 1H), 8.04 (d, J = 6.9 Hz, 1H), 7.83 (dd, J = 8.2, 7.0 Hz, 1H), 7.73 (d, J = 7.3 Hz, 1H), 7.08 (d, J = 7.3 Hz, 1H), 6.94 (d, J = 8.5 Hz, 1H), 6.73 (dd, J = 8.4, 2.6 Hz, 1H), 6.47 (d, J = 2.5 Hz, 1H), 4.06 (s, 2H), 3.36 (s, 3H), 2.89 (d, J = 11.9 Hz, 2H), 2.56 (d, J = 12.2 Hz, 2H), 1.91 (s, 3H), 1.32-1.30 (m, 2H), 1.23 (s, 4H), 1.20-1.18 (m, 2H). MS: m/z [M+1]⁺ = 467.32.

### Example 7

### Preparation of Compound P7: 2-Methyl-5-(1-methyl-1,2,3,6-tetrahydropiperidin -4-yl)-N-(1-(1-methyl-2-oxo-1,2-dihydrobenzo[cd]indol-6-yl)cyclopropyl)benzamide

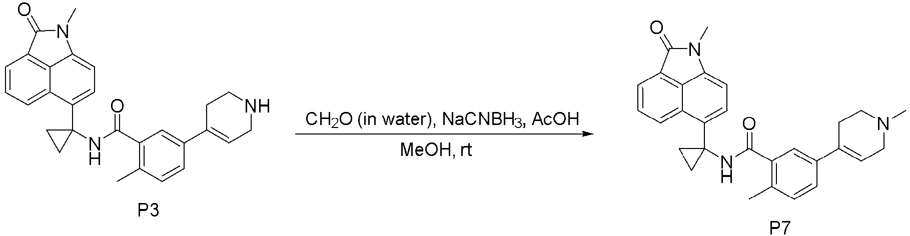

### (1) Preparation of P7: 2-methyl-5-(1-methyl-1,2,3,6-tetrahydropiperidin -4-yl)-N-(1-(1-methyl-2-oxo-1,2-dihydrobenzo[cd]indol-6-yl)cyclopropyl)benzamide

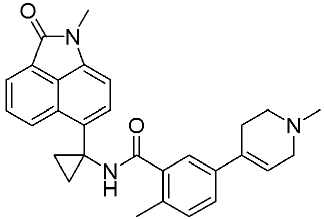

P3 (20 mg, 0.046 mmol) was placed in a single-necked flask. MeOH (1.0 mL), formaldehyde aqueous solution (0.30 mL) and acetic acid (7 mg, 0.12 mmol) were added, and finally NaCNBH₃ (4 mg, 0.069 mmol) was added. The mixture was stirred at room temperature for 2 hours. After the starting materials had completely reacted, a saturated sodium bicarbonate solution (5 mL) was added to the reaction solution, extracted three times with DCM, and the organic phases were combined. The combined organic phases were concentrated under reduced pressure to obtain a yellow solid. The yellow solid was then purified by column chromatography (PE:EA = 1:1, DCM:MeOH = 30:1, 20:1) to obtain a bright yellow solid P7 (12 mg, 57% yield). ¹ H NMR (600 MHz, DMSO-*d₆*) δ 9.16 (s, 1H), 8.80 (d, J = 8.2 Hz, 1H), 8.18 (s, 1H), 8.04 (d, J = 6.9 Hz, 1H), 7.84 (dd, J = 8.2, 7.0 Hz, 1H), 7.74 (d, J = 7.3 Hz, 1H), 7.31 (dd, J = 8.0, 1.9 Hz, 1H), 7.10 (q, J = 3.6, 3.1 Hz, 2H), 6.06 (t, J = 3.4 Hz, 1H), 3.36 (s, 3H), 3.02-3.01 (m, 2H), 2.57 (t, J = 5.7 Hz, 2H), 2.39 (s, 2H), 2.29 (s, 3H), 2.03 (s, 3H), 1.34-1.31 (m, 2H), 1.21-1.19 (m, 2H). MS: m/z [M+1]⁺ = 452.35.

### Example 8

### Preparation of Compound P8: 2-Methyl-N-(1-(1-methyl-2-oxo-1,2-dihydrobenzo[cd]indol-6-yl)cyclopropyl)-3-methyl-3,8-diazabicyclo[3.2.1]octan-8 -yl benzamide

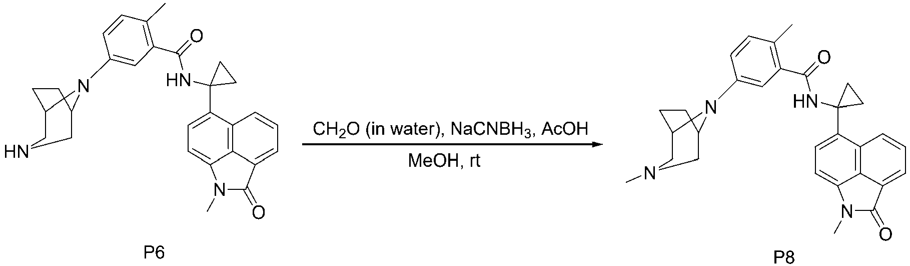

### (1) Preparation of P8: 2-methyl-N-(1-(1-methyl-2-oxo-1,2-dihydrobenzo[cd]indol-6-yl)cyclopropyl)-3-methyl-3,8-diazabicyclo[3.2.1]octan-8-yl benzamide

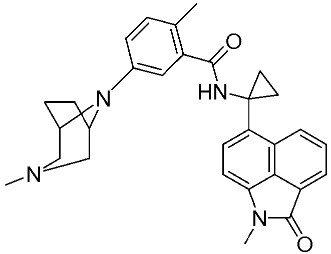

P6 (20 mg, 0.043 mmol) was placed in a single-necked flask. MeOH (1.0 mL), formaldehyde aqueous solution (0.30 mL) and acetic acid (7 mg, 0.11 mmol) were added, and finally NaCNBH₃ (4 mg, 0.065 mmol) was added. The mixture was stirred at room temperature for 2 hours. After the starting materials had completely reacted, a saturated sodium bicarbonate solution (5 mL) was added to the reaction solution, extracted three times with DCM, and the organic phases were combined. The combined organic phases were concentrated under reduced pressure to obtain a yellow solid. The yellow solid was then purified by column chromatography (PE:EA = 1:1 to DCM:MeOH = 30:1, 20:1) to obtain a bright yellow solid P8 (20 mg, 95% yield). MS: m/z [M+1]⁺ = 481.36.

### Example 9

### Preparation of Compound P9: 5-(hexahydropyrrolo[3,4-c]pyrrol-2(1H)-yl)-2-methyl-N-(1-(1-methyl-2-oxo-1,2-dihydrobenzo[cd]indol-6-yl)cycloprop yl)benzamide

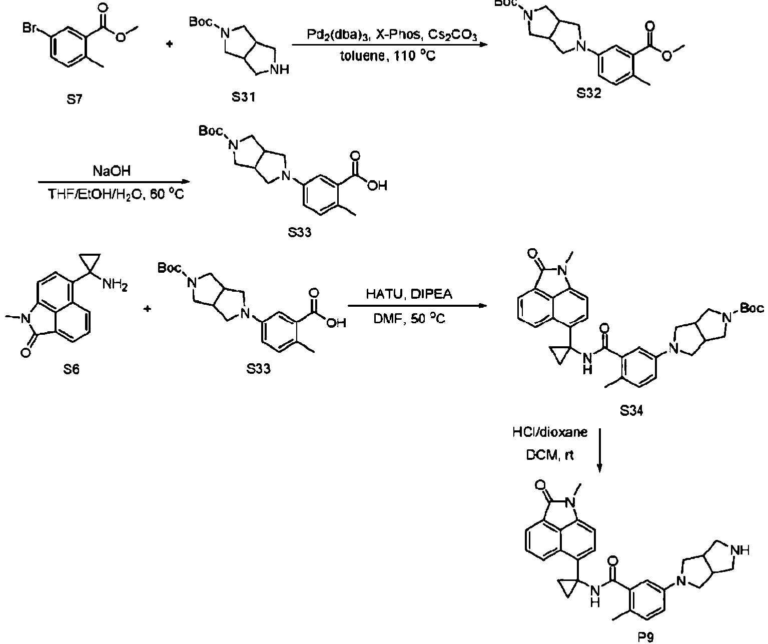

### (1) Preparation of Intermediate S32: tert-butyl 5-(3-(methoxycarbonyl)-4-methylphenyl)hexahydropyrrolo[3,4-c]pyrrol-2(1H)-carboxylate

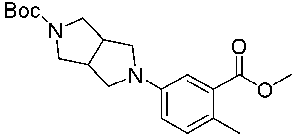

S7 (150 mg, 0.65 mmol) and S31 (165 mg, 0.78 mmol) were placed in a sealed tube. Toluene (3 mL), X-Phos (12 mg, 0.025 mmol), Cs₂CO₃ (423 mg, 1.30 mmol) and Pd₂(dba)₃ (12 mg, 0.013 mmol) were added. The mixture was bubbled with N₂ for 30 seconds, then heated to 110°C and stirred overnight. After the starting materials had completely reacted, the reaction solution was cooled to room temperature and concentrated under reduced pressure, and then purified by column chromatography (PE:EA=10:1) to obtain a pale yellow oil S32 (171 mg, 73% yield). MS: m/z [M+1]⁺ = 361.18.

### (2) Preparation of Intermediate S33: 5-(5-(tert-butoxycarbonyl)hexahydropyrrolo[3,4-c]pyrrol-2(1H)-yl)-2-methylbenzoic acid

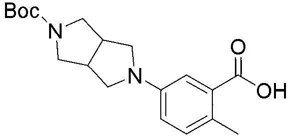

S32 (171 mg, 0.47 mmol) was placed in a single-necked flask. THF (1.5 mL) and EtOH (1.5 mL) were added, followed by the addition of a solution of NaOH (94 mg, 2.35 mmol) in H₂O (1.5 mL). The mixture was heated to 60 °C and stirred for 2 hours. After the starting materials had completely reacted, the reaction solution was concentrated under reduced pressure to remove THF and EtOH, and the pH was then adjusted to 4-5 with a saturated citric acid solution, resulting in the precipitation of a pale yellow solid. Water (3 mL) was added, and the mixture was filtered. The filter cake was washed with a small amount of water and then dissolved with DCM, dried with anhydrous sodium sulfate, and then concentrated under reduced pressure to obtain a yellow solid S33 (163 mg, 100% yield). MS: m/z [M+1]⁺ = 347.23.

### (3) Preparation of Intermediate S34: tert-butyl 5-(4-methyl-3-((1-(1-methyl-2-oxo-1,2-dihydrobenzo[cd]indol-6-yl)cyclopropyl)carbamoyl)phenyl) hexahydropyrrolo[3,4-c]pyrrol-2(1H)-carboxylate

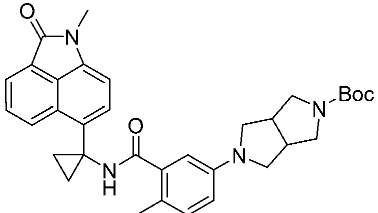

S6 (30 mg, 0.13 mmol) and S33 (52 mg, 0.15 mmol) were placed in a single-necked flask. DMF (1.5 mL), DIPEA (55 mg, 0.42 mmol) and HATU (66 mg, 0.17 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was stirred at room temperature for 0.5 h, filtered, the filter cake was washed with water and then purified by column chromatography (PE:EA=4:1, 1:1) to obtain a pale yellow solid S34 (58 mg, 78% yield). MS: m/z [M+1]⁺ = 567.23.

### (4) Preparation of P9: 5-(hexahydropyrrolo[3,4-c]pyrrol-2(1H)-yl)-2-methyl-N-(1-(1-methyl-2-oxo-1,2-dihydrobenzo[cd]indol-6-yl)cycloprop yl)benzamide

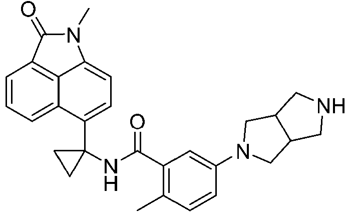

S34 (58 mg, 0.10 mmol) was placed in a single-necked flask. DCM (2.0 mL) and hydrochloric acid (1.0 mL, 4.0 M in 1,4-dioxane) were added. The mixture was stirred at room temperature for 1.0 h. After the starting materials had completely reacted, the reaction solution was concentrated under reduced pressure to remove solvent and obtain a yellow solid mixture. A saturated sodium bicarbonate solution (5 mL) was added to the obtained mixture, extracted three times with DCM, the organic phase was collected and concentrated under reduced pressure, and then purified by column chromatography (DCM:MeOH=20:1, 10:1, 8:1) to obtain a bright yellow solid P9 (42 mg, 89% yield). ¹H NMR (600 MHz, DMSO-*d₆*) δ 9.03 (s, 1H), 8.81 (d, J = 8.2 Hz, 1H), 8.04 (d, J = 6.9 Hz, 1H), 7.83 (dd, J = 8.2, 7.0 Hz, 1H), 7.73 (d, J = 7.3 Hz, 1H), 7.08 (d, J = 7.3 Hz, 1H), 6.91 (d, J = 8.4 Hz, 1H), 6.53 (dd, J = 8.3, 2.6 Hz, 1H), 6.30 (d, J = 2.6 Hz, 1H), 3.36 (s, 3H), 3.23-3.21 (m, 2H), 2.97 (dd, J = 11.0, 6.8 Hz, 2H), 2.93 (dd, J = 9.6, 3.1 Hz, 2H), 2.60 (dd, J = 11.2, 3.3 Hz, 2H), 1.91 (s, 3H), 1.31-1.29 (m, 2H), 1.23 (s, 3H), 1.19-1.17 (m, 2H). MS: m/z [M+1]⁺ = 467.32.

### Example 10

### Preparation of Compound P10: 2-Methyl-N-(1-(1-methyl-2-oxo-1,2-dihydrobenzo[cd]indol-6-yl)cyclopropyl)-5-(2-methylpiperazin-1-yl)benzamide

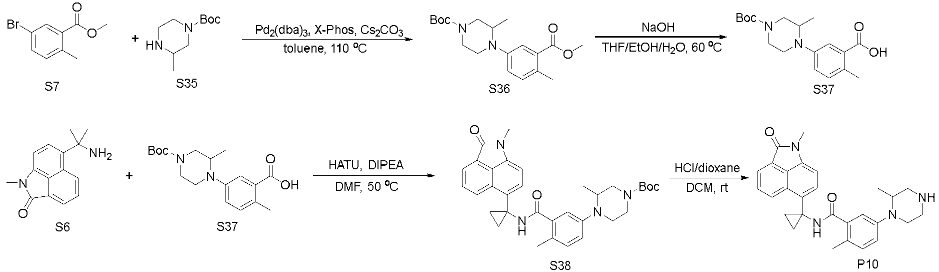

### (1) Preparation of Intermediate S36: tert-butyl 4-(3-(methoxycarbonyl)-4-methylphenyl)-3-methylpiperazin-1-carboxylate

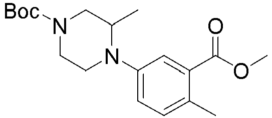

S7 (150 mg, 0.65 mmol) and S35 (156 mg, 0.78 mmol) were placed in a sealed tube. Toluene (3 mL), X-Phos (12 mg, 0.025 mmol), Cs₂CO₃ (423 mg, 1.30 mmol) and Pd₂(dba)₃ (12 mg, 0.013 mmol) were added. The mixture was bubbled with N₂ for 30 seconds, then heated to 110°C and stirred overnight. After the starting materials had completely reacted, the reaction solution was cooled to room temperature and concentrated under reduced pressure, and then purified by column chromatography (PE:EA=10:1) to obtain a pale yellow oil S36 (110 mg, 49% yield). MS: m/z [M+1]⁺ = 349.36.

### (2) Preparation of Intermediate S37: 5-(4-(tert-butoxycarbonyl)-2-methylpiperazin-1-yl)-2-methylbenzoic acid

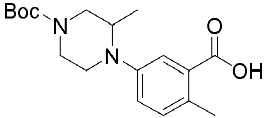

S36 (110 mg, 0.31 mmol) was placed in a single-necked flask. THF (1 mL) and EtOH (1 mL) were added, followed by the addition of a solution of NaOH (63 mg, 1.58 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with a saturated citric acid solution, resulting in the precipitation of a pale yellow solid. Water (3 mL) was added, and the mixture was filtered. The filter cake was washed with a small amount of water and dissolved with DCM, dried with anhydrous sodium sulfate, and then concentrated under reduced pressure to obtain a pale yellow solid S37 (103 mg, 100% yield). MS: m/z [M+1]⁺ = 335.19.

### (3) Preparation of Intermediate S38: tert-butyl 3-methyl-4-(4-methyl-3-((1-(1-methyl-2-oxo-1,2-dihydrobenzo[cd]indol-6-yl)cyclopropyl)carbamoyl)phenyl)piperazin-1-carboxylate

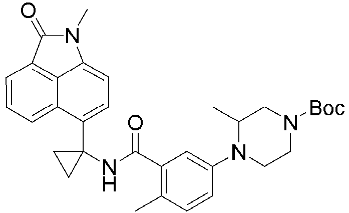

S6 (30 mg, 0.13 mmol) and S37 (50 mg, 0.15 mmol) were placed in a single-necked flask. DMF (1.5 mL), DIPEA (55 mg, 0.42 mmol) and HATU (66 mg, 0.17 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was stirred at room temperature for 0.5 h, filtered, and the filter cake was washed with water and then purified by column chromatography (PE:EA=4:1, 1:1) to obtain a pale yellow solid S38 (58 mg, 81% yield). MS: m/z [M+1]⁺ = 555.25.

### (4) Preparation of P10: 2-methyl-N-(1-(1-methyl-2-oxo-1,2-dihydrobenzo[cd]indol-6-yl)cyclopropyl)-5-(2-methylpiperazin-1-yl)benzamide

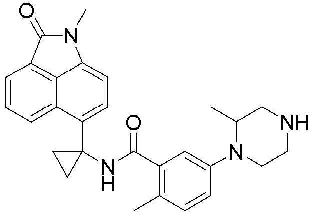

S38 (58 mg, 0.10 mmol) was placed in a single-necked flask. DCM (2.0 mL) and hydrochloric acid (1.0 mL, 4.0 M in 1,4-dioxane) were added. The mixture was stirred at room temperature for 1.0 h. After the starting materials had completely reacted, the reaction solution was concentrated under reduced pressure to remove solvent and obtain a yellow solid mixture. A saturated sodium bicarbonate solution (5 mL) was added to the obtained mixture, extracted three times with DCM, the organic phase was collected and concentrated under reduced pressure, and then purified by column chromatography (DCM:MeOH=20:1, 10:1, 8:1) to obtain a bright yellow solid P10 (45 mg, 100% yield). ¹ H NMR (600 MHz, DMSO-d6) δ 9.06 (s, 1H), 8.81 (d, J = 8.2 Hz, 1H), 8.04 (d, J = 6.9 Hz, 1H), 7.83 (dd, J = 8.2, 7.0 Hz, 1H), 7.73 (d, J = 7.3 Hz, 1H), 7.09 (d, J = 7.3 Hz, 1H), 6.98 (d, J = 8.5 Hz, 1H), 6.82 (dd, J = 8.4, 2.6 Hz, 1H), 6.56 (d, J = 2.6 Hz, 1H), 3.36 (s, 3H), 3.07 (dt, J = 11.1, 2.8 Hz, 1H), 3.02 (d, J = 11.8 Hz, 1H), 2.98 (dd, J = 12.2, 3.5 Hz, 1H), 2.87-2.82 (m, 2H), 1.94 (s, 3H), 1.32-1.30 (m, 2H), 1.23 (s, 2H), 1.19 (dd, J = 4.8, 3.0 Hz, 2H), 0.89 (d, J = 6.6 Hz, 3H). MS: m/z [M+1]⁺ = 455.27.

### Example 11

### Preparation of Compound P11: 2-methyl-N-(1-(1-methyl-2-oxo-1,2-dihydrobenzo[cd]indol-6-yl)cyclopropyl)-5-(6-methyl-3,6-diazabicyclo[3.1.1]hepta n-3-yl)benzamide

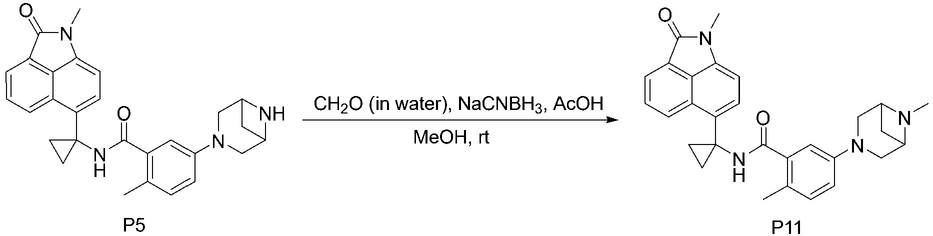

### (1) Preparation of P11: 2-methyl-N-(1-(1-methyl-2-oxo-1,2-dihydrobenzo[cd]indol-6-yl)cyclopropyl)-5-(6-methyl-3,6-diazabicyclo[3.1.1]hepta n-3-yl)benzamide

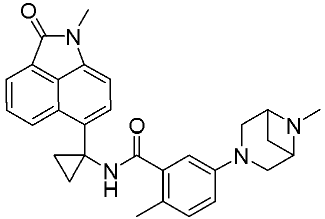

P5 (18 mg, 0.040 mmol) was placed in a single-necked flask. MeOH (1.0 mL), formaldehyde aqueous solution (0.30 mL) and acetic acid (6 mg, 0.10 mmol) were added, and finally NaCNBH₃ (4 mg, 0.060 mmol) was added. The mixture was stirred at room temperature for 2 hours. After the starting materials had completely reacted, a saturated sodium bicarbonate solution (5 mL) was added to the reaction solution, extracted three times with DCM, and the organic phases were combined. The combined organic phases were concentrated under reduced pressure to obtain a yellow solid. The yellow solid was then purified by column chromatography (DCM:MeOH=10:1, 7:1) to obtain a bright yellow solid P11 (14 mg, 77% yield). MS: m/z [M+1]⁺ = 467.32.

### Example 12

### Preparation of Compound P12: 2-Methyl-N-(1-(1-methyl-2-oxo-1,2-dihydrobenzo[cd]indol-6-yl)cyclopropyl)-5-(4,7-diazaspiro[2,5]octan-7-yl) benzamide

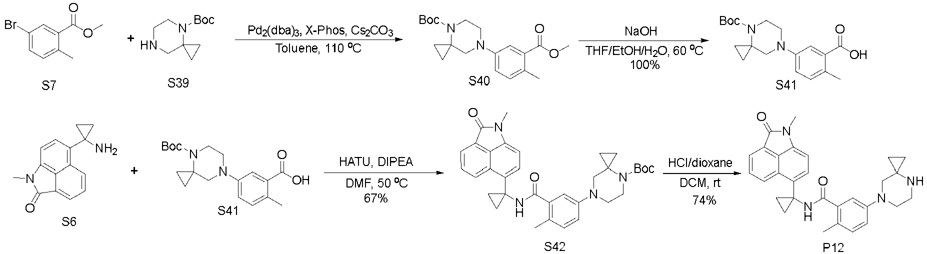

### (1) Preparation of Intermediate S40: tert-butyl 7-(3-(methoxycarbonyl)-4-methylphenyl)-4,7-diazaspiro[2,5]octan-4-carboxylate

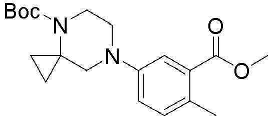

S7 (42 mg, 0.18 mmol) and S39 (46 mg, 0.22 mmol) were placed in a sealed tube. Toluene (1 mL), X-Phos (3 mg, 0.0069 mmol), Cs₂CO₃ (117 mg, 0.36 mmol) and Pd₂(dba)₃ (3 mg, 0.0036 mmol) were added. The mixture was bubbled with N₂ for 30 seconds, then heated to 110°C and stirred overnight. After the starting materials had completely reacted, the reaction solution was cooled to room temperature and concentrated under reduced pressure, and then purified by column chromatography (PE:EA=20:1, 10:1, 8:1) to obtain a pale yellow oil S40 (35 mg, 54% yield). MS: m/z [M+1]⁺ = 361.38.

### (2) Preparation of Intermediate S41: 5-(4-(tert-butoxycarbonyl)-4,7-diazaspiro[2,5]octan-7-yl)-2-methylbenzoic acid

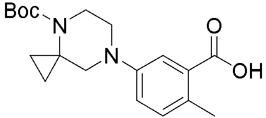

S40 (35 mg, 0.097 mmol) was placed in a single-necked flask. THF (0.5 mL) and EtOH (0.5 mL) were added, followed by the addition of a solution of NaOH (19 mg, 0.49 mmol) in H₂O (0.5 mL). The mixture was heated to 60 °C and stirred for 2 hours. After the starting materials had completely reacted, the reaction solution was concentrated under reduced pressure to remove THF and EtOH, and the pH was then adjusted to 4-5 with a saturated citric acid solution, resulting in the precipitation of a pale yellow solid. Water (3 mL) was added, and the mixture was filtered. The filter cake was washed with a small amount of water and then dissolved with DCM, dried with anhydrous sodium sulfate, and then concentrated under reduced pressure to obtain a yellow solid S41 (33 mg, 100% yield). MS: m/z [M+1]⁺ = 347.15.

### (3) Preparation of Intermediate S42: tert-butyl 7-(4-methyl-3-((1-(1-methyl-2-oxo-1,2-dihydrobenzo[cd]indol-6-yl)cyclopropyl)carbamoyl)phenyl)-4,7-diazaspiro[2,5] octan-4-carboxylate

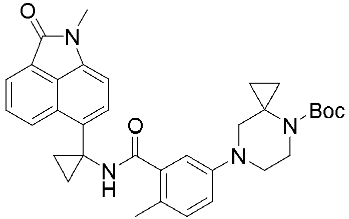

S6 (21 mg, 0.086 mmol) and S41 (33 mg, 0.095 mmol) were placed in a single-necked flask. DMF (1.5 mL), DIPEA (36 mg, 0.28 mmol) and HATU (43 mg, 0.11 mmol) were added. The mixture was heated to 50°C and stirred for 1.0 hour. After the starting materials had completely reacted, the reaction solution was cooled to room temperature. Water was added, resulting in the precipitation of a yellow solid. The resulting mixture was stirred at room temperature for 0.5 h, filtered, the filter cake was washed with water and then purified by column chromatography (PE:EA=4:1, 2:1, 1:1) to obtain a pale yellow solid S42 (33 mg, 67% yield). MS: m/z [M+1]⁺ = 567.23.

### (4) Preparation of P12: 2-methyl-N-(1-(1-methyl-2-oxo-1,2-dihydrobenzo[cd]indol-6-yl)cyclopropyl)-5-(4,7-diazaspiro[2,5]octan-7-yl) benzamide

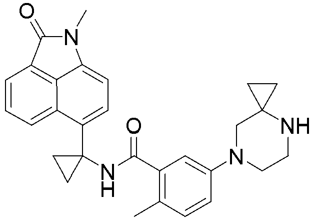

S42 (33 mg, 0.058 mmol) was placed in a single-necked flask. DCM (2.0 mL) and hydrochloric acid (1.0 mL, 4.0 M in 1,4-dioxane) were added. The mixture was stirred at room temperature for 1.0 h. After the starting materials had completely reacted, the reaction solution was concentrated under reduced pressure to remove solvent and obtain a yellow solid mixture. A saturated sodium bicarbonate solution (5 mL) was added to the obtained mixture, extracted three times with DCM, the organic phase was collected and concentrated under reduced pressure, and then purified by column chromatography (DCM:MeOH=10:1) to obtain a bright yellow solid P12 (20 mg, 74% yield). MS: m/z [M+1]⁺ = 467.37.

### Example 13

### Preparation of Compound P13: 5-(4-(Dimethylamino)piperidin-1-yl)-2-methyl-N-(1-(1-methyl-2-oxo-1,2-dihydrobenzo[cd]indol-6-yl)cyclopropyl)ben zamide

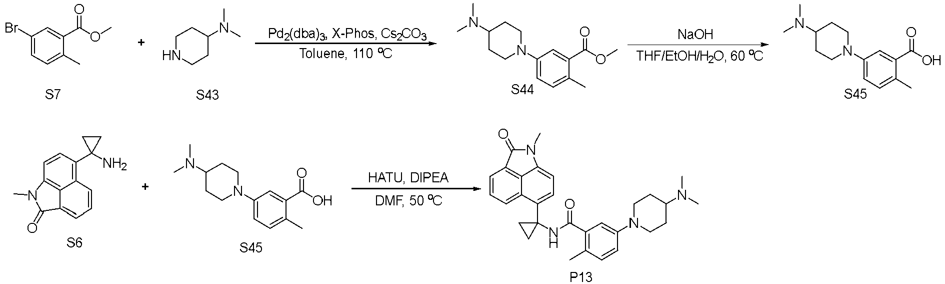

### (1) Preparation of Intermediate S44: methyl 5-(4-(dimethylamino)piperidin-1-yl)-2-methylbenzoate

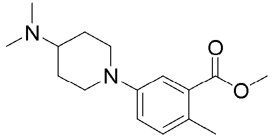

S7 (150 mg, 0.65 mmol) and S43 (100 mg, 0.78 mmol) were placed in a sealed tube. Toluene (3 mL), X-Phos (12 mg, 0.025 mmol), Cs₂CO₃ (423 mg, 1.30 mmol) and Pd₂(dba)₃ (12 mg, 0.013 mmol) were added. The mixture was bubbled with N₂ for 30 seconds, then heated to 110°C and stirred overnight. After the starting materials had completely reacted, the reaction solution was cooled to room temperature and concentrated under reduced pressure, and then purified by column chromatography (DCM:MeOH=20:1, 10:1, 8:1) to obtain a pale yellow oil S44 (70 mg, 39% yield). MS: m/z [M+1]⁺ = 277.38.

### (2) Preparation of Intermediate 45: 5-(4-(Dimethylamino)piperidin-1-yl)-2-methylbenzoic acid

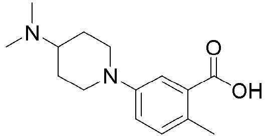

S44 (70 mg, 0.25 mmol) was placed in a single-necked flask. THF (1 mL) and EtOH (1 mL) were added, followed by the addition of a solution of NaOH (51 mg, 1.26 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2.0 M). The resulting mixture was concentrated under reduced pressure to remove the solvent and obtain a solid. The obtained solid was dissolved in DCM, dried with anhydrous sodium sulfate and then filtered, and the filter cake was washed with DCM. The filtrate was collected and concentrated under reduced pressure to remove solvent and thus obtained a pale yellow solid S45 (65 mg, 100% yield). MS: m/z [M+1]⁺ = 263.19.

### (3) Preparation of P13: 5-(4-(dimethylamino)piperidin-1-yl)-2-methyl-N-(1-(1-methyl-2-oxo-1,2-dihydrobenzo[cd]indol-6-yl)cyclopropyl)benz amide

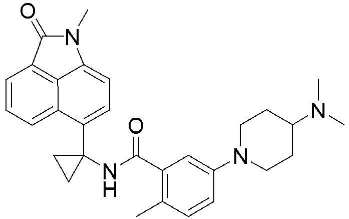

S6 (20 mg, 0.084 mmol) and S45 (26 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was stirred at room temperature for 0.5 h and then filtered. The filter cake was washed with water and then purified by column chromatography (PE:EA=1:1, DCM:MeOH=20:1-10:1) to obtain a bright yellow solid P13 (23 mg, 57% yield). MS: m/z [M+1]⁺ = 483.32.

### Example 14

### Preparation of Compound P14: (S)-5-(4-ethyl-3-methylpiperazin-1-yl)-2-methyl-N-(1-(1-methyl-2-oxo-1,2-dihydrobenzo[cd]indol-6-yl)cyclopropyl)be nzamide

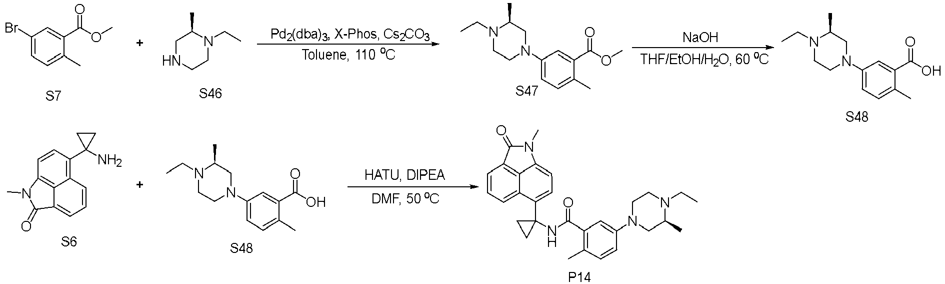

### (1) Preparation of Intermediate S47: methyl (S)-5-(4-ethyl-3-methylpiperazin-1-yl)-2-methylbenzoate

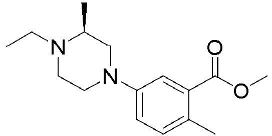

S7 (150 mg, 0.65 mmol) and S46 (100 mg, 0.78 mmol) were placed in a sealed tube. Toluene (3 mL), X-Phos (12 mg, 0.025 mmol), Cs₂CO₃ (423 mg, 1.30 mmol) and Pd₂(dba)₃ (12 mg, 0.013 mmol) were added. The mixture was bubbled with N₂ for 30 seconds, then heated to 110°C and stirred overnight. After the starting materials had completely reacted, the reaction solution was cooled to room temperature and concentrated under reduced pressure, and then purified by column chromatography (PE:EA=1:1, 1:2) to obtain a colorless oil S47 (66 mg, 37% yield). MS: m/z [M+1]⁺ = 277.38.

### (2) Preparation of Intermediate S48: (S)-5-(4-ethyl-3-methylpiperazin-1-yl)-2-methylbenzoic acid

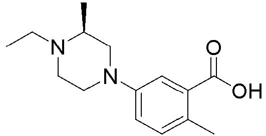

S47 (66 mg, 0.24 mmol) was placed in a single-necked flask. THF (1 mL) and EtOH (1 mL) were added, followed by the addition of a solution of NaOH (48 mg, 1.20 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2.0 M). The resulting mixture was then concentrated under reduced pressure to remove the solvent and obtain a solid. The obtained solid was dissolved in DCM, dried with anhydrous sodium sulfate, and filtered. The filter cake was washed with DCM. The filtrate was collected and concentrated under reduced pressure to remove the solvent to obtain a pale yellow solid S48 (63 mg, 100% yield). MS: m/z [M+1]⁺ = 263.19.

### (3) Preparation of P14: (S)-5-(4-ethyl-3-methylpiperazin-1-yl)-2-methyl-N-(1-(1-methyl-2-oxo-1,2-dihydrobenzo[cd]indol-6-yl)cyclopropyl)be nzamide

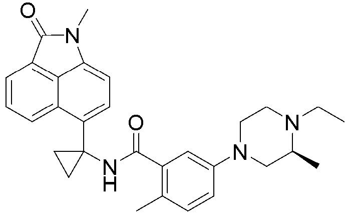

S6 (20 mg, 0.084 mmol) and S48 (26 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was stirred at room temperature for 0.5 h, filtered, the filter cake was washed with water and then purified by column chromatography (PE:EA=1:1, DCM:MeOH=20:1, 10:1) to obtain a bright yellow solid P14 (18 mg, 45% yield). MS: m/z [M+1]⁺ = 483.32.

### Example 15

### Preparation of Compound P15: 5-(3-(dimethylamino)pyrrol-1-yl)-2-methyl-N-(1-(1-methyl-2-oxo-1,2-dihydrobenzo[cd]indol-6-yl)cyclopropyl)benzam ide

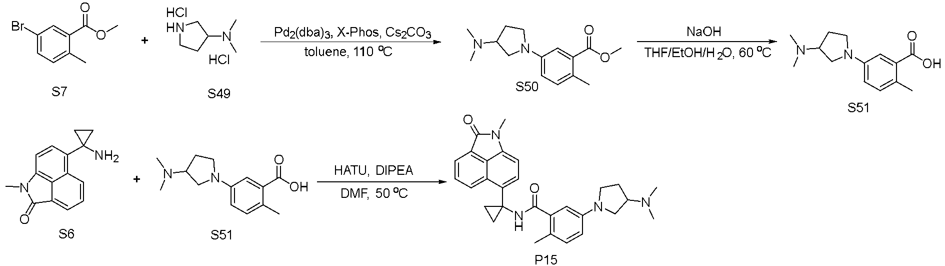

### (1) Preparation of Intermediate S50: methyl 5-(3-(dimethylamino)pyrrol-1-yl)-2-methylbenzoate

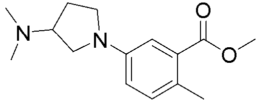

S7 (101 mg, 0.44 mmol) and S49 (100 mg, 0.53 mmol) were placed in a sealed tube. Toluene (3 mL), X-Phos (8 mg, 0.017 mmol), Cs₂CO₃ (717 mg, 2.20 mmol) and Pd₂(dba)₃ (8 mg, 0.0088 mmol) were added. The mixture was bubbled with N₂ for 30 seconds, then heated to 110°C and stirred overnight. After the starting materials had completely reacted, the reaction solution was cooled to room temperature and filtered through diatomite, and the filter cake was washed with EA. The resulting filtrate was concentrated under reduced pressure and then purified by column chromatography (PE:EA=1:1, 1:2) to obtain a pale yellow oil S50 (91 mg, 79% yield). MS: m/z [M+1]⁺ = 263.38.

### (2) Preparation of Intermediate S51: 5-(3-(dimethylamino)pyrrol-1-yl)-2-methylbenzoic acid

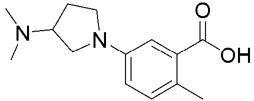

S50 (91 mg, 0.34 mmol) was placed in a single-necked flask. THF (1 mL) and EtOH (1 mL) were added, followed by the addition of a solution of NaOH (68 mg, 1.70 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2.0 M). The resulting mixture was concentrated under reduced pressure to remove the solvent and obtain a solid. The obtained solid was dissolved in DCM, then dried with anhydrous sodium sulfate and filtered, and the filter cake was washed with DCM. The filtrate was concentrated under reduced pressure to remove solvent and thus obtained a pale yellow solid S51 (84 mg, 100% yield). MS: m/z [M+1]⁺ = 249.33.

### (3) Preparation of P15: 5-(3-(dimethylamino)pyrrol-1-yl)-2-methyl-N-(1-(1-methyl-2-oxo-1,2-dihydrobenzo[cd]indol-6-yl)cyclopropyl)benzam ide

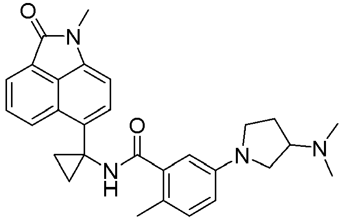

S6 (20 mg, 0.084 mmol) and S51 (25 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was stirred at room temperature for 0.5 h, filtered, the filter cake was washed with water and then purified by column chromatography (PE:EA=1:1, DCM:MeOH=20:1, 10:1) to obtain a bright yellow solid P15 (21 mg, 54% yield). MS: m/z [M+1]⁺ = 469.31.

### Example 16

### Preparation of Compound P16: 5-(3,6-diazabicyclo[3.1.1]heptan-3-yl)-N-(1-(1-isopropyl-2-oxo-1,2-dihydrobenzo[cd]indol-6-yl)cyclopropyl)benzamid e

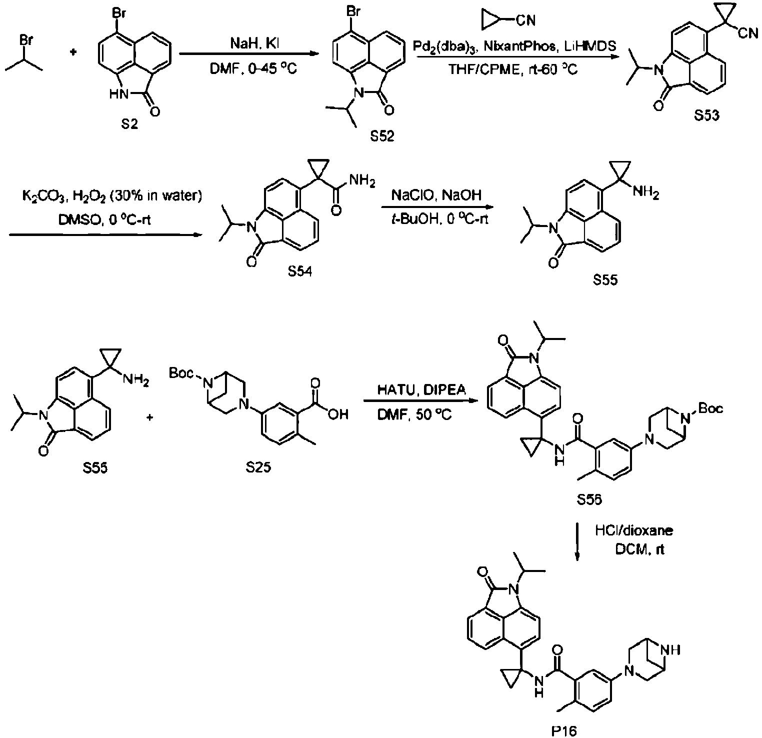

### (1) Preparation of Intermediate S52: 6-bromo-1-isopropylbenzo[cd]indol-2(1H)-one

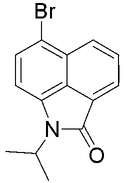

S2 (1000 mg, 4.03 mmol) was placed in a single-necked flask. DMF (15 mL) was added. The mixture was cooled to 0°C. NaH (242 mg, 6.05 mmol, 60% dispersed in mineral oil) was then added and the mixture was stirred for 10 minutes. Then, potassium iodide (792 mg, 4.77 mmol) and 2-bromopropane (1174 mg, 9.54 mmol) were added. The mixture was heated to 45°C and stirred overnight. The reaction solution was cooled to room temperature. Water (50 mL) was added, resulting in the precipitation of an orange solid. The resulting mixture was stirred at room temperature for 0.5 h, filtered, and the filter cake was washed with water, dried, and then purified by column chromatography (PE:EA=30:1) to obtain yellow solid S52 (933 mg, 80% yield). MS: m/z [M+1]⁺ = 291.26.

### (2) Preparation of Intermediate S53: 1-(1-isopropyl-2-oxo-1,2-dihydrobenzo[cd]indol-6-yl)cyclopropan-1-carbonitrile

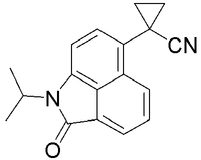

Pd₂(dba)₃ (157 mg, 0.17 mmol) and NixantPhos (176 mg, 0.32 mmol) were placed in a three-necked flask, followed by the addition of THF (10 mL). The mixture was bubbled with nitrogen for 30 seconds and stirred at room temperature for 5 minutes. Cyclopentyl methyl ether (10 mL), S52 (933 mg, 3.22 mmol), and cyclopropyl cyanide (325 mg, 4.84 mmol) were added sequentially. Then, LiHMDS (6 mL, 6.44 mmol, 1.0 M in THF) was added dropwise. The mixture was heated to 60 °C and stirred for 1.0 hour. After the starting materials had completely reacted, the reaction solution was cooled to room temperature. A saturated ammonium chloride solution (20 mL) was added. The mixture was extracted three times with EA, and the organic phases were combined. The combined organic phases were concentrated under reduced pressure and then purified by column chromatography (PE:EA=20:1, 10:1, 5:1) to obtain a yellow solid S53 (267 mg, 30% yield). MS: m/z [M+1]⁺ = 277.32.

### (3) Preparation of Intermediate S54: 1-(1-isopropyl-2-oxo-1,2-dihydrobenzo[cd]indol-6-yl)cyclopropae-1-carboxamide

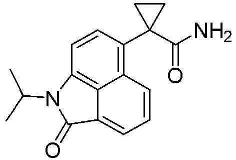

S53 (267 mg, 0.97 mmol) was placed in a single-necked flask, DMSO (2 mL) and K₂CO₃ (67 mg, 0.48 mmol) were added, and the mixture was cooled to 0°C, hydrogen peroxide (0.2 mL, 1.46 mmol, 30% in water) was added dropwise, then heated to room temperature and stirred for 2.0 hours. After the starting materials had completely reacted, water (20 mL) was added to the reaction solution, resulting in the precipitation of a yellow solid. The mixture was filtered, and the filter cake was washed with water and then purified by column chromatography (PE:EA=2:1-1:1) to obtain a yellow solid S54 (158 mg, 55% yield). MS: m/z [M+1]⁺ = 295.38.

### (4) Preparation of Intermediate S55: 6-(1-aminocyclopropyl)-1-isopropylbenzo[cd]indol-2(1H)-one

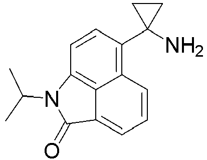

S54 (158 mg, 0.54 mmol) was placed in a single-necked flask, and *t*-BuOH (2 mL) was added. The mixture was cooled to 0 °C. NaClO (0.63 mL, 1.51 mmol) solution and NaOH (3 N, 0.50 mL, 1.51 mmol) solution were added. The mixture was heated to room temperature and stirred for 2 h. After the starting materials had completely reacted, the reaction solution was concentrated under reduced pressure to remove the solvent, and then purified by column chromatography (PE:EA=2:1, 1:1) to obtain a yellow oil S55 (65 mg, 45% yield). MS: m/z [M+1]⁺ = 267.36.

### (5) Preparation of Intermediate S56: tert-butyl 3-(3-((1-(1-isopropyl-2-oxo-1,2-dihydrobenzo[cd]indol-6-yl)-cyclopropyl)carbamoyl)-4-methylphenyl)-3,6-diazabicycl o[3.1.1]heptan-6-carboxylate

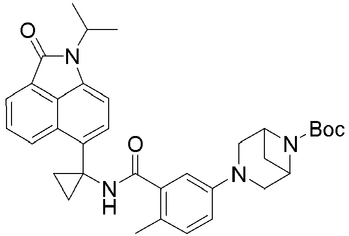

S55 (29 mg, 0.11 mmol) and S25 (41 mg, 0.12 mmol) were placed in a single-necked flask. DMF (1.5 mL), DIPEA (47 mg, 0.37 mmol) and HATU (55 mg, 0.14 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was stirred at room temperature for 20 minutes and filtered. The filter cake was washed with water and purified by column chromatography (PE:EA=3:1, 1:1) to obtain a yellow solid S56 (53 mg, 83% yield). MS: m/z [M+1]⁺ = 581.37.

### (6) Preparation of P16: 5-(3,6-diazabicyclo[3.1.1]heptan-3-yl)-N-(1-(1-isopropyl-2-oxo-1,2-dihydrobenzo[cd]indol-6-yl)cyclopropyl)benzamid e

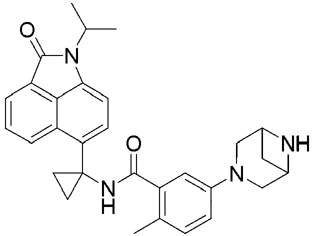

S56 (53 mg, 0.091 mmol) was placed in a single-necked flask. DCM (2.0 mL) and hydrochloric acid (2.0 mL, 4.0 M in 1,4-dioxane) were added. The mixture was stirred at room temperature for 5.5 h. After the starting materials had completely reacted, the reaction solution was concentrated under reduced pressure to remove solvent and obtain a yellow solid mixture. A saturated sodium bicarbonate solution (4 mL) was added to the obtained mixture, extracted three times with DCM, the organic phase was collected and concentrated under reduced pressure, and then purified by column chromatography (PE:EA=1:1; DCM:MeOH=20:1, 10:1) to obtain a bright yellow solid P16 (32 mg, 73% yield). MS: m/z [M+1]⁺ = 481.28.

### Example 17

### Preparation of Compound P17: 5-(3-(4-Hydroxy-4-methylpiperidin-1-yl)azetidin-1-yl)-2-methyl-N-(1-(1-methyl-2-oxo-1,2-dihydrobenzo[cd]indol-6-yl )cyclopropyl)benzamide

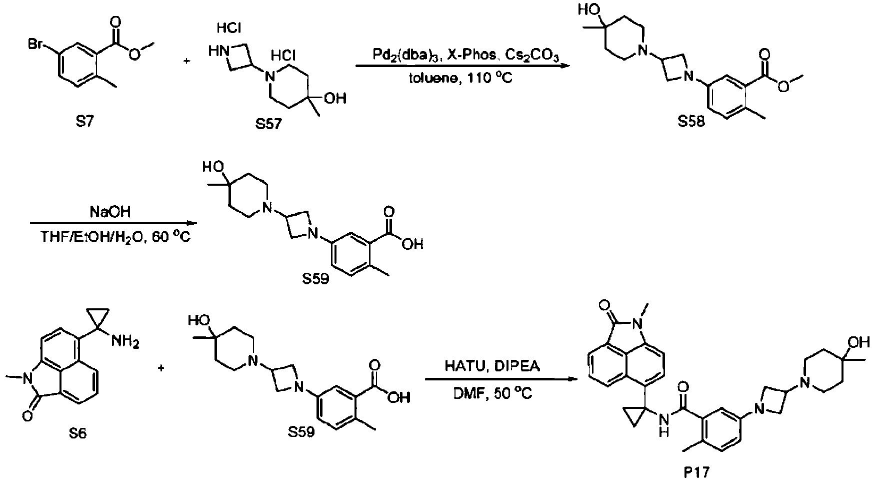

### (1) Preparation of Intermediate S58: Methyl 5-(3-(4-hydroxy-4-methylpiperidin-1-yl)azetidin-1-yl)-2-methylbenzoate

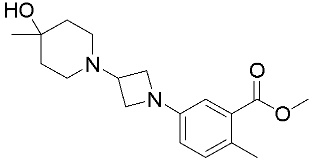

S7 (101 mg, 0.44 mmol) and S57 (129 mg, 0.53 mmol) were placed in a sealed tube. Toluene (3 mL), X-Phos (8 mg, 0.017 mmol), Cs₂CO₃ (717 mg, 2.20 mmol) and Pd₂(dba)₃ (8 mg, 0.0088 mmol) were added. The mixture was bubbled with N₂ for 30 seconds, then heated to 110°C and stirred overnight. After the starting materials had completely reacted, the reaction solution was cooled to room temperature. A saturated ammonium chloride solution (6 mL) was added to the reaction solution. The resulting mixture was extracted three times with DCM, and the organic phases were combined. The combined organic phases were concentrated under reduced pressure and then purified by column chromatography (PE:EA=1:1-1:3) to obtain a pale yellow oil S58 (92 mg, 66% yield). MS: m/z [M+1]⁺ = 319.27.

### (2) Preparation of Intermediate S59: 5-(3-(4-hydroxy-4-methylpiperidin-1-yl)azetidin-1-yl)-2-methylbenzoic acid

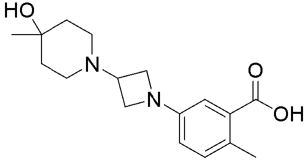

S58 (92 mg, 0.29 mmol) was placed in a single-necked flask. THF (1 mL) and EtOH (1 mL) were added, followed by the addition of a solution of NaOH (58 mg, 1.45 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2.0 M). The resulting mixture was concentrated under reduced pressure to remove the solvent and obtain a solid mixture. The obtained solid mixture was dissolved in DCM, dried with anhydrous sodium sulfate and then filtered, and the filter cake was washed with DCM. The filtrate was concentrated under reduced pressure to obtain a pale yellow solid S59 (88 mg, 100% yield). MS: m/z [M+1]⁺ = 305.31.

### (3) Preparation of P17: 5-(3-(4-hydroxy-4-methylpiperidin-1-yl)azetidin-1-yl)-2-methyl-N-(1-(1-methyl-2-oxo-1,2-dihydrobenzo[cd]indol-6-yl )cyclopropyl)benzamide

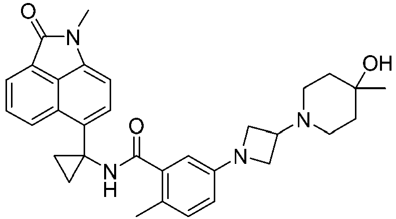

S6 (20 mg, 0.084 mmol) and S59 (30 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was stirred at room temperature for 20 minutes and then filtered. The filter cake was washed with water and then purified by column chromatography (PE:EA=1:1; DCM:MeOH=20:1, 10:1) to obtain a bright yellow solid P17 (27 mg, 61% yield). MS: m/z [M+1]⁺ = 525.25.

### Example 18

### Preparation of Compound P18: 5-(3-(3,3-difluoropyrrol-1-yl)azetidin-1-yl)-2-methyl-N-(1-(1-methyl-2-oxo-1,2-dihydrobenzo[cd]indol-6-yl)cycloprop yl)benzamide

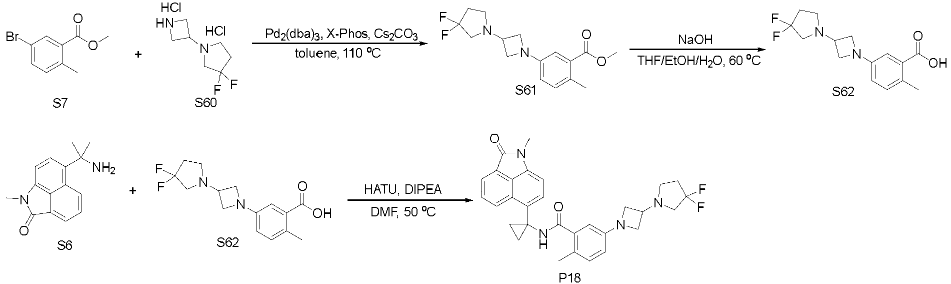

### (1) Preparation of Iintermediate S61: methyl 5-(3-(3,3-difluoropyrrol-1-yl)azetidin-1-yl)-2-methylbenzoate

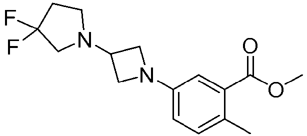

S7 (101 mg, 0.44 mmol) and S60 (125 mg, 0.53 mmol) were placed in a sealed tube. Toluene (3 mL), X-Phos (8 mg, 0.017 mmol), Cs₂CO₃ (717 mg, 2.20 mmol), and Pd₂(dba)₃ (8 mg, 0.0088 mmol) were added. The mixture was bubbled with N₂ for 30 seconds, then heated to 110°C and stirred overnight. After the starting materials had completely reacted, the reaction solution was cooled to room temperature. A saturated ammonium chloride solution (6 mL) was added. The mixture was extracted three times with DCM, and the organic phases were combined. The combined organic phases were concentrated under reduced pressure and then purified by column chromatography (PE:EA=10:1, 7:1) to obtain a pale yellow oil S61 (115 mg, 85% yield). MS: m/z [M+1]⁺ = 311.26.

### (2) Preparation of Intermediate S62: 5-(3-(3,3-difluoropyrrol-1-yl)azetidin-1-yl)-2-methylbenzoic acid

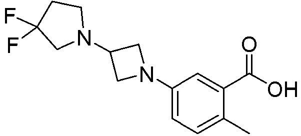

S61 (115 mg, 0.37 mmol) was placed in a single-necked flask. THF (1 mL) and EtOH (1 mL) were added, followed by the addition of a solution of NaOH (74 mg, 1.85 mmol) in H₂O (1 mL). The mixture was heated to 60°C and stirred for 2 hours. After the starting materials had completely reacted, the reaction solution was concentrated under reduced pressure to remove THF and EtOH, and the pH was then adjusted to 4-5 with dilute hydrochloric acid (2.0 M). The resulting mixture was concentrated under reduced pressure to remove the solvent and obtain a solid. The obtained solid was dissolved in DCM, dried with anhydrous sodium sulfate and then filtered, and the filter cake was washed with DCM. The filtrate was concentrated under reduced pressure to obtain a pale yellow solid S62 (109 mg, 100% yield). MS: m/z [M+1]⁺ = 297.31.

### (3) Preparation of P18: 5-(3-(3,3-difluoropyrrol-1-yl)azetidin-1-yl)-2-methyl-N-(1-(1-methyl-2-oxo-1,2-dihydrobenzo[cd]indol-6-yl)cycloprop yl)benzamide

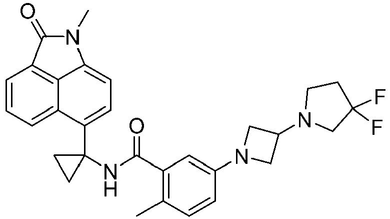

S6 (20 mg, 0.084 mmol) and S62 (30 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was stirred at room temperature for 20 minutes and then filtered. The filter cake was washed with water and then purified by column chromatography (PE:EA=3:1, 1:1) to obtain a bright yellow solid P18 (25 mg, 58% yield). MS: m/z [M+1]⁺ = 517.23.

### Example 19

### Preparation of Compound P19: 2-Methyl-N-(1-(1-methyl-2-oxo-1,2-dihydrobenzo[cd]indol-6-yl)cyclopropyl)-5-(6-methyl-2,6-diazaspiro[3,4]octan-2-yl benzamide

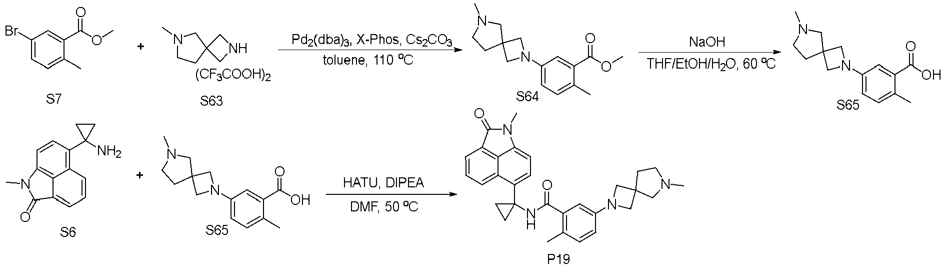

### (1) Preparation of Intermediate S64: methyl 2-methyl-5-(6-methyl-2,6-diazaspiro[3,4]octan-2-yl)benzoate

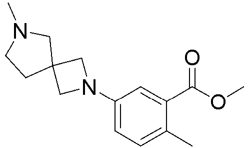

S7 (60 mg, 0.26 mmol) and S63 (111 mg, 0.31 mmol) were placed in a sealed tube. Toluene (2 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (8 mL) was added. The mixture was extracted three times with DCM, and the organic phases were combined. The combined organic phases were concentrated under reduced pressure and then purified by column chromatography (PE:EA=1:1; DCM:MeOH=30:1, 20:1, 10:1) to obtain a pale yellow oil S64 (71 mg, 100% yield). MS: m/z [M+1]⁺ = 275.32.

### (2) Preparation of Intermediate S65: 2-methyl-5-(6-methyl-2,6-diazaspiro[3,4]octan-2-yl)benzoic acid

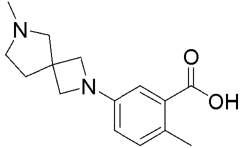

S64 (70 mg, 0.26 mmol) was placed in a single-necked flask. THF (1 mL) and EtOH (1 mL) were added, followed by the addition of a solution of NaOH (52 mg, 1.30 mmol) in H₂O (1 mL). The mixture was heated to 60°C and stirred for 2 hours. After the starting materials had completely reacted, the reaction solution was concentrated under reduced pressure to remove THF and EtOH, and the pH was then adjusted to 4-5 with dilute hydrochloric acid (2.0 M). The resulting mixture was concentrated under reduced pressure to remove the solvent and obtain a solid. The obtained solid was dissolved in DCM, dried with anhydrous sodium sulfate and then filtered, and the filter cake was washed with DCM. The filtrate was concentrated under reduced pressure to obtain a pale yellow solid S65 (68 mg, 100% yield). MS: m/z [M+1]⁺ = 261.31.

### (3) Preparation of P19: 2-methyl-N-(1-(1-methyl-2-oxo-1,2-dihydrobenzo[cd]indol-6-yl)cyclopropyl)-5-(6-methyl-2,6-diazaspiro[3,4]octan-2-y 1 benzamide

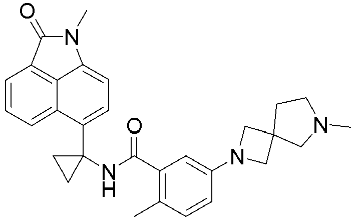

S6 (20 mg, 0.084 mmol) and S65 (26 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was stirred at room temperature for 20 minutes and then filtered, the filter cake was washed with water and then purified by column chromatography (PE:EA=1:1; DCM:MeOH=20:1, 10:1, 8:1) to obtain a bright yellow solid P19 (27 mg, 67% yield). MS: m/z [M+1]⁺ = 481.23.

### Example 20

### Preparation of Compound P20: 2-Methyl-N-(1-(1-methyl-2-oxo-1,2-dihydrobenzo[cd]indol-6-yl)cyclopropyl)-5-((1R,5S)-3-methyl-3,6-diazabicyclo[3. 2.0]heptan-6-yl)benzamide

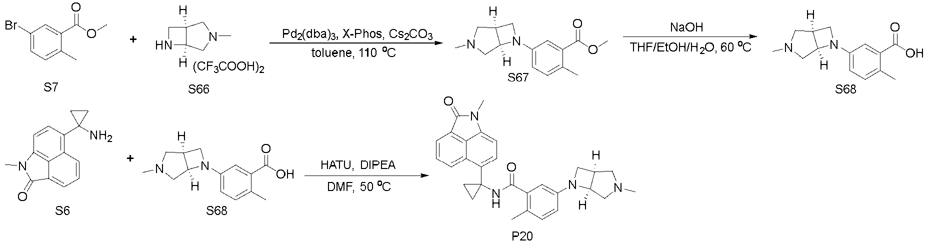

### (1) Preparation of Intermediate S67: methyl 2-methyl-5-((1S,5R)-3-methyl-3,6-diazabicyclo[3.2.0]heptan-6-yl)benzoate

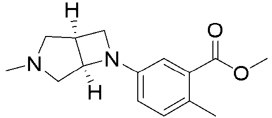

S7 (60 mg, 0.26 mmol) and S66 (105 mg, 0.31 mmol) were placed in a sealed tube. Toluene (2 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (8 mL) was added. The mixture was extracted three times with DCM, and the organic phases were combined. The combined organic phases were concentrated under reduced pressure and then purified by column chromatography (DCM:MeOH=20: 1, 10:1) to obtain a pale yellow oil S67 (67 mg, 99% yield). MS: m/z [M+1]⁺ = 261.32.

### (2) Preparation of Intermediate S68: 2-methyl-5-((1S,5R)-3-methyl-3,6-diazabicyclo[3.2.0]heptan-6-yl)benzoic acid

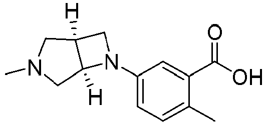

S67 (67 mg, 0.26 mmol) was placed in a single-necked flask. THF (1 mL) and EtOH (1 mL) were added, followed by the addition of a solution of NaOH (52 mg, 1.30 mmol) in H₂O (1 mL). The mixture was heated to 60°C and stirred for 2 hours. After the starting materials had completely reacted, the reaction solution was concentrated under reduced pressure to remove THF and EtOH, and the pH was then adjusted to 4-5 with dilute hydrochloric acid (2.0 M). The resulting mixture was concentrated under reduced pressure to remove the solvent and obtain a solid. The obtained solid was dissolved in DCM, dried with anhydrous sodium sulfate and then filtered, and the filter cake was washed with DCM. The filtrate was concentrated under reduced pressure to obtain a pale yellow solid S68 (64 mg, 100% yield). MS: m/z [M+1]⁺ = 247.33.

### (3) Preparation of P20: 2-methyl-N-(1-(1-methyl-2-oxo-1,2-dihydrobenzo[cd]indol-6-yl)cyclopropyl}-5-((1R,5S)-3-methyl-3,6-diazabicyclo[3. 2.0]heptan-6-yl)benzamide

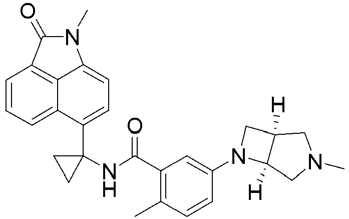

S6 (20 mg, 0.084 mmol) and S68 (25 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was stirred at room temperature for 20 minutes and then filtered, the filter cake was washed with water and then purified by column chromatography (PE:EA=1:1; DCM:MeOH=20:1, 10:1, 8:1) to obtain a bright yellow solid P20 (22 mg, 56% yield). MS: m/z [M+1]⁺ = 467.23.

### Example 21

### Preparation of Compound P21: N-(1-(1-isopropyl-2-oxo-1,2-dihydrobenzo[cd]indol-6-yl)cyclopropyl)-2-methyl-5-(6-methyl-3,6-diazabicyclo[3.1.1]he ptan-3-yl)benzamide

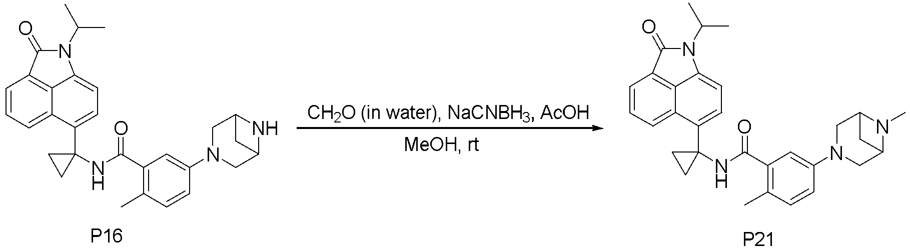

### (1) Preparation of P21: N-(1-(1-isopropyl-2-oxo-1,2-dihydrobenzo[cd]indol-6-yl)cyclopropyl)-2-methyl-5-(6-methyl-3,6-diazabicyclo[3.1.1]he ptan-3-yl)benzamide

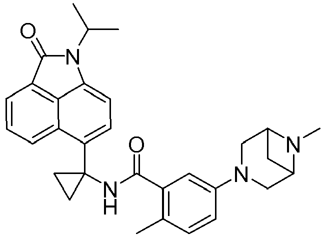

P16 (20 mg, 0.042 mmol) was placed in a single-necked flask. MeOH (1.0 mL), formaldehyde aqueous solution (0.30 mL) and acetic acid (7 mg, 0.11 mmol) were added, and finally NaCNBH₃ (4 mg, 0.063 mmol) was added. The mixture was stirred at room temperature for 2 hours. After the starting materials had completely reacted, a saturated sodium bicarbonate solution (5 mL) was added to the reaction solution, extracted three times with DCM, and the organic phases were combined. The combined organic phases were concentrated under reduced pressure to obtain a yellow solid. The yellow solid was then purified by column chromatography (PE:EA = 1:1; DCM:MeOH = 30:1, 20:1) to obtain a bright yellow solid P21 (14 mg, 69% yield). MS: m/z [M+1]⁺ = 495.35.

### Example 22

### Preparation of Compound P22: 2-methyl-N-(1-(1-methyl-2-oxo-1,2-dihydrobenzo[cd]indol-6-yl)cyclopropyl)-5-(1-methylpiperidin-4-yl)benzamide

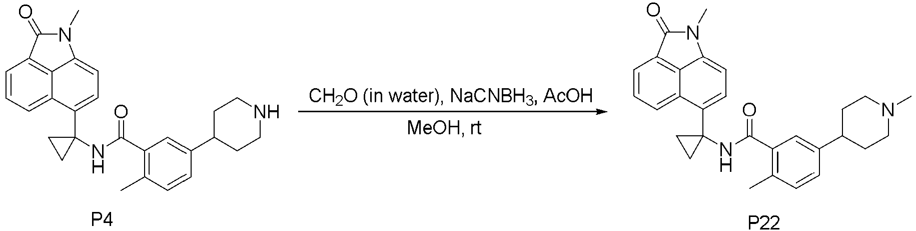

### (1) Preparation of P22: 2-methyl-N-(1-(1-methyl-2-oxo-1,2-dihydrobenzo[cd]indol-6-yl)cyclopropyl)-5-(1-methylpiperidin-4-yl)benzamide

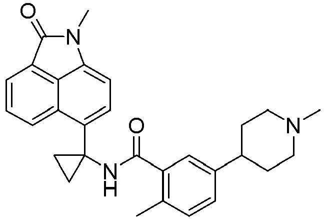

P4 (26 mg, 0.059 mmol) was placed in a single-necked flask. MeOH (1.0 mL), formaldehyde aqueous solution (0.50 mL) and acetic acid (9 mg, 0.15 mmol) were added, and finally NaCNBH₃ (6 mg, 0.089 mmol) was added. The mixture was stirred at room temperature for 2 hours. After the starting materials had completely reacted, a saturated sodium bicarbonate solution (5 mL) was added to the reaction solution, extracted three times with DCM, and the organic phases were combined. The combined organic phases were concentrated under reduced pressure to obtain a yellow solid. The yellow solid was then purified by column chromatography (PE:EA = 1:1; DCM:MeOH = 20:1, 10:1) to obtain a bright yellow solid P22 (15 mg, 54% yield). MS: m/z [M+1]⁺ = 454.29.

### Example 23

### Preparation of Compound M23: 2-Methyl-N-(1-(1-methyl-2-oxo-1,2-dihydrobenzo[cd]indol-6-yl)cyclopropyl)-5-(3-(4-methylpiperazin-1-yl)azetidin-1-yl)benzamide

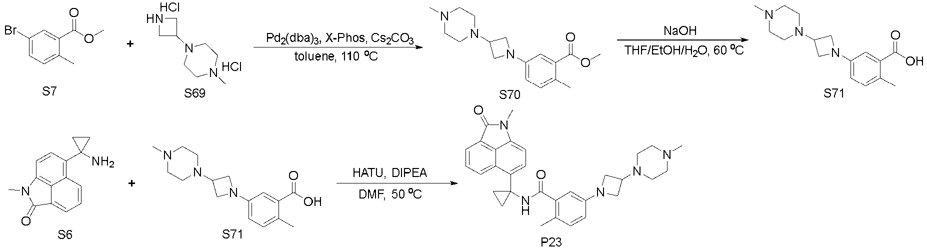

### (1) Preparation of Intermediate S70: methyl 2-methyl-5-(3-(4-methylpiperazin-1-yl)azetidin-1-yl)benzoate

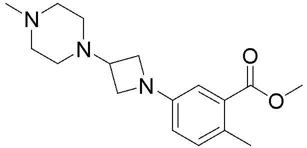

S7 (50 mg, 0.22 mmol) and S69 (60 mg, 0.26 mmol) were placed in a sealed tube. Toluene (2 mL), X-Phos (4 mg, 0.0085 mmol), Cs₂CO₃ (358 mg, 1.10 mmol), and Pd₂(dba)₃ (4 mg, 0.0044 mmol) were added. The mixture was bubbled with N₂ for 30 seconds, then heated to 110°C and stirred overnight. After the starting materials had completely reacted, the reaction solution was cooled to room temperature. Water (6 mL) was added, the mixture was extracted three times with DCM, and the organic phases were combined. The combined organic phases were concentrated under reduced pressure and then purified by column chromatography (PE:EA=1:1; DCM:MeOH=20:1) to obtain a white solid S70 (59 mg, 88% yield). MS: m/z [M+1]⁺ = 304.21.

### (2) Preparation of Intermediate S71: 2-methyl-5-(3-(4-methylpiperazin-1-yl)azetidin-1-yl)benzoic acid

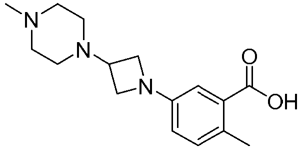

S70 (59 mg, 0.19 mmol) was placed in a single-necked flask. THF (1.0 mL) and EtOH (1.0 mL) were added, followed by the addition of a solution of NaOH (39 mg, 0.97 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was concentrated under reduced pressure to remove the solvent and obtain a solid mixture. The obtained solid mixture was dissolved in DCM, dried with anhydrous sodium sulfate and then filtered, and the filter cake was washed with DCM. The filtrate was concentrated under reduced pressure to obtain a white solid S71 (55 mg, 100% yield). MS: m/z [M+1]⁺ = 290.22.

### (3) Preparation of P23: 2-methyl-N-(1-(1-methyl-2-oxo-1,2-dihydrobenzo[cd]indol-6-yl)cyclopropyl)-5-(3-(4-methylpiperazin-1-yl)azetidin-1-yl)benzamide

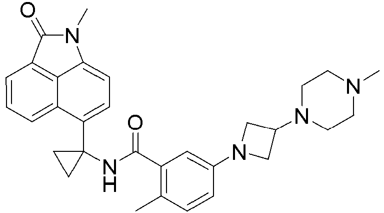

S6 (20 mg, 0.084 mmol) and S71 (29 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was extracted three times with DCM, and the organic phases were combined. The combined organic phases were concentrated under reduced pressure and then purified by column chromatography (PE:EA=1:1; DCM:MeOH=20:1; 10:1) to obtain a bright yellow solid P23 (35 mg, 81% yield). MS: m/z [M+1]⁺ = 510.33.

### Example 24

### Preparation of Compound P24: 2-Methyl-N-(1-(1-methyl-2-oxo-1,2-dihydrobenzo[cd]indol-6-yl)cyclopropyl)-5-(3-(piperidin-1-yl)azetidin-1-yl)benza mide

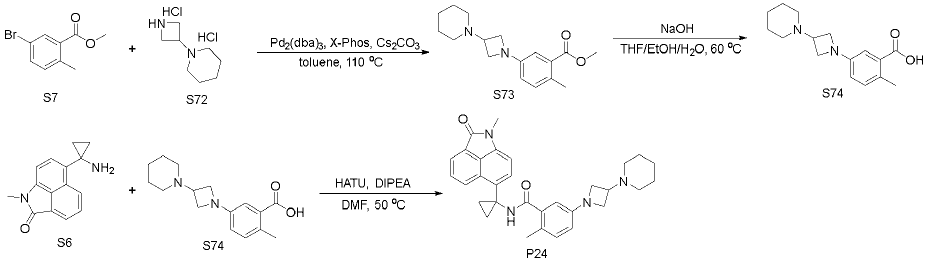

### (1) Preparation of Intermediate S73: methyl 2-methyl-5-(3-(piperidin-1-yl)azetidin-1-yl)benzoate

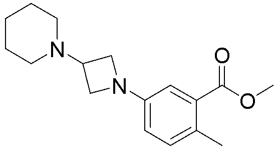

S7 (60 mg, 0.26 mmol) and S72 (66 mg, 0.31 mmol) were placed in a sealed tube. Toluene (2 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=4:1; 3:1) to obtain a colorless oil S73 (53 mg, 71% yield). MS: m/z [M+1]⁺ = 289.21.

### (2) Preparation of Intermediate S74: 2-methyl-5-(3-(piperidin-1-yl)azetidin-1-yl)benzoic acid

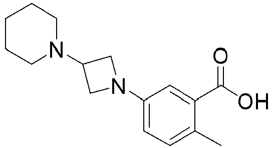

S73 (53 mg, 0.18 mmol) was placed in a single-necked flask. THF (1.0 mL) and EtOH (1.0 mL) were added, followed by the addition of a solution of NaOH (37 mg, 0.92 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a white solid S74 (49 mg, 100% yield). MS: m/z [M+1]⁺ = 275.26.

### (3) Preparation of P24: 2-methyl-N-(1-(1-methyl-2-oxo-1,2-dihydrobenzo[cd]indol-6-yl)cyclopropyl)-5-(3-(piperidin-1-yl)azetidin-1-yl)benza mide

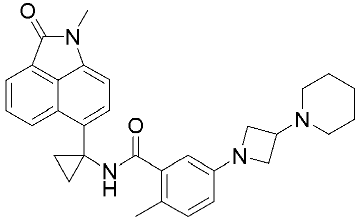

S6 (20 mg, 0.084 mmol) and S74 (27 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1, DCM:MeOH=20:1) to obtain a bright yellow solid P24 (28 mg, 67% yield). MS: m/z [M+1]⁺ = 495.32.

### Example 25

### Preparation of Compound P25: (R)-5-(hexahydropyrrolo[1,2-a]pyrazin-2(1H)-yl)-2-methyl-N-(1-(1-methyl-2-oxo-1,2-dihydrobenzo[cd]indol-6-yl)cycl opropyl)benzamide

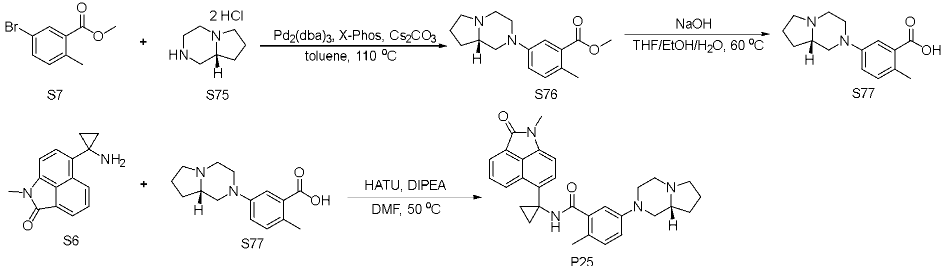

### (1) Preparation of Intermediate S76: methyl (S)-5-(hexahydropyrrolo[1,2-a]pyrazin-2(1H)-yl)-2-methylbenzoate

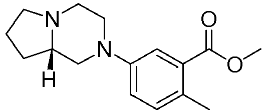

S7 (60 mg, 0.26 mmol) and S75 (62 mg, 0.31 mmol) were placed in a sealed tube. Toluene (2 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1, 1:3) to obtain a white solid S76 (65 mg, 92% yield). MS: m/z [M+1]⁺ = 275.21.

### (2) Preparation of Intermediate S77: (S)-5-(hexahydropyrrolo[1,2-a]pyrazin-2(1H)-yl)-2-methylbenzoic acid

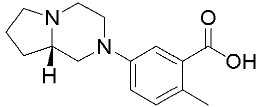

S76 (65 mg, 0.24 mmol) was placed in a single-necked flask. THF (1.0 mL) and EtOH (1.0 mL) were added, followed by the addition of a solution of NaOH (48 mg, 1.20 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a white solid S77 (62 mg, 100% yield). MS: m/z [M+1]⁺ = 261.26.

### (3) Preparation of P25: (R)-5-(hexahydropyrrolo[1,2-a]pyrazin-2(1H)-yl)-2-methyl-N-(1-(1-methyl-2-oxo-1,2-dihydrobenzo[cd]indol-6-yl)cycl opropyl)benzamide

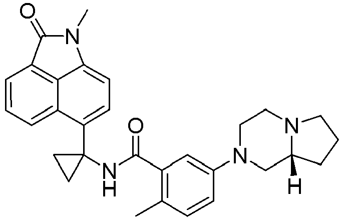

S6 (20 mg, 0.084 mmol) and S77 (26 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1, DCM:MeOH=20:1) to obtain a bright yellow solid P25 (16 mg, 40% yield). MS: m/z [M+1]⁺ = 481.32.

### Example 26

### Preparation of Compound P26: 5-(3-(4-fluoropiperidin-1-yl)azetidin-1-yl)-2-methyl-N-(1-(1-methyl-2-oxo-1,2-dihydrobenzo[cd]indol-6-yl)cyclopropyl )benzamide

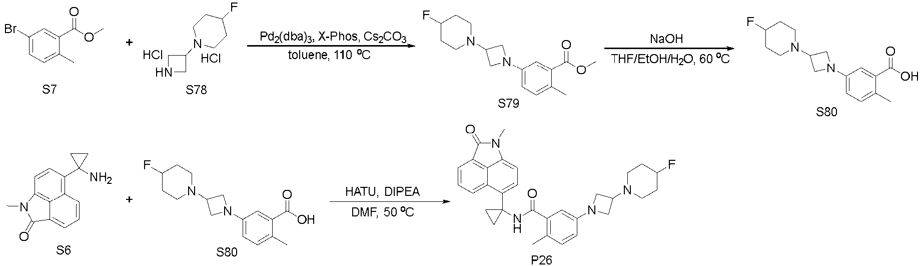

### (1) Preparation of Intermediate S79: Methyl 5-(3-(4-fluoropiperidin-1-yl)azetidin-1-yl)-2-methylbenzoate

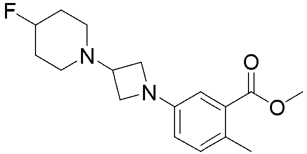

S7 (60 mg, 0.26 mmol) and S78 (72 mg, 0.31 mmol) were placed in a sealed tube. Toluene (2 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=5:1, 3:1) to obtain a white solid S79 (72 mg, 90% yield). MS: m/z [M+1]⁺ = 307.21.

### (2) Preparation of Intermediate S80: 5-(3-(4-fluoropiperidin-1-yl)azetidin-1-yl)-2-methylbenzoic acid

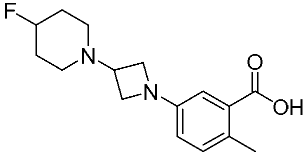

S79 (72 mg, 0.23 mmol) was placed in a single-necked flask. THF (1.0 mL) and EtOH (1.0 mL) were added, followed by the addition of a solution of NaOH (46 mg, 1.15 mmol) in H₂O (1 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a white solid S80 (67 mg, 100% yield). MS: m/z [M+1]⁺ = 293.26.

### (3) Preparation of P26: 5-(3-(4-fluoropiperidin-1-yl)azetidin-1-yl)-2-methyl-N-(1-(1-methyl-2-oxo-1,2-dihydrobenzo[cd]indol-6-yl)cyclopropyl )benzamide

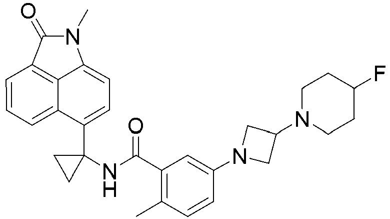

S6 (20 mg, 0.084 mmol) and S80 (29 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=3:1, 1:1, 1:2) to obtain a bright yellow solid P26 (27 mg, 63% yield). MS: m/z [M+1]⁺ = 513.32.

### Example 27

### Preparation of Compound P27: N-(1-(1-ethyl-2-oxo-1,2-dihydrobenzo[cd]indol-6-yl)cyclopropyl)-2-methyl-5-(4-methylpiperazin-1-yl)benzamide

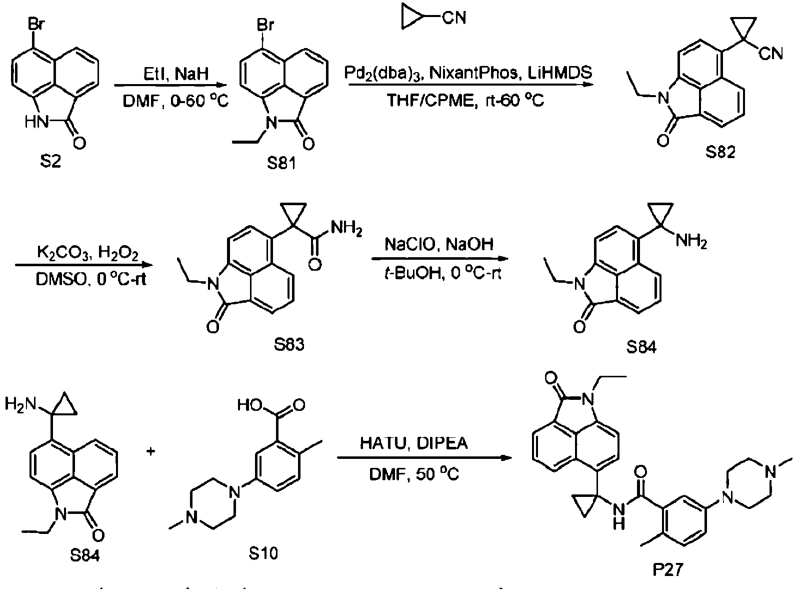

### (1) Preparation of Intermediate S81: 6-bromo-1-ethylbenzo[cd]indol-2(1H)-one

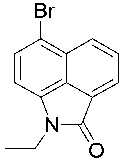

S2 (1000 mg, 4.02 mmol) was placed in a single-necked flask, DMF (10 mL) was added, and the mixture was cooled to 0°C. NaH (241 mg, 6.03 mmol, 60% dispersed in mineral oil) was added. The mixture was stirred for 0.5 h. EtI (753 mg, 4.82 mmol) was added. The mixture was heated to 60°C and stirred overnight. Then, water (50 mL) was added to the reaction solution. The resulting mixture was extracted three times with EA, and the organic phases were combined. The combined organic phases were concentrated under reduced pressure and then purified by column chromatography (PE:EA=20:1) to obtain a yellow solid S81 (1000 mg, 90% yield). MS: m/z [M+1]⁺ = 277.21.

### (2) Preparation of Intermediate S82: 1-(1-ethyl-2-oxo-1,2-dihydrobenzo[cd]indol-6-yl)cyclopropyl-1-carbonitrile

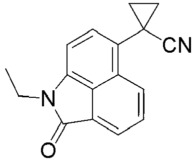

S81 (1000 mg, 3.60 mmol) and cyclopropyl cyanide (364 mg, 5.40 mmol) were placed in a three-necked flask, followed by the addition of THF (15 mL), CPME (15 mL), Pd₂(dba)₃ (165 mg, 0.18 mmol) and Nixantphos (198 mg, 0.36 mmol). The mixture was bubbled with nitrogen for 30 seconds and stirred at room temperature for 0.5 h. LiHMDS (7.2 mL, 7.20 mmol, 1M in tetrahydrofuran) was added dropwise. After addition was complete, the mixture was heated to 60 °C and stirred for 1.0 hour. After the starting materials had completely reacted, the reaction solution was cooled to room temperature. A saturated ammonium chloride solution (50 mL) was added. The resulting mixture was extracted three times with EA, and the organic phases were combined. The combined organic phases were concentrated under reduced pressure and then purified by column chromatography (PE:EA=5:1, 3:1, 2:1) to obtain an orange-yellow solid S82 (360 mg, 38% yield). MS: m/z [M+1]⁺ = 263.26.

### (3) Preparation of Intermediate S83: 1-(1-ethyl-2-oxo-1,2-dihydrobenzo[cd]indol-6-yl)cyclopropyl-1-carboxamide

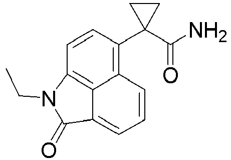

S82 (360 mg, 1.38 mmol) was placed in a single-necked flask. DMSO (4 mL) and K₂CO₃ (95 mg, 0.69 mmol) were added, and the mixture was cooled to 0 °C. H₂O₂ (170 µL, 2.07 mmol) was then added dropwise. The mixture was heated to room temperature and stirred for 1.5 h. After the starting materials had completely reacted, water was added to the reaction solution, resulting in the precipitation of a yellow solid. The mixture was stirred at room temperature for 20 minutes and then filtered, the filter cake was washed with water and then purified by column chromatography (PE:EA=1:1, DCM:MeOH=30:1) to obtain a yellow solid S83 (137 mg, 35% yield). MS: m/z [M+1]⁺ = 281.26.

### (4) Preparation of Intermediate S84: 6-(1-aminocyclopropyl)-1-ethylbenzo[cd]indol-2(1H)-one

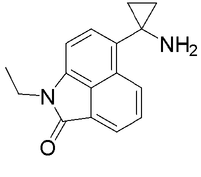

S83 (137 mg, 0.49 mmol) was placed in a single-necked flask, and t-BuOH (4 mL) was added. The mixture was cooled to 0°C. NaClO (0.83 mL, 1.37 mmol) solution and NaOH (3 N, 0.45 mL, 1.37 mmol) solution were added. The mixture was heated to room temperature and stirred for 3 hours. After the starting materials had completely reacted, the reaction solution was concentrated under reduced pressure to remove the solvent and obtain a solid mixture. The obtained solid mixture was purified by column chromatography (PE:EA=1:1, DCM:MeOH=30:1) to obtain a yellow solid S84 (98 mg, 79% yield). MS: m/z [M+1]⁺ = 281.26.

### (5) Preparation of P27: N-(1-(1-ethyl-2-oxo-1,2-dihydrobenzo[cd]indol-6-yl)cyclopropyl)-2-methyl-5-(4-methylpiperazin-1-yl)benzamide

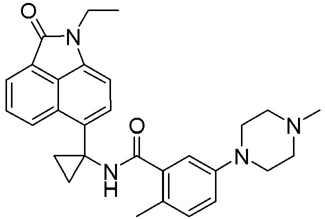

S84 (20 mg, 0.079 mmol) and S10 (22 mg, 0.094 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P27 (8 mg, 22% yield). MS: m/z [M+1]⁺ = 469.32.

### Example 28

### Preparation of Compound P28: N-(1-(1-Ethyl-2-oxo-1,2-dihydrobenzo[cd]indol-6-yl)cyclopropyl)-5-(3-(4-hydroxy-4-methylpiperidin-1-yl)azetidin-1-y l)-2-methyl-benzamide

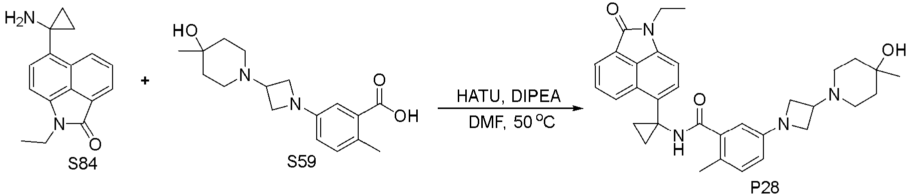

### (1) Preparation of P28: N-(1-(1-ethyl-2-oxo-1,2-dihydrobenzo[cd]indol-6-yl)cyclopropyl)-5-(3-(4-hydroxy-4-methylpiperidin-1-yl)azetidin-1-y 1)-2-methyl-benzamide

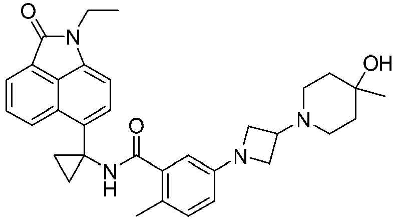

S84 (20 mg, 0.079 mmol) and S59 (29 mg, 0.094 mmol) were placed in a single-necked flask. DMF (1.5 mL), DIPEA (34 mg, 0.26 mmol) and HATU (39 mg, 0.10 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and purified by column chromatography (PE:EA=1:1; DCM:MeOH=20:1, 10:1) to obtain a bright yellow solid P28 (16 mg, 38% yield). MS: m/z [M+1]⁺ = 539.32.

### Example 29

### Preparation of Compound P29: N-(1-(1-ethyl-2-oxo-1,2-dihydrobenzo[cd]indol-6-yl)cyclopropyl)-5-(3-(4-hydroxy-4-methylpiperidin-1-yl)azetidin-1-y 1)-2-methyl-benzamide

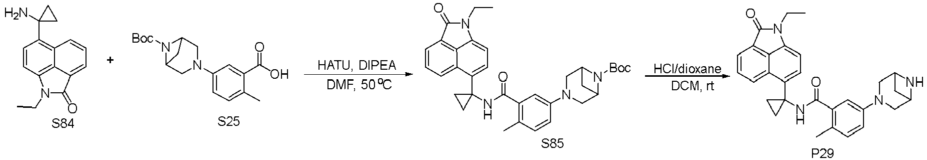

### (1) Preparation of Intermediate S85: tert-butyl 3-(3-((1-(1-ethyl-2-oxo-1,2-dihydrobenzo[cd]indol-6-yl)cyclopropyl)carbamoyl-4-methylphenyl)-3,6-diazabicyclo[3.1. 1]heptan-6-carboxylate

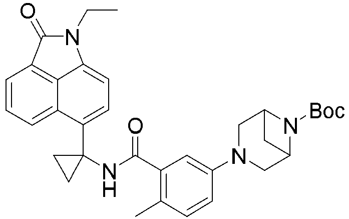

S84 (20 mg, 0.079 mmol) and S25 (32 mg, 0.095 mmol) were placed in a single-necked flask. DMF (1.5 mL), DIPEA (34 mg, 0.26 mmol) and HATU (39 mg, 0.10 mmol) were added. The mixture was heated to 50°C and stirred for 1.0 h. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and purified by column chromatography (PE:EA=3:1, 1:1, 1:2) to obtain a yellow solid S85 (27 mg, 60% yield). MS: m/z [M+1]⁺ = 567.32.

### (2) Preparation of P29: N-(1-(1-ethyl-2-oxo-1,2-dihydrobenzo[cd]indol-6-yl)cyclopropyl)-5-(3-(4-hydroxy-4-methylpiperidin-1-yl)azetidin-1-y l)-2-methyl-benzamide

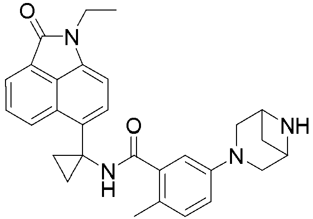

S85 (27 mg, 0.048 mmol) was placed in a single-necked flask. DCM (2.0 mL) and hydrochloric acid (1.0 mL, 4.0 M in 1,4-dioxane) were added. The mixture was stirred at room temperature for 1.0 h. After the starting materials had completely reacted, the reaction solution was concentrated under reduced pressure to remove the solvent and obtain a yellow solid mixture. A saturated sodium bicarbonate solution (4 mL) was added to the obtained mixture, and the mixture was extracted three times with DCM. The organic phase was collected and concentrated under reduced pressure, then purified by column chromatography (DCM:MeOH=20:1, 10:1, 8:1) to obtain a bright yellow solid P29 (15 mg, 70% yield). MS: m/z [M+1]⁺ = 467.32.

### Example 30

### Preparation of Compound P30: 5-(3-(3-fluoropyrrolidin-1-yl)azetidin-1-yl)-2-methyl-N-(1-(1-methyl-2-oxo-1,2-dihydrobenzo[cd]indol-6-yl)cycloprop yl)benzamide

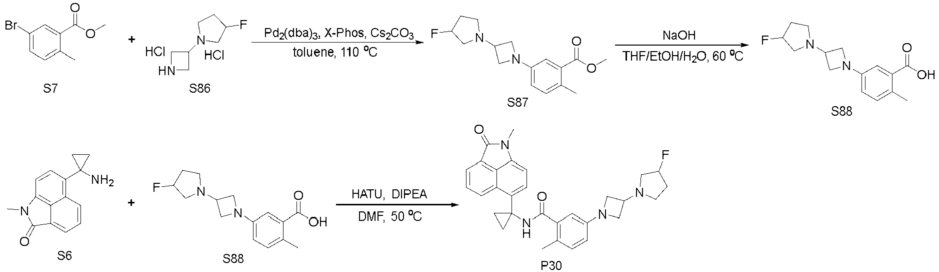

### (1) Preparation of Intermediate S87: methyl 5-(3-(3-fluoropyrrolidin-1-yl)azetidin-1-yl)-2-methylbenzoate

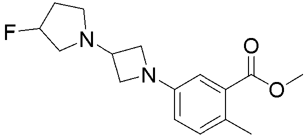

S7 (60 mg, 0.26 mmol) and S86 (67 mg, 0.31 mmol) were placed in a sealed tube. Toluene (2 mL), X-Phos (5 mg, 0.010 mmol), Cs₂CO₃ (424 mg, 1.30 mmol) and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added to the reaction solution. The resulting mixture was extracted three times with DCM, and the organic phases were combined. The combined organic phases were concentrated under reduced pressure to remove solvent and then purified by column chromatography (PE:EA=5:1, 3:1) to obtain a white solid S87 (70 mg, 92% yield). MS: m/z [M+1]⁺ = 293.21.

### (2) Preparation of Intermediate S88: 5-(3-(3-fluoropyrrolidin-1-yl)azetidin-1-yl)-2-methylbenzoic acid

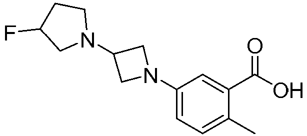

S87 (70 mg, 0.24 mmol) was placed in a single-necked flask. THF (1.0 mL) and EtOH (1.0 mL) were added, followed by the addition of a solution of NaOH (48 mg, 1.20 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was concentrated under reduced pressure to remove the solvent and obtain a solid mixture. The obtained solid mixture was dissolved in DCM, dried with anhydrous sodium sulfate, and then filtered. The filter cake was washed with DCM. The filtrate was concentrated under reduced pressure to obtain a white solid S88 (67 mg, 100% yield). MS: m/z [M+1]⁺ = 279.26.

### (3) Preparation of P30: 5-(3-(3-fluoropyrrolidin-1-yl)azetidin-1-yl)-2-methyl-N-(1-(1-methyl-2-oxo-1,2-dihydrobenzo[cd]indol-6-yl)cycloprop yl)benzamide

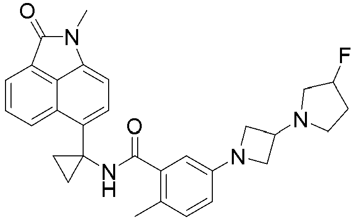

S6 (20 mg, 0.084 mmol) and S88 (28 mg, 0.10 mmol) were placed in a single-necked flask. DMF (1.5 mL), DIPEA (36 mg, 0.28 mmol) and HATU (43 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was extracted three times with DCM, and the organic phases were combined. The combined organic phases were concentrated under reduced pressure and then purified by column chromatography (PE:EA=3:1, 1:1, 1:2) to obtain a bright yellow solid P30 (31 mg, 75% yield). MS: m/z [M+1]⁺ = 499.32.

### Example 31

### Preparation of Compound P31: 5-(3-(4,4-difluoropiperidin-1-yl)azetidin-1-yl)-2-methyl-N-(1-(1-methyl-2-oxo-1,2-dihydrobenzo[cd]indol-6-yl)cyclopr opyl)benzamide

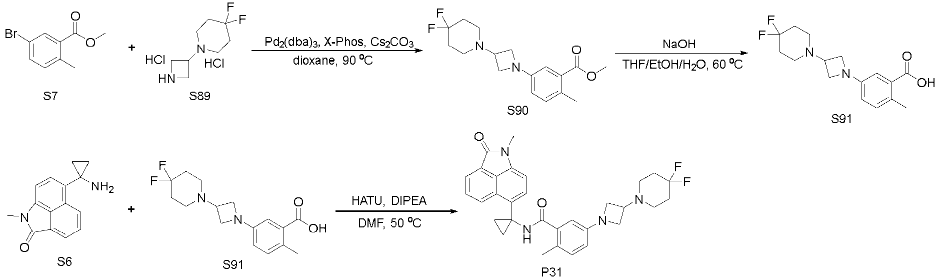

### (1) Preparation of Intermediate S90: methyl 5-(3-(4,4-difluoropiperidin-1-yl)azetidin-1-yl)-2-methylbenzoate

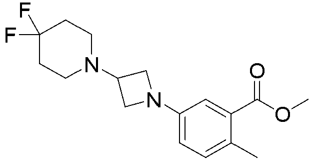

S7 (54 mg, 0.24 mmol) and S89 (50 mg, 0.24 mmol) were placed in a sealed tube. Dioxane (2 mL), X-Phos (4 mg, 0.0094 mmol), Cs₂CO₃ (383 mg, 1.17 mmol) and Pd₂(dba)₃ (4 mg, 0.0047 mmol) were added. The mixture was bubbled with N₂ for 30 seconds, then heated to 90°C and stirred overnight. After the starting materials had completely reacted, the reaction solution was cooled to room temperature. Water (6 mL) was added, the mixture was extracted three times with DCM, and the organic phases were combined. The combined organic phases were concentrated under reduced pressure to remove solvent and then purified by column chromatography (PE:EA=5:1, 3:1) to obtain a white solid S90 (58 mg, 76% yield). MS: m/z [M+1]⁺ = 325.21.

### (2) Preparation of Intermediate S91: 5-(3-(4,4-difluoropiperidin-1-yl)azetidin-1-yl)-2-methylbenzoic acid

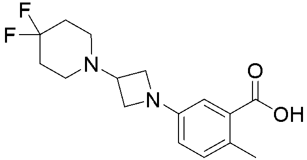

S90 (58 mg, 0.18 mmol) was placed in a single-necked flask. THF (1.0 mL) and EtOH (1.0 mL) were added, followed by the addition of a solution of NaOH (36 mg, 0.90 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was concentrated under reduced pressure to remove the solvent and obtain a solid mixture. The obtained solid mixture was dissolved in DCM, dried with anhydrous sodium sulfate, and then filtered. The filter cake was washed with DCM. The filtrate was concentrated under reduced pressure to obtain a white solid S91 (56 mg, 100% yield). MS: m/z [M+1]⁺ = 311.26.

### (3) Preparation of P31: 5-(3-(4,4-difluoropiperidin-1-yl)azetidin-1-yl)-2-methyl-N-(1-(1-methyl-2-oxo-1,2-dihydrobenzo[cd]indol-6-yl)cyclopr opyl)benzamide

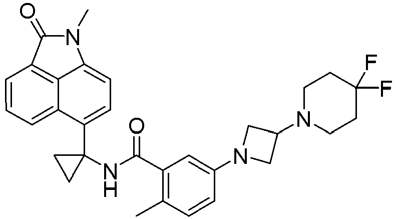

S6 (18 mg, 0.075 mmol) and S91 (28 mg, 0.090 mmol) were placed in a single-necked flask. DMF (1.5 mL), DIPEA (32 mg, 0.25 mmol), and HATU (37 mg, 0.098 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was extracted three times with DCM, and the organic phases were combined. The combined organic phases were concentrated under reduced pressure and then purified by column chromatography (PE:EA=3:1, 1:1, 1:2) to obtain a bright yellow solid P31 (22 mg, 55% yield). MS: m/z [M+1]⁺ = 531.32.

### Example 32

### Preparation of Compound P32: 5-(3-(4-hydroxypiperidin-1-yl)azetidin-1-yl)-2-methyl-N-(1-(1-methyl-2-oxo-1,2-dihydrobenzo[cd]indol-6-yl)cyclopro pyl)benzamide

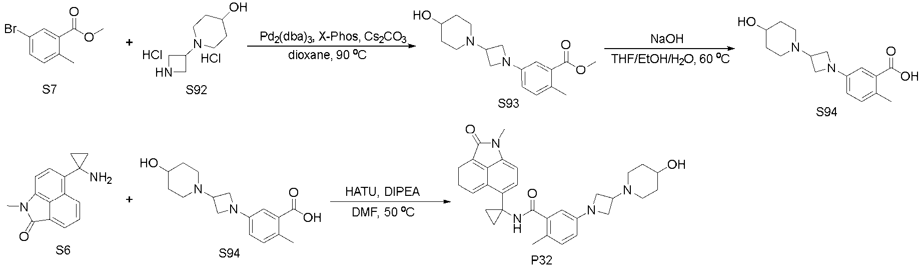

### (1) Preparation of Intermediate S93: Methyl 5-(3-(4-hydroxypiperidin-1-yl)azetidin-1-yl)-2-methylbenzoate

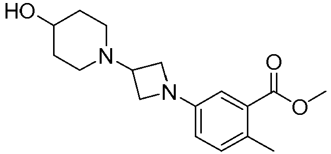

S7 (50 mg, 0.22 mmol) and S92 (50 mg, 0.22 mmol) were placed in a sealed tube. Dioxane (2 mL), X-Phos (4 mg, 0.0088 mmol), Cs₂CO₃ (355 mg, 1.09 mmol), and Pd₂(dba)₃ (4 mg, 0.0044 mmol) were added. The mixture was bubbled with N₂ for 30 seconds, then heated to 90°C and stirred overnight. After the starting materials had completely reacted, the reaction solution was cooled to room temperature. A saturated ammonium chloride solution (6 mL) was added. The mixture was extracted three times with DCM, and the organic phases were combined. The combined organic phases were concentrated under reduced pressure to remove solvent and then purified by column chromatography (PE:EA=3:1, 1:1) to obtain a colorless oil S93 (69 mg, 100% yield). MS: m/z [M+1]⁺ = 305.21.

### (2) Preparation of Intermediate S94: 5-(3-(4-hydroxypiperidin-1-yl)azetidin-1-yl)-2-methylbenzoic acid

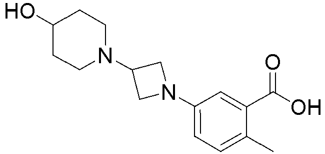

S93 (69 mg, 0.22 mmol) was placed in a single-necked flask. THF (1.0 mL) and EtOH (1.0 mL) were added, followed by the addition of a solution of NaOH (45 mg, 1.13 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S94 (63 mg, 100% yield). MS: m/z [M+1]⁺ = 291.26.

### (3) Preparation of P32: 5-(3-(4-Hydroxypiperidin-1-yl)azetidin-1-yl)-2-methyl-N-(1-(1-methyl-2-oxo-1,2-dihydrobenzo[cd]indol-6-yl)cyclopro pyl)benzamide

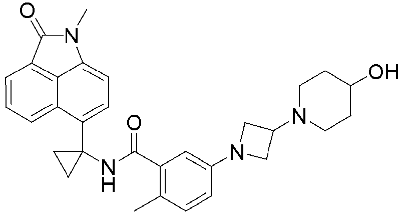

S6 (13 mg, 0.054 mmol) and S94 (19 mg, 0.065 mmol) were placed in a single-necked flask. DMF (1.5 mL), DIPEA (23 mg, 0.18 mmol), and HATU (27 mg, 0.070 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1, DCM:MeOH=20:1, 10:1) to obtain a bright yellow solid P32 (10 mg, 37% yield). MS: m/z [M+1]⁺ = 511.32.

### Example 33

### Preparation of Compound P33: 5-(3-(2,2-dimethylmorpholinyl)azetidin-1-yl)-2-methyl-N-(1-(1-methyl-2-oxo-1,2-dihydrobenzo[cd]indol-6-yl)cyclopro pyl)benzamide

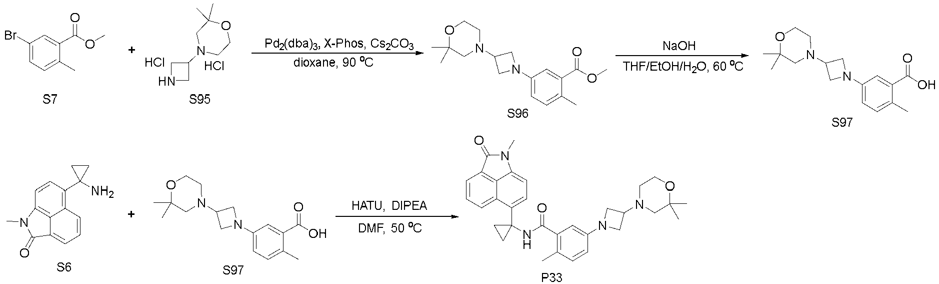

### (1) Preparation of Intermediate S96: methyl 5-(3-(2,2-dimethylmorpholinyl)azetidin-1-yl)-2-methylbenzoate

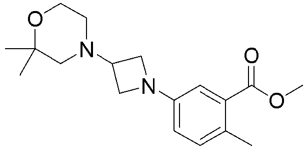

S7 (29 mg, 0.13 mmol) and S95 (50 mg, 0.13 mmol) were placed in a sealed tube. Dioxane (2 mL), X-Phos (2 mg, 0.0050 mmol), Cs₂CO₃ (205 mg, 0.63 mmol), and Pd₂(dba)₃ (2 mg, 0.0025 mmol) were added. The mixture was bubbled with N₂ for 30 seconds, then heated to 90°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=3:1, 1:1) to obtain a pale yellow oil S96 (35 mg, 87% yield). MS: m/z [M+1]⁺ = 319.21.

### (2) Preparation of Intermediate S97: 5-(3-(2,2-dimethylmorpholinyl)azetidin-1-yl)-2-methylbenzoic acid

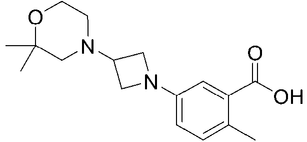

S96 (35 mg, 0.11 mmol) was placed in a single-necked flask. THF (1.0 mL) and EtOH (1.0 mL) were added, followed by the addition of a solution of NaOH (22 mg, 0.55 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S97 (30 mg, 89% yield). MS: m/z [M+1]⁺ = 305.26.

### (3) Preparation of P33: 5-(3-(2,2-dimethylmorpholin)azetidin-1-yl)-2-methyl-N-(1-(1-methyl-2-oxo-1,2-dihydrobenzo[cd]indol-6-yl)cycloprop yl)benzamide

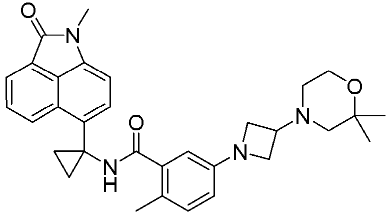

S6 (20 mg, 0.082 mmol) and S97 (30 mg, 0.098 mmol) were placed in a single-necked flask. DMF (1.5 mL), DIPEA (35 mg, 0.27 mmol), and HATU (41 mg, 0.11 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P33 (16 mg, 37% yield). MS: m/z [M+1]⁺ = 525.32.

### Example 34

### Preparation of Compound P34: 2-Methyl-N-(1-(1-methyl-2-oxo-1,2-dihydrobenzo[cd]indol-6-yl)cyclopropyl)-5-(3-(2-methylpiperidin-1-yl)azetidin-1-yl)benzamide

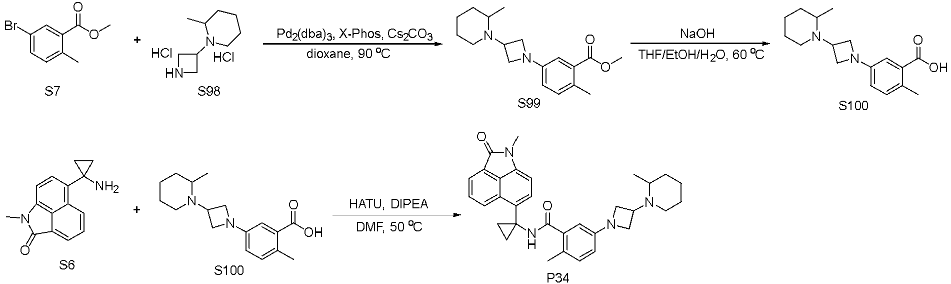

### (1) Preparation of Intermediate S99: methyl 2-methyl-5-(3-(2-methylpiperidin-1-yl)azetidin-1-yl)benzoate

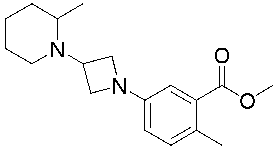

S7 (74 mg, 0.32 mmol) and S98 (50 mg, 0.32 mmol) were placed in a sealed tube. Dioxane (2 mL), X-Phos (6 mg, 0.013 mmol), Cs₂CO₃ (317 mg, 0.97 mmol), and Pd₂(dba)₃ (6 mg, 0.0065 mmol) were added. The mixture was bubbled with N₂ for 30 seconds, then heated to 90°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1, DCM:MeOH=20:1) to obtain a pale yellow oil S99 (96 mg, 97% yield). MS: m/z [M+1]⁺ = 303.21.

### (2) Preparation of Intermediate S100: 2-methyl-5-(3-(2-methylpiperidin-1-yl)azetidin-1-yl)benzoic acid

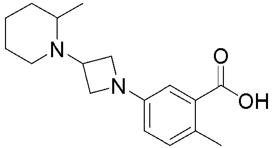

S99 (96 mg, 0.32 mmol) was placed in a single-necked flask. THF (1.0 mL) and EtOH (1.0 mL) were added, followed by the addition of a solution of NaOH (63 mg, 1.58 mmol) in H₂O (1.0 mL). The mixture was heated to 60°C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S100 (88 mg, 96% yield). MS: m/z [M+1]⁺ = 289.26.

### (3) Preparation of P34: 2-methyl-N-(1-(1-methyl-2-oxo-1,2-dihydrobenzo[cd]indol-6-yl)cyclopropyl)-5-(3-(2-methylpiperidin-1-yl)azetidin-1-yl)benzamide

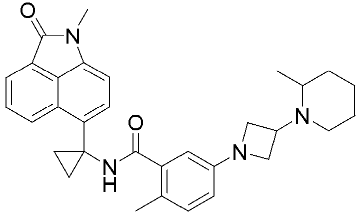

S6 (15 mg, 0.063 mmol) and S100 (22 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P34 (18 mg, 56% yield). MS: m/z [M+1]⁺ = 509.32.

### Example 35

### Preparation of Compound P35: 5-(3-(1,1-dioxothiomorpholin)azetidin-1-yl)-2-methyl-N-(1-(1-methyl-2-oxo-1,2-dihydrobenzo[cd]indol-6-yl)cycloprop yl)benzamide

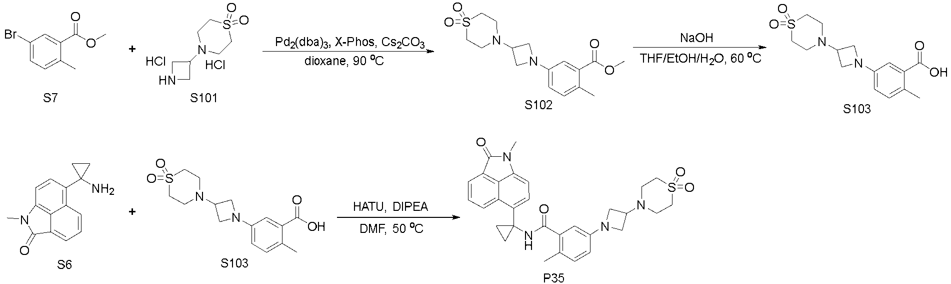

### (1) Preparation of Intermediate S102: methyl 5-(3-(1,1- dioxothiomorpholin)azetidin-1-yl)-2-methylbenzoate

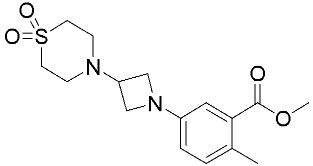

S7 (44 mg, 0.19 mmol) and S101 (50 mg, 0.19 mmol) were placed in a sealed tube. Dioxane (2 mL), X-Phos (4 mg, 0.0076 mmol), Cs₂CO₃ (310 mg, 0.95 mmol), and Pd₂(dba)₃ (3 mg, 0.0038 mmol) were added. The mixture was bubbled with N₂ for 30 seconds, then heated to 90°C and stirred overnight. After the starting materials had completely reacted, the reaction solution was cooled to room temperature. Water (6 mL) was added, the mixture was extracted three times with DCM, and the organic phases were combined. The combined organic phases were concentrated under reduced pressure to remove solvent and then purified by column chromatography (PE:EA=1:1, DCM:MeOH=20:1) to obtain a pale yellow solid S102 (60 mg, 93% yield). MS: m/z [M+1]⁺ = 339.21.

### (2) Preparation of Intermediate S103: 5-(3-(1,1-dioxothiomorpholin)azetidin-1-yl)-2-methylbenzoic acid

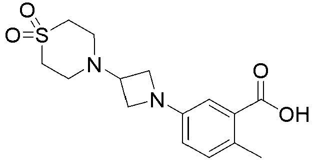

S102 (60 mg, 0.18 mmol) was placed in a single-necked flask. THF (1.0 mL) and EtOH (1.0 mL) were added, followed by the addition of a solution of NaOH (35 mg, 0.89 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was concentrated under reduced pressure to remove the solvent and obtain a solid mixture. The obtained solid mixture was dissolved in DCM, dried with anhydrous sodium sulfate, and then filtered. The filter cake was washed with DCM. The filtrate was concentrated under reduced pressure to obtain a pale yellow solid S103 (41 mg, 71% yield). MS: m/z [M+1]⁺ = 325.26.

### (3) Preparation of P35: 5-(3-(1,1-dioxothiomorpholin)azetidin-1-yl)-2-methyl-N-(1-(1-methyl-2-oxo-1,2-dihydrobenzo[cd]indol-6-yl)cycloprop yl)benzamide

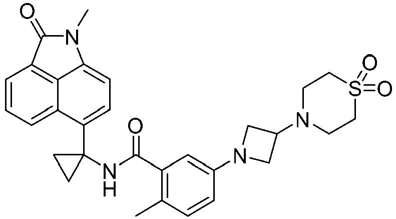

S6 (15 mg, 0.063 mmol) and S103 (24 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P35 (7 mg, 20% yield). MS: m/z [M+1]⁺ = 545.32.

### Example 36

### Preparation of Compound P36: (S)-5-(3-(3-(isopropyl(methyl)amino)pyrrolidin-1-yl)-2-methyl-N-(1-(1-methyl-2-oxo-1,2-dihydrobenzo[cd]indol-6-yl) cyclopropyl)benzamide

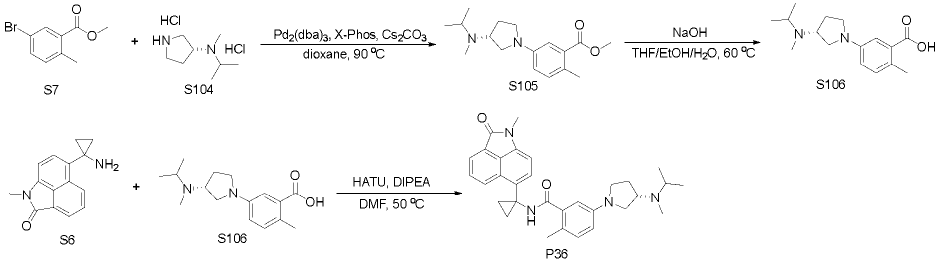

### (1) Preparation of Intermediate S105: methyl (R)-5-(3-(3-(isopropyl(methyl)amino)pyrrolidin-1-yl)-2-methylbenzoate

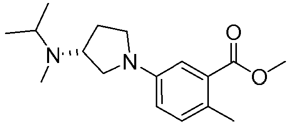

S7 (53 mg, 0.23 mmol) and S104 (50 mg, 0.23 mmol) were placed in a sealed tube. Dioxane (2 mL), X-Phos (4 mg, 0.0092 mmol), Cs₂CO₃ (378 mg, 1.16 mmol), and Pd₂(dba)₃ (4 mg, 0.0046 mmol) were added. The mixture was bubbled with N₂ for 30 seconds, then heated to 90°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1, DCM:MeOH=20:1) to obtain a pale yellow oil S105 (51 mg, 76% yield). MS: m/z [M+1]⁺ = 291.21.

### (2) Preparation of Intermediate S106: (R)-5-(3-(3-(isopropyl(methyl)amino)pyrrolidin-1-yl)-2-methylbenzoic acid

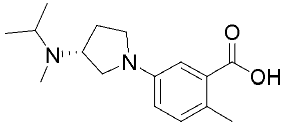

S105 (51 mg, 0.17 mmol) was placed in a single-necked flask. THF (1.0 mL) and EtOH (1.0 mL) were added, followed by the addition of a solution of NaOH (35 mg, 0.87 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S106 (41 mg, 85% yield). MS: m/z [M+1]⁺ = 277.26.

### (3) Preparation of P36: (S)-5-(3-(3-(isopropyl(methyl)amino)pyrrolidin-1-yl)-2-methyl-N-(1-(1-methyl-2-oxo-1,2-dihydrobenzo[cd]indol-6-yl) cyclopropyl)benzamide

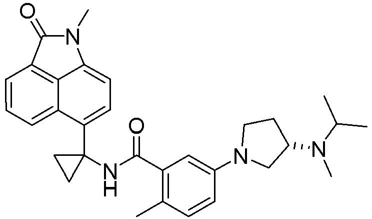

S6 (15 mg, 0.063 mmol) and S106 (21 mg, 0.075 mmol) were placed in a single-necked flask. DMF (1.5 mL), DIPEA (27 mg, 0.21 mmol), and HATU (31 mg, 0.082 mmol) were added. The mixture was heated to 50°C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P36 (20 mg, 64% yield). MS: m/z [M+1]⁺ = 497.32.

### Example 37

### Preparation of Compound P37: 5-([1,3'-dipyrrolidin]-1'-yl)-2-methyl-N-(1-(1-methyl-2-oxo-1,2-dihydrobenzo[cd]indol-6-yl)cyclopropyl)benzamide

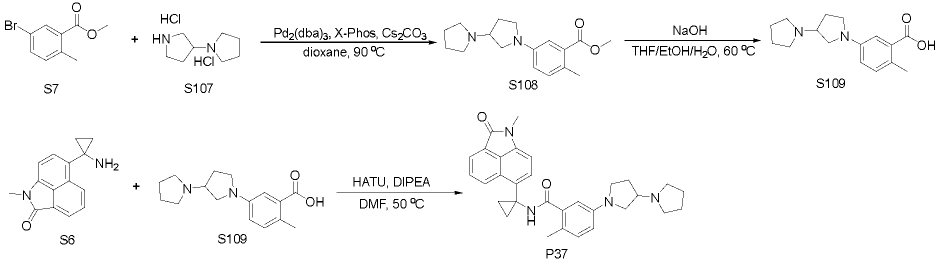

### (1) Preparation of Intermediate S108: methyl 5-([1,3'-dipyrrolidin]-1'-yl)-2-methylbenzoate

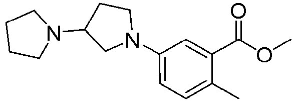

S7 (54 mg, 0.23 mmol) and S107 (50 mg, 0.23 mmol) were placed in a sealed tube. Dioxane (2 mL), X-Phos (4 mg, 0.0094 mmol), Cs₂CO₃ (378 mg, 1.16 mmol), and Pd₂(dba)₃ (4 mg, 0.0047 mmol) were added. The mixture was bubbled with N₂ for 30 seconds, then heated to 90°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1, DCM:MeOH=20:1) to obtain a pale yellow solid S108 (45 mg, 66% yield). MS: m/z [M+1]⁺ = 289.21.

### (2) Preparation of Intermediate S109: 5-([1,3'-dipyrrolidin]-1'-yl)-2-methylbenzoic acid

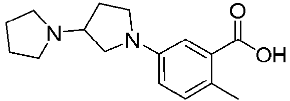

S108 (45 mg, 0.15 mmol) was placed in a single-necked flask. THF (1.0 mL) and EtOH (1.0 mL) were added, followed by the addition of a solution of NaOH (31 mg, 0.78 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S109 (41 mg, 96% yield). MS: m/z [M+1]⁺ = 275.26.

### (3) Preparation of P37: 5-([1,3'-dipyrrolidin]-1'-yl)-2-methyl-N-(1-(1-methyl-2-oxo-1,2-dihydrobenzo[cd]indol-6-yl)cyclopropyl)benzamide

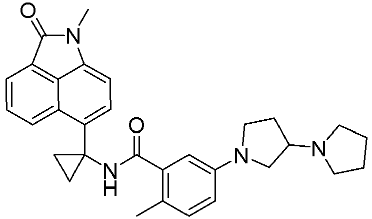

S6 (15 mg, 0.063 mmol) and S109 (20 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P37 (7 mg, 22% yield). MS: m/z [M+1]⁺ = 495.32.

### Example 38

### Preparation of Compound P38: 2-Methyl-N-(1-(1-methyl-2-oxo-1,2-dihydrobenzo[cd]indol-6-yl)cyclopropyl)-5-(4-(morpholinomethyl)piperidin-1-yl)b enzamide

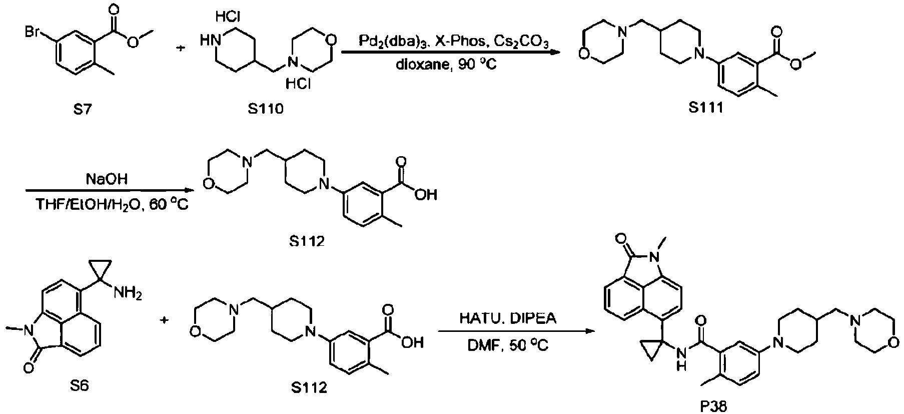

### (1) Preparation of Intermediate S111: methyl 2-methyl-5-(4-(morpholinomethyl)piperidin-1-yl)benzoate

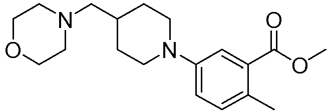

S7 (62 mg, 0.27 mmol) and S110 (50 mg, 0.27 mmol) were placed in a sealed tube. Dioxane (2 mL), X-Phos (5 mg, 0.011 mmol), Cs₂CO₃ (262 mg, 0.80 mmol), and Pd₂(dba)₃ (5 mg, 0.0054 mmol) were added. The mixture was bubbled with N₂ for 30 seconds, then heated to 90°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1, DCM:MeOH=20:1) to obtain a pale yellow solid S111 (86 mg, 96% yield). MS: m/z [M+1]⁺ = 333.21.

### (2) Preparation of Intermediate S112: 2-methyl-5-(4-(morpholinomethyl)piperidin-1-yl)benzoic acid

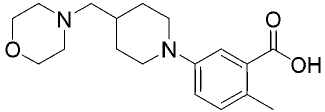

S111 (86 mg, 0.26 mmol) was placed in a single-necked flask. THF (1.0 mL) and EtOH (1.0 mL) were added, followed by the addition of a solution of NaOH (52 mg, 1.30 mmol) in H₂O (1.0 mL). The mixture was heated to 60°C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S112 (80 mg, 97% yield). MS: m/z [M+1]⁺ = 319.26.

### (3) Preparation of P38: 2-methyl-N-(1-(1-methyl-2-oxo-1,2-dihydrobenzo[cd]indol-6-yl)cyclopropyl)-5-(4-(morpholinomethyl)piperidin-1-yl)b enzamide

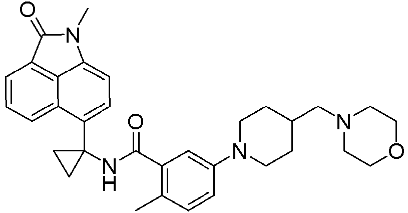

S6 (15 mg, 0.063 mmol) and S112 (24 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P38 (10 mg, 29% yield). MS: m/z [M+1]⁺ = 539.32.

### Example 39

### Preparation of Compound P39: 2-Methyl-N-(1-(1-methyl-2-oxo-1,2-dihydrobenzo[cd]indol-6-yl)cyclopropyl)-5-(1-methyloctahydro-6H-pyrrolo[3,4-b] pyridin-6-yl)benzamide

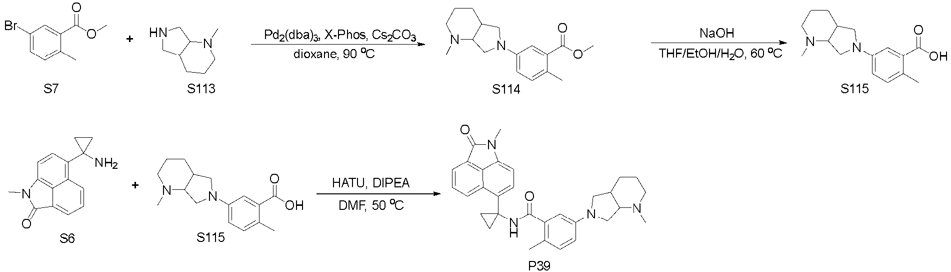

### (1) Preparation of Intermediate S114: methyl 2-methyl-5-(1-methyloctahydro-6H-pyrrolo[3,4-b]pyridin-6-yl)benzoate

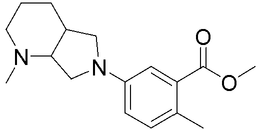

S7 (82 mg, 0.36 mmol) and S113 (50 mg, 0.36 mmol) were placed in a sealed tube. Dioxane (2 mL), X-Phos (7 mg, 0.014 mmol), Cs₂CO₃ (349 mg, 1.07 mmol), and Pd₂(dba)₃ (7 mg, 0.0071 mmol) were added. The mixture was bubbled with N₂ for 30 seconds, then heated to 90°C and stirred overnight. After the starting materials had completely reacted, the reaction solution was cooled to room temperature. Water (6 mL) was added, the mixture was extracted three times with DCM, and the organic phases were combined. The combined organic phases were concentrated under reduced pressure to remove solvent and then purified by column chromatography (PE:EA=1:1, DCM:MeOH=20:1) to obtain a pale yellow solid S114 (70 mg, 68% yield). MS: m/z [M+1]⁺ = 289.21.

### (2) Preparation of Intermediate S115: 2-methyl-5-(1-methyloctahydro-6H-pyrrolo[3,4-b]pyridin-6-yl)benzoic acid

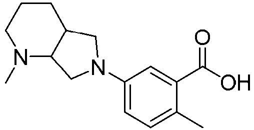

S114 (70 mg, 0.24 mmol) was placed in a single-necked flask. THF (1.0 mL) and EtOH (1.0 mL) were added, followed by the addition of a solution of NaOH (49 mg, 1.21 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was concentrated under reduced pressure to remove the solvent and obtain a solid mixture. The obtained solid mixture was dissolved in DCM, dried with anhydrous sodium sulfate, and then filtered. The filter cake was washed with DCM. The filtrate was collected and concentrated under reduced pressure to obtain a pale yellow solid S115 (60 mg, 90% yield). MS: m/z [M+1]⁺ = 275.26.

### (3) Preparation of P39: 2-methyl-N-(1-(1-methyl-2-oxo-1,2-dihydrobenzo[cd]indol-6-yl)cyclopropyl)-5-(1-methyloctahydro-6H-pyrrolo[3,4-b] pyridin-6-yl)benzamide

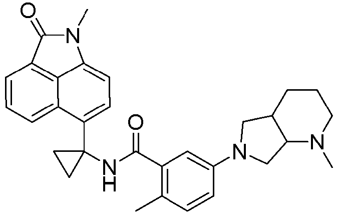

S6 (15 mg, 0.063 mmol) and S115 (21 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P39 (14 mg, 45% yield). MS: m/z [M+1]⁺ = 495.32.

### Example 40

### Preparation of Compound P40: 2-Methyl-N-(1-(1-methyl-2-oxo-1,2-dihydrobenzo[cd]indol-6-yl)cyclopropyl)-5-((1R,4R)-5-methyl-2,5-diazabicyclo[2. 2.1]heptan-2-yl)benzamide

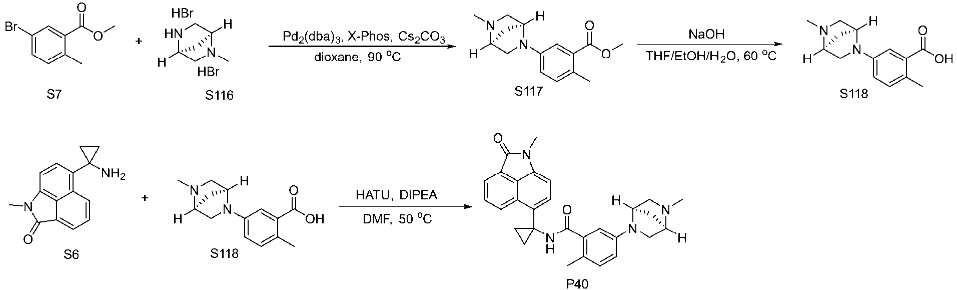

### (1) Preparation of Intermediate S117: methyl 2-methyl-5-((1S,4S)-5-methyl-2,5-diazabicyclo[2.2.1]heptan-2-yl)benzoate

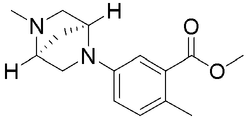

S7 (42 mg, 0.18 mmol) and S116 (50 mg, 0.18 mmol) were placed in a sealed tube. Dioxane (2 mL), X-Phos (4 mg, 0.0070 mmol), Cs₂CO₃ (296 mg, 0.91 mmol), and Pd₂(dba)₃ (3 mg, 0.0036 mmol) were added. The mixture was bubbled with N₂ for 30 seconds, then heated to 90°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1; DCM:MeOH=20:1, 10:1) to obtain a pale yellow oil S117 (42 mg, 88% yield). MS: m/z [M+1]⁺ = 261.21.

### (2) Preparation of Intermediate S118: 2-methyl-5-((1S,4S)-5-methyl-2,5-diazabicyclo[2.2.1]heptan-2-yl)benzoic acid

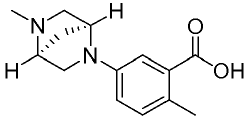

S117 (42 mg, 0.16 mmol) was placed in a single-necked flask. THF (1.0 mL) and EtOH (1.0 mL) were added, followed by the addition of a solution of NaOH (32 mg, 0.80 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S118 (35 mg, 90% yield). MS: m/z [M+1]⁺ = 247.26.

### (3) Preparation of P40: 2-methyl-N-(1-(1-methyl-2-oxo-1,2-dihydrobenzo[cd]indol-6-yl)cyclopropyl)-5-((1R,4R)-5-methyl-2,5-diazabicyclo[2. 2.1]heptan-2-yl)benzamide

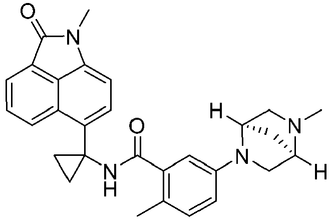

S6 (15 mg, 0.063 mmol) and S118 (18 mg, 0.075 mmol) were placed in a single-necked flask. DMF (1.5 mL), DIPEA (27 mg, 0.21 mmol), and HATU (31 mg, 0.082 mmol) were added. The mixture was heated to 50°C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P40 (9 mg, 31% yield). MS: m/z [M+1]⁺ = 467.32.

### Example 41

### Preparation of Compound P41: 5-(4-(2-hydroxypropyl)piperazin-1-yl)-2-methyl-N-(1-(1-methyl-2-oxo-1,2-dihydrobenzo[cd]indol-6-yl)cyclopropyl)be nzamide

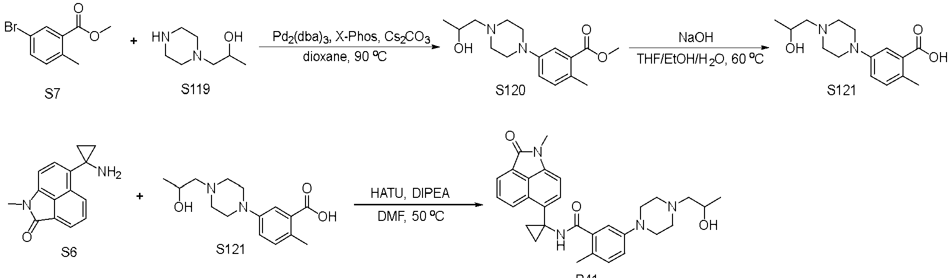

### (1) Preparation of Intermediate S120: methyl 5-(4-(2-hydroxypropyl)piperazin-1-yl)-2-methylbenzoate

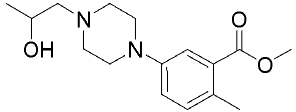

S7 (79 mg, 0.35 mmol) and S119 (50 mg, 0.35 mmol) were placed in a sealed tube. Dioxane (2 mL), X-Phos (7 mg, 0.014 mmol), Cs₂CO₃ (339 mg, 1.04 mmol), and Pd₂(dba)₃ (6 mg, 0.0069 mmol) were added. The mixture was bubbled with N₂ for 30 seconds, then heated to 90°C and stirred overnight. After the starting materials had completely reacted, the reaction solution was cooled to room temperature. A saturated ammonium chloride solution (6 mL) was added. The mixture was extracted three times with DCM, and the organic phases were combined. The combined organic phases were concentrated under reduced pressure to remove solvent and then purified by column chromatography (PE:EA=1:1; DCM:MeOH=20:1, 10:1) to obtain a pale yellow oil S120 (42 mg, 88% yield). MS: m/z [M+1]⁺ = 293.21.

### (2) Preparation of Intermediate S121: 5-(4-(2-hydroxypropyl)piperazin-1-yl)-2-methylbenzoic acid

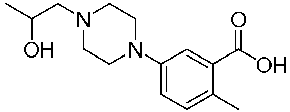

S120 (30 mg, 0.10 mmol) was placed in a single-necked flask. THF (1.0 mL) and EtOH (1.0 mL) were added, followed by the addition of a solution of NaOH (20 mg, 0.50 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S121 (24 mg, 86% yield). MS: m/z [M+1]⁺ = 279.26.

### (3) Preparation of P41: 5-(4-(2-hydroxypropyl)piperazin-1-yl)-2-methyl-N-(1-(1-methyl-2-oxo-1,2-dihydrobenzo[cd]indol-6-yl)cyclopropyl)be nzamide

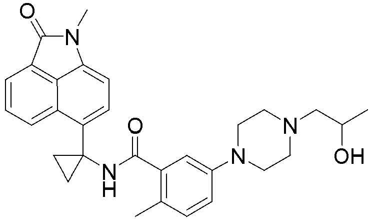

S6 (11 mg, 0.048 mmol) and S121 (16 mg, 0.057 mmol) were placed in a single-necked flask. DMF (1.5 mL), DIPEA (20 mg, 0.16 mmol), and HATU (24 mg, 0.062 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P41 (9 mg, 37% yield). MS: m/z [M+1]⁺ = 499.32.

### Example 42

### Preparation of Compound P42: 5-([1,3'-diazetidin]-1'-yl)-2-methyl-N-(1-(1-methyl-2-oxo-1,2-dihydrobenzo[cd]indol-6-yl)cyclopropyl)benzamide

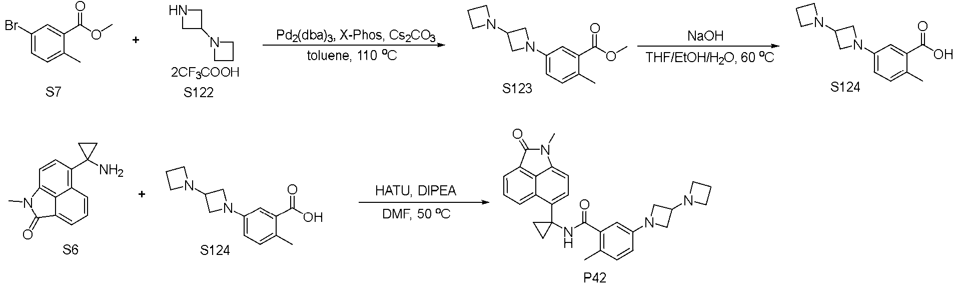

### (1) Preparation of Intermediate S123: methyl 5-([1,3'-diazetidin] -1'-yl)-2-methylbenzoate

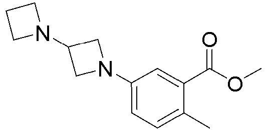

S7 (42 mg, 0.18 mmol) and S122 (67 mg, 0.20 mmol) were placed in a sealed tube. Toluene (1.5 mL), X-Phos (3 mg, 0.069 mmol), Cs₂CO₃ (318 mg, 0.90 mmol), and Pd₂(dba)₃ (3 mg, 0.0036 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1; DCM:MeOH=20:1, 10:1) to obtain a pale yellow solid S123 (47 mg, 100% yield). MS: m/z [M+1]⁺ = 261.21.

### (2) Preparation of Intermediate S124: 5-([1,3'-diazetidin]-1'-yl]-2-methylbenzoic acid

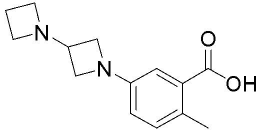

S123 (47 mg, 0.18 mmol) was placed in a single-necked flask. THF (1.0 mL) and EtOH (1.0 mL) were added, followed by the addition of a solution of NaOH (36 mg, 0.90 mmol) in H₂O (1.0 mL). The mixture was heated to 60°C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S124 (37 mg, 84% yield). MS: m/z [M+1]⁺ = 247.26.

### (3) Preparation of P42: 5-([1,3'-diazetidin]-1'-yl)-2-methyl-N-(1-(1-methyl-2-oxo-1,2-dihydrobenzo[cd]indol-6-yl)cyclopropyl)benzamide

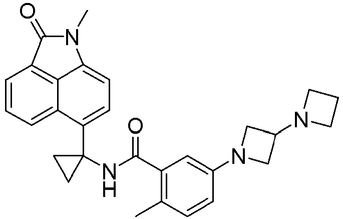

S6 (15 mg, 0.063 mmol) and S124 (18 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P42 (23 mg, 80% yield). MS: m/z [M+1]⁺ = 467.32.

### Example 43

### Preparation of Compound P43: 5-(3-(3-hydroxypyrrolidin-1-yl)azetidin-1-yl)-2-methyl-N-(1-(1-methyl-2-oxo-1,2-dihydrobenzo[cd]indol-6-yl)cyclopro pyl)benzamide

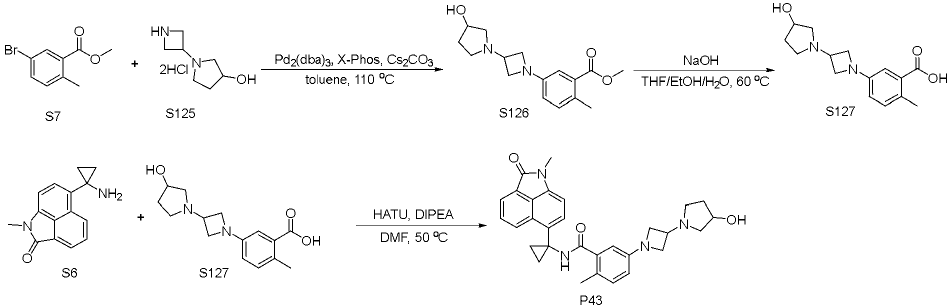

### (1) Preparation of Intermediate S126: Methyl 5-(3-(3-hydroxypyrrolidin-1-yl)azetidin-1-yl)-2-methylbenzoate

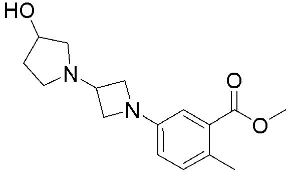

S7 (60 mg, 0.26 mmol) and S125 (67 mg, 0.31 mmol) were placed in a sealed tube. Toluene (2.0 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. A saturated ammonium chloride solution (6 mL) was added, the mixture was extracted three times with DCM, and the organic phases were combined. The combined organic phases were concentrated under reduced pressure to remove solvent and then purified by column chromatography (PE:EA=1:1; DCM:MeOH=20:1; 10:1) to obtain a colorless oil S126 (16 mg, 21% yield). MS: m/z [M+1]⁺ = 291.21.

### (2) Preparation of Intermediate S127: 5-(3-(3-hydroxypyrrolidin-1-yl)azetidin-1-yl)-2-methylbenzoic acid

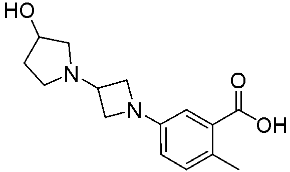

S126 (16 mg, 0.055 mmol) was placed in a single-necked flask. THF (0.5 mL) and EtOH (0.5 mL) were added, followed by the addition of a solution of NaOH (11 mg, 0.28 mmol) in H₂O (0.5 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was concentrated under reduced pressure to remove the solvent and obtain a solid mixture. The obtained solid mixture was dissolved in DCM, dried with anhydrous sodium sulfate, and then filtered. The filter cake was washed with DCM. The filtrate was collected and concentrated under reduced pressure to obtain a pale yellow solid S127 (15 mg, 100% yield). MS: m/z [M+1]⁺ = 247.26.

### (3) Preparation of P43: 5-(3-(3-hydroxypyrrolidin-1-yl)azetidin-1-yl)-2-methyl-N-(1-(1-methyl-2-oxo-1,2-dihydrobenzo[cd]indol-6-yl)cyclopro pyl)benzamide

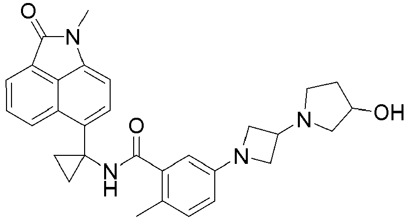

S6 (11 mg, 0.045 mmol) and S127 (15 mg, 0.054 mmol) were placed in a single-necked flask. DMF (1.5 mL), DIPEA (19 mg, 0.15 mmol), and HATU (22 mg, 0.058 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was extracted three times with DCM, and the organic phases were combined. The combined organic phases were concentrated under reduced pressure and then purified by column chromatography (PE:EA=1:1; DCM:MeOH=20:1, 10:1, 8:1) to obtain a bright yellow solid P43 (9 mg, 41% yield). MS: m/z [M+1]⁺ = 497.32.

### Example 44

### Preparation of Compound P44: 2-Methyl-N-(1-(1-methyl-2-oxo-1,2-dihydrobenzo[cd]indol-6-yl)cyclopropyl)-5-(3-(4-methyl-3-oxopiperazin-1-yl)azeti din-1-yl)benzamide

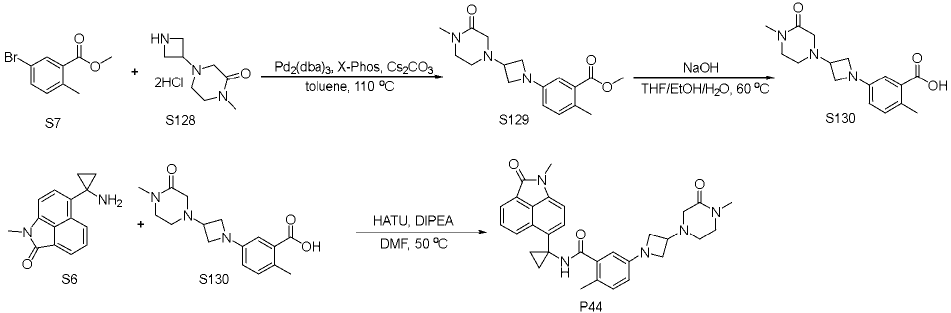

### (1) Preparation of Intermediate S129: methyl 2-methyl-5-(3-(4-methyl-3-oxopiperazin-1-yl)azetidin-1-yl)benzoate

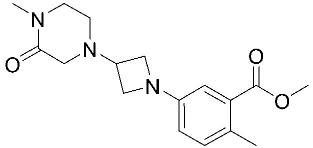

S7 (60 mg, 0.26 mmol) and S128 (75 mg, 0.31 mmol) were placed in a sealed tube. Toluene (2.0 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1; DCM:MeOH=20:1, 10:1) to obtain a pale yellow solid S129 (56 mg, 68% yield). MS: m/z [M+1]⁺ = 318.21.

### (2) Preparation of Intermediate S130: 2-methyl-5-(3-(4-methyl-3-oxopiperazin-1-yl)azetidin-1-yl)benzoic acid

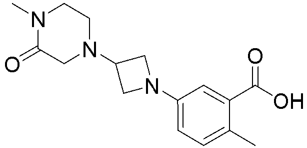

S129 (56 mg, 0.18 mmol) was placed in a single-necked flask. THF (1.0 mL) and EtOH (1.0 mL) were added, followed by the addition of a solution of NaOH (35 mg, 0.88 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S130 (54 mg, 100% yield). MS: m/z [M+1]⁺ = 304.26.

### (3) Preparation of P44: 2-methyl-N-(1-(1-methyl-2-oxo-1,2-dihydrobenzo[cd]indol-6-yl)cyclopropyl)-5-(3-(4-methyl-3-oxopiperazin-1-yl)azeti din-1-yl)benzamide

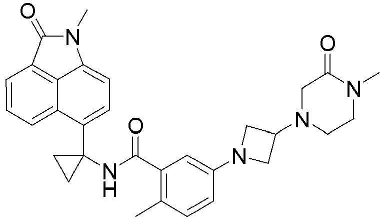

S6 (15 mg, 0.063 mmol) and S130 (23 mg, 0.075 mmol) were placed in a single-necked flask. DMF (1.5 mL), DIPEA (27 mg, 0.21 mmol), and HATU (31 mg, 0.082 mmol) were added. The mixture was heated to 50°C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1, 8:1) to obtain a bright yellow solid P44 (12 mg, 36% yield). MS: m/z [M+1]⁺ = 524.32.

### Example 45

### Preparation of Compound P45: 2-methyl-N-(1-(1-methyl-2-oxo-1,2-dihydrobenzo[cd]indol-6-yl)cyclopropyl)-5-(3-(pyrrolidin-1-yl)azetidin-1-yl)benza mide

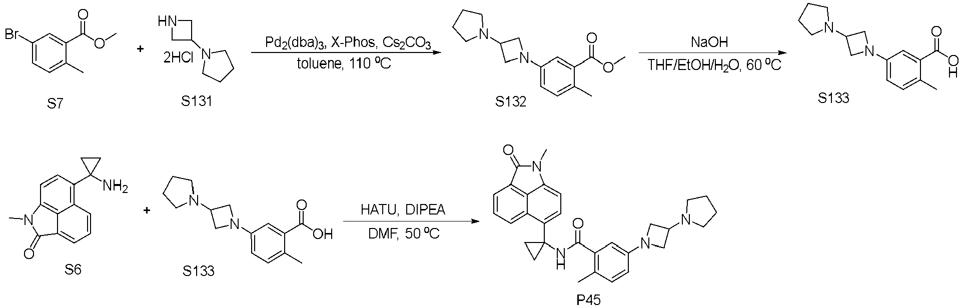

### (1) Preparation of Intermediate S132: methyl 2-methyl-5-(3-(pyrrolidin-1-yl)azetidin-1-yl)benzoate

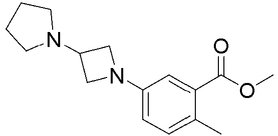

S7 (60 mg, 0.26 mmol) and S131 (62 mg, 0.31 mmol) were placed in a sealed tube. Toluene (2.0 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1; DCM:MeOH=20:1, 10:1) to obtain a pale yellow oil S132 (71 mg, 100% yield). MS: m/z [M+1]⁺ = 275.21.

### (2) Preparation of Intermediate S133: 2-methyl-5-(3-(pyrrolidin-1-yl)azetidin-1-yl)benzoic acid

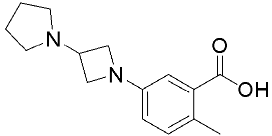

S132 (71 mg, 0.26 mmol) was placed in a single-necked flask. THF (1.0 mL) and EtOH (1.0 mL) were added, followed by the addition of a solution of NaOH (52 mg, 1.30 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S133 (68 mg, 100% yield). MS: m/z [M+1]⁺ = 261.26.

### (3) Preparation of P45: 2-methyl-N-(1-(1-methyl-2-oxo-1,2-dihydrobenzo[cd]indol-6-yl)cyclopropyl)-5-(3-(pyrrolidin-1-yl)azetidin-1-yl)benza mide

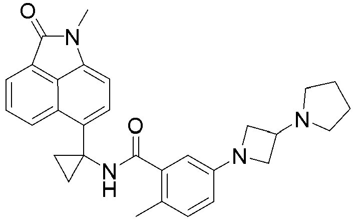

S6 (15 mg, 0.063 mmol) and S133 (20 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P45 (14 mg, 47% yield). MS: m/z [M+1]⁺ = 481.32.

### Example 46

### Preparation of Compound P46: 2-methyl-N-(1-(1-methyl-2-oxo-1,2-dihydrobenzo[cd]indol-6-yl)cyclopropyl)-5-(3-morpholinazetidin-1-yl)benzamide

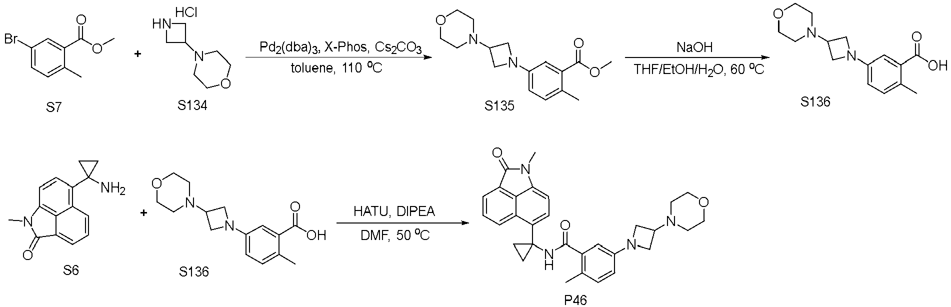

### (1) Preparation of Intermediate S135: methyl 2-methyl-5-(3-morpholinazetidin -1-yl)benzoate

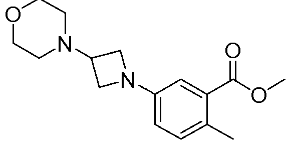

S7 (60 mg, 0.26 mmol) and S134 (55 mg, 0.31 mmol) were placed in a sealed tube. Toluene (2.0 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=3:1, 1:1) to obtain a white solid S135 (75 mg, 100% yield). MS: m/z [M+1]⁺ = 291.21.

### (2) Preparation of Intermediate S136: 2-methyl-5-(3-morpholinazetidin -1-yl)benzoic acid

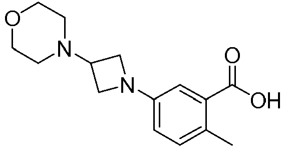

S135 (75 mg, 0.26 mmol) was placed in a single-necked flask. THF (1.0 mL) and EtOH (1.0 mL) were added, followed by the addition of a solution of NaOH (52 mg, 1.30 mmol) in H₂O (1.0 mL). The mixture was heated to 60°C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a white solid S136 (72 mg, 100% yield). MS: m/z [M+1]⁺ = 277.26.

### (3) Preparation of P46: 2-methyl-N-(1-(1-methyl-2-oxo-1,2-dihydrobenzo[cd]indol-6-yl)cyclopropyl)-5-(3-morpholinazetidin -1-yl)benzamide

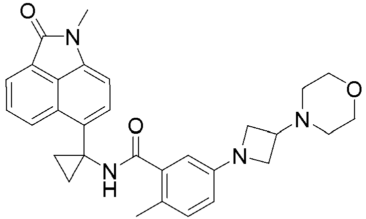

S6 (15 mg, 0.063 mmol) and S136 (21 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P46 (20 mg, 65% yield). MS: m/z [M+1]⁺ = 497.32.

### Example 47

### Preparation of Compound P47: 2-methyl-N-(1-(1-methyl-2-oxo-1,2-dihydrobenzo[cd]indol-6-yl)cyclopropyl)-5-(3aR,6aR)-1-methyl hexahydropyrrolo[3,4-b]pyrrol-5(1H)-yl)benzamide

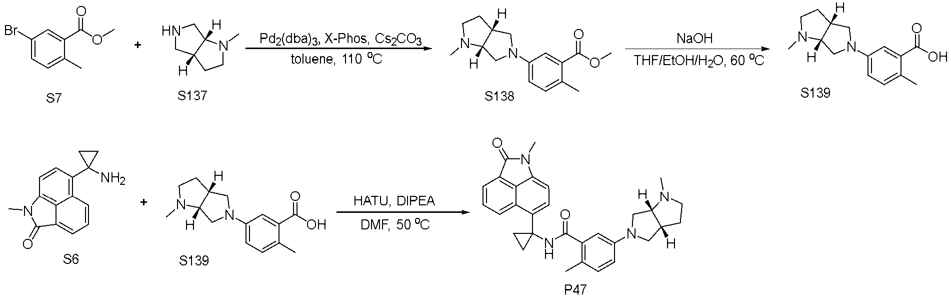

### (1) Preparation of Intermediate S138: methyl 2-methyl-5-(3aR,6aR)-1-methyl hexahydropyrrolo[3,4-b]pyrrol-5(1H)-yl)benzoate

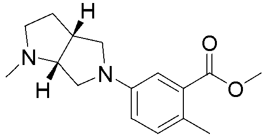

S7 (60 mg, 0.26 mmol) and S137 (39 mg, 0.31 mmol) were placed in a sealed tube. Toluene (2.0 mL), X-Phos (5 mg, 0.010 mmol), Cs₂CO₃ (169 mg, 0.52 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added, the mixture was extracted three times with DCM, and the organic phases were combined. The combined organic phases were concentrated under reduced pressure to remove solvent and then purified by column chromatography (PE:EA=1:1, DCM:MeOH=20:1) to obtain a pale yellow solid S138 (52 mg, 73% yield). MS: m/z [M+1]⁺ = 275.21.

### (2) Preparation of Intermediate S139: 2-methyl-5-(3aR,6aR)-1-methyl hexahydropyrrolo[3,4-b]pyrrol-5(1H)-yl)benzoic acid

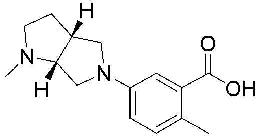

S138 (52 mg, 0.19 mmol) was placed in a single-necked flask. THF (1.0 mL) and EtOH (1.0 mL) were added, followed by the addition of a solution of NaOH (38 mg, 0.95 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was concentrated under reduced pressure to remove the solvent and obtain a solid mixture. The obtained solid mixture was dissolved in DCM, dried with anhydrous sodium sulfate, and then filtered. The filter cake was washed with DCM. The filtrate was collected and concentrated under reduced pressure to obtain a pale yellow solid S139 (49 mg, 100% yield). MS: m/z [M+1]⁺ = 261.26.

### (3) Preparation of P47: 2-methyl-N-(1-(1-methyl-2-oxo-1,2-dihydrobenzo[cd]indol-6-yl)cyclopropyl)-5-(3aR,6aR)-1-methyl hexahydropyrrolo[3,4-b]pyrrol-5(1H)-yl)benzamide

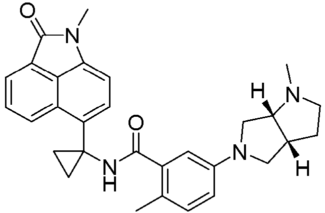

S6 (15 mg, 0.063 mmol) and S139 (20 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was extracted three times with DCM, and the organic phases were combined. The combined organic phases were concentrated under reduced pressure and then purified by column chromatography (PE:EA=1:1; DCM:MeOH=20:1, 10:1) to obtain a bright yellow solid P47 (17 mg, 57% yield). MS: m/z [M+1]⁺ = 481.32.

### Example 48

### Preparation of Compound P48: 5-(3,3-difluoro-4-(pyrrolidin-1-yl)piperidin-1-yl)-2-methyl-N-(1-(1-methyl-2-oxo-1,2-dihydrobenzo[cd]indol-6-yl)cycl opropyl)benzamide

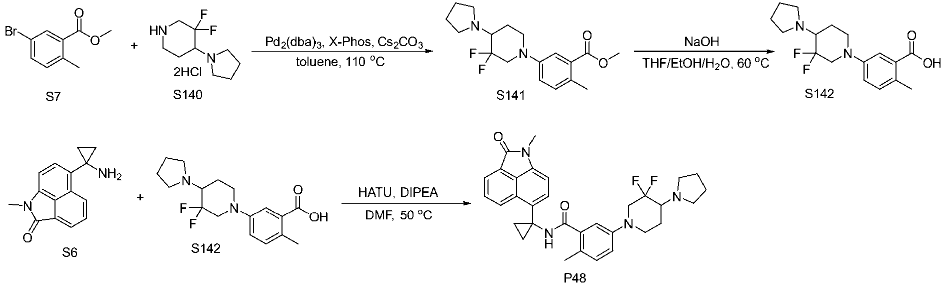

### (1) Preparation of Intermediate S141: methyl 5-(3,3-difluoro-4-(pyrrolidin-1-yl)piperidin-1-yl)-2-methylbenzoate

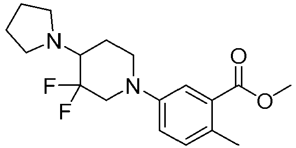

S7 (42 mg, 0.18 mmol) and S140 (53 mg, 0.20 mmol) were placed in a sealed tube. Toluene (1.5 mL), X-Phos (3 mg, 0.0069 mmol), Cs₂CO₃ (318 mg, 0.90 mmol), and Pd₂(dba)₃ (3 mg, 0.0036 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=3:1, 2:1) to obtain a white solid S141 (43 mg, 70% yield). MS: m/z [M+1]⁺ = 339.21.

### (2) Preparation of Intermediate S142: 5-(3,3-difluoro-4-(pyrrolidin-1-yl)piperidin-1-yl)-2-methylbenzoic acid

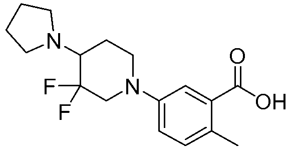

S141 (43 mg, 0.13 mmol) was placed in a single-necked flask. THF (1.0 mL) and EtOH (1.0 mL) were added, followed by the addition of a solution of NaOH (25 mg, 0.63 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a white solid S142 (34 mg, 81% yield). MS: m/z [M+1]⁺ = 325.26.

### (3) Preparation of P48: 5-(3,3-difluoro-4-(pyrrolidin-1-yl)piperidin-1-yl)-2-methyl-N-(1-(1-methyl-2-oxo-1,2-dihydrobenzo[cd]indol-6-yl)cycl opropyl)benzamide

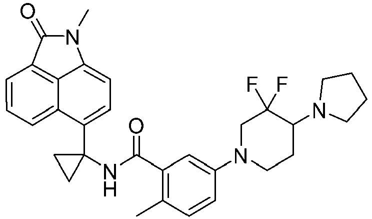

S6 (15 mg, 0.063 mmol) and S142 (24 mg, 0.075 mmol) were placed in a single-necked flask. DMF (1.5 mL), DIPEA (27 mg, 0.21 mmol), and HATU (31 mg, 0.082 mmol) were added. The mixture was heated to 50°C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=3:1, 1:1) to obtain a bright yellow solid P48 (15 mg, 44% yield). MS: m/z [M+1]⁺ = 545.32.

### Example 49

### Preparation of Compound P49: 5-(4-(2-Hydroxy-2-methylpropyl)piperazin-1-yl)-2-methyl-N-(1-(1-methyl-2-oxo-1,2-dihydrobenzo[cd]indol-6-yl)cycl opropyl)benzamide

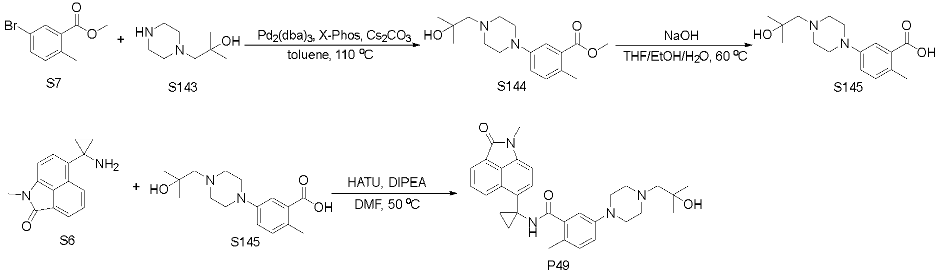

### (1) Preparation of Intermediate S144: methyl 5-(4-(2-hydroxy-2-methylpropyl)piperazin-1-yl)-2-methylbenzoate

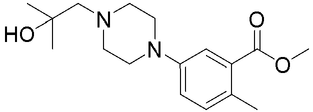

S7 (60 mg, 0.26 mmol) and S143 (49 mg, 0.31 mmol) were placed in a sealed tube. Toluene (2.0 mL), X-Phos (5 mg, 0.010 mmol), Cs₂CO₃ (169 mg, 0.52 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. A saturated ammonium chloride solution (6 mL) was added. The mixture was extracted three times with DCM, and the organic phases were combined. The combined organic phases were concentrated under reduced pressure to remove solvent and then purified by column chromatography (PE:EA=1:1; DCM:MeOH=20:1, 10:1) to obtain a white solid S144 (69 mg, 87% yield). MS: m/z [M+1]⁺ = 307.21.

### (2) Preparation of Intermediate S145: 5-(4-(2-hydroxy-2-methylpropyl)piperazin-1-yl)-2-methylbenzoic acid

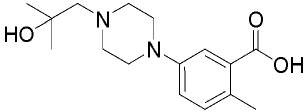

S144 (69 mg, 0.28 mmol) was placed in a single-necked flask. THF (1.0 mL) and EtOH (1.0 mL) were added, followed by the addition of a solution of NaOH (56 mg, 1.39 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a white solid S145 (41 mg, 50% yield). MS: m/z [M+1]⁺ = 293.26.

### (3) Preparation of P49: 5-(4-(2-hydroxy-2-methylpropyl)piperazin-1-yl)-2-methyl-N-(1-(1-methyl-2-oxo-1,2-dihydrobenzo[cd]indol-6-yl)cyclo propyl)benzamide

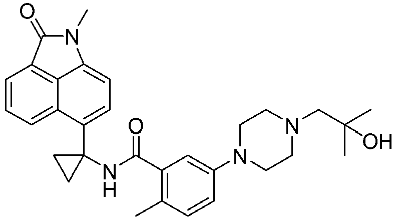

S6 (15 mg, 0.063 mmol) and S145 (22 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1, 8:1) to obtain a bright yellow solid P49 (21 mg, 66% yield). MS: m/z [M+1]⁺ = 513.32.

### Example 50

### Preparation of Compound P50: 2-Methyl-N-(1-(1-methyl-2-oxo-1,2-dihydrobenzo[cd]indol-6-yl)cyclopropyl)-5-(3-(piperidin-1-yl)pyrrolidin-1-yl)benz amide

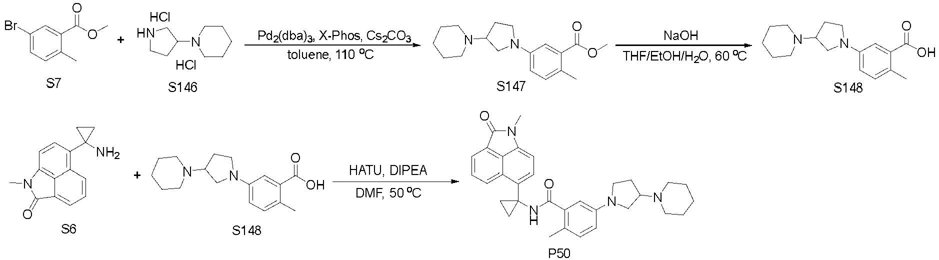

### (1) Preparation of intermediate S147: methyl 2-methyl-5-(3-(piperidin-1-yl)pyrrolidin-1-yl)benzoate

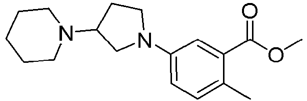

S7 (60 mg, 0.26 mmol) and S146 (70 mg, 0.31 mmol) were placed in a sealed tube. Toluene (2.0 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1, DCM:MeOH=20:1) to obtain a pale yellow oil S147 (73 mg, 93% yield). MS: m/z [M+1]⁺ = 303.21.

### (2) Preparation of Intermediate S148: 2-methyl-5-(3-(piperidin-1-yl)pyrrolidin-1-yl)benzoic acid

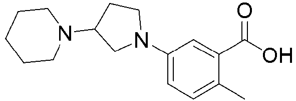

S147 (73 mg, 0.24 mmol) was placed in a single-necked flask. THF (1.0 mL) and EtOH (1.0 mL) were added, followed by the addition of a solution of NaOH (48 mg, 1.20 mmol) in H₂O (1.0 mL). The mixture was heated to 60°C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow oil S148 (69 mg, 100% yield). MS: m/z [M+1]⁺ = 289.26.

### (3) Preparation of P50: 2-methyl-N-(1-(1-methyl-2-oxo,2-dihydrobenzo[cd]indol-6-yl)cyclopropyl)-5-(3-(piperidin-1-yl)pyrrolidin-1-yl)benz amide

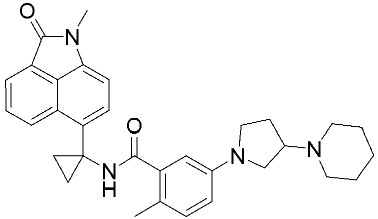

S6 (15 mg, 0.063 mmol) and S148 (22 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1, 8:1) to obtain a bright yellow solid P50 (16 mg, 50% yield). MS: m/z [M+1]⁺ = 509.32.

### Example 51

### Preparation of Compound P51: 2-Methyl-N-(1-(1-methyl-2-oxo-1,2-dihydrobenzo[cd]indol-6-yl)cyclopropyl)-5-(4-(3-methanesulfonyl)propyl) piperazin-1-yl)benzamide

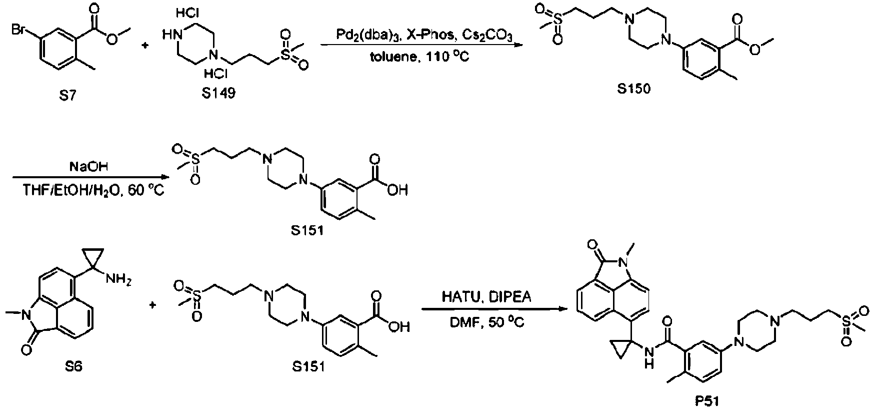

### (1) Preparation of Intermediate S150: methyl 2-methyl-5-(4-(3-methanesulfonyl)propyl)piperazin-1-yl)benzoate

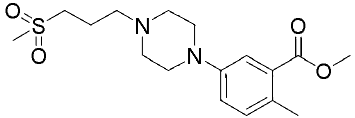

S7 (60 mg, 0.26 mmol) and S149 (86 mg, 0.31 mmol) were placed in a sealed tube. Toluene (2.0 mL), X-Phos (5 mg, 0.010 mmol), Cs₂CO₃ (424 mg, 1.30 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added, the mixture was extracted three times with DCM, and the organic phases were combined. The combined organic phases were concentrated under reduced pressure to remove solvent and then purified by column chromatography (PE:EA=1:1; DCM:MeOH=20:1, 10:1) to obtain a pale yellow solid S150 (24 mg, 26% yield). MS: m/z [M+1]⁺ = 355.21.

### (2) Preparation of Intermediate S151: 2-methyl-5-(4-(3-methanesulfonyl)propyl)piperazin-1-yl)benzoic acid

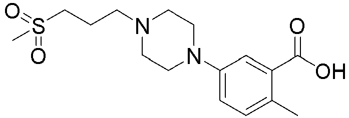

S150 (24 mg, 0.068 mmol) was placed in a single-necked flask. THF (0.5 mL) and EtOH (0.5 mL) were added, followed by the addition of a solution of NaOH (14 mg, 0.34 mmol) in H₂O (0.5 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was concentrated under reduced pressure to remove the solvent and obtain a solid mixture. The obtained solid mixture was dissolved in DCM, dried with anhydrous sodium sulfate, and then filtered. The filter cake was washed with DCM. The filtrate was collected and concentrated under reduced pressure to obtain a pale yellow solid S151 (23 mg, 100% yield). MS: m/z [M+1]⁺ = 341.26.

### (3) Preparation of P51: 2-methyl-N-(1-(1-methyl-2-oxo-1,2-dihydrobenzo[cd]indol-6-yl)cyclopropyl)-5-(4-(3-methanesulfonyl)propyl)piperazi n-1-yl)benzamide

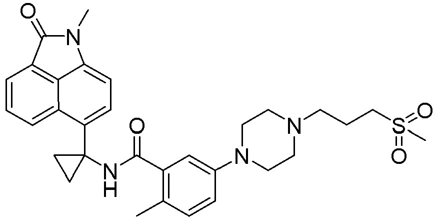

S6 (15 mg, 0.063 mmol) and S151 (23 mg, 0.068 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The mixture was extracted three times with DCM, and the organic phases were combined. The combined organic phases were concentrated under reduced pressure and then purified by column chromatography (PE:EA=1:1; DCM:MeOH=20:1, 10:1, 8:1) to obtain a bright yellow solid P51 (16 mg, 46% yield). MS: m/z [M+1]⁺ = 561.32.

### Example 52

### Preparation of Compound P52: 5-(4-(2-(Dimethylamino)ethyl)piperazin-1-yl)-2-methyl-N-(1-(1-methyl-2-oxo-1,2-dihydrobenzo[cd]indol-6-yl)cyclopr opyl)benzamide

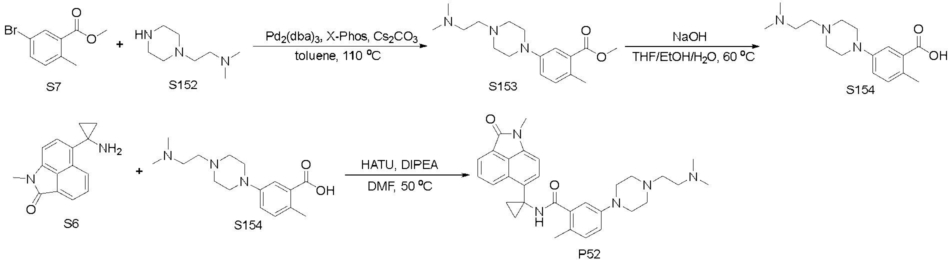

### (1) Preparation of Intermediate S153: methyl 5-(4-(2-(dimethylamino)ethyl)piperazin-1-yl)-2-methylbenzoate

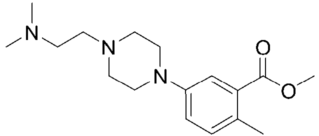

S7 (60 mg, 0.26 mmol) and S152 (49 mg, 0.31 mmol) were placed in a sealed tube. Toluene (2.0 mL), X-Phos (5 mg, 0.010 mmol), Cs₂CO₃ (169 mg, 0.52 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=1:1; DCM:MeOH=20:1, 10:1) to obtain a pale yellow solid S153 (69 mg, 87% yield). MS: m/z [M+1]⁺ = 306.21.

### (2) Preparation of Intermediate S154: 5-(4-(2-(dimethylamino)ethyl)piperazin-1-yl)-2-methylbenzoic acid

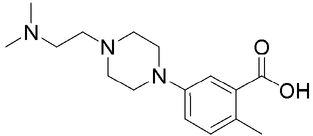

S153 (69 mg, 0.23 mmol) was placed in a single-necked flask. THF (1.0 mL) and EtOH (1.0 mL) were added, followed by the addition of a solution of NaOH (45 mg, 1.13 mmol) in H₂O (1.0 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow solid S154 (67 mg, 100% yield). MS: m/z [M+1]⁺ = 292.26.

### (3) Preparation of P52: 5-(4-(2-(dimethylamino)ethyl)piperazin-1-yl)-2-methyl-N-(1-(1-methyl-2-oxo-1,2-dihydrobenzo[cd]indol-6-yl)cyclopro pyl)benzamide

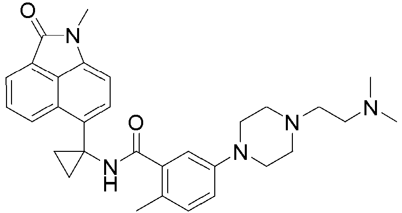

S6 (15 mg, 0.063 mmol) and S154 (22 mg, 0.075 mmol) were placed in a single-necked flask. DMF (1.5 mL), DIPEA (27 mg, 0.21 mmol), and HATU (31 mg, 0.082 mmol) were added. The mixture was heated to 50°C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1, 8:1) to obtain a bright yellow solid P52 (19 mg, 60% yield). MS: m/z [M+1]⁺ = 512.32.

### Example 53

### Preparation of Compound P53: 5-(4-(2-Hydroxyethyl)piperazin-1-yl)-2-methyl-N-(1-(1-methyl-2-oxo-1,2-dihydrobenzo[cd]indol-6-yl)cyclopropyl)ben zamide

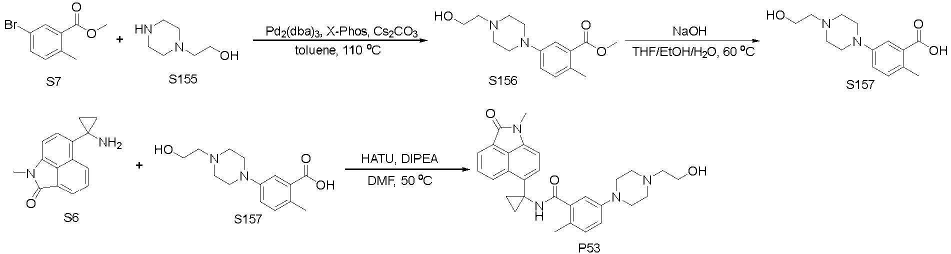

### (1) Preparation of Intermediate S156: methyl 5-(4-(2-hydroxyethyl)piperazin-1-yl)-2-methylbenzoate

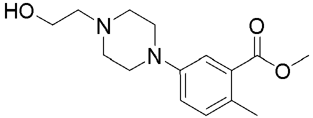

S7 (60 mg, 0.26 mmol) and S155 (40 mg, 0.31 mmol) were placed in a sealed tube. Toluene (2.0 mL), X-Phos (5 mg, 0.010 mmol), Cs₂CO₃ (169 mg, 0.52 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. A saturated ammonium chloride solution (6 mL) was added. The mixture was extracted three times with DCM, and the organic phases were combined. The combined organic phases were concentrated under reduced pressure to remove solvent and then purified by column chromatography (PE:EA=1:1, DCM:MeOH=20:1) to obtain a colorless oil S156 (8 mg, 11% yield). MS: m/z [M+1]⁺ = 279.21.

### (2) Preparation of Intermediate S157: 5-(4-(2-Hydroxyethyl)piperazin-1-yl)-2-methylbenzoic acid

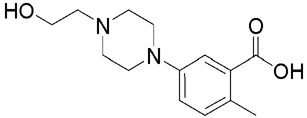

S156 (8 mg, 0.028 mmol) was placed in a single-necked flask. THF (0.4 mL) and EtOH (0.4 mL) were added, followed by the addition of a solution of NaOH (6 mg, 0.14 mmol) in H₂O (0.4 mL). The mixture was heated to 60 °C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow oil S157 (7 mg, 100% yield). MS: m/z [M+1]⁺ = 265.26.

### (3) Preparation of P53: 5-(4-(2-Hydroxyethyl)piperazin-1-yl)-2-methyl-N-(1-(1-methyl-2-oxo-1,2-dihydrobenzo[cd]indol-6-yl)cyclopropyl)ben zamide

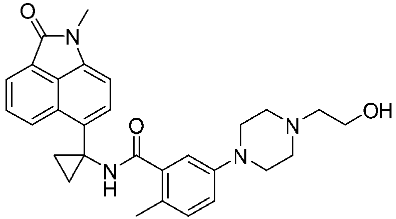

S6 (6 mg, 0.023 mmol) and S157 (7 mg, 0.028 mmol) were placed in a single-necked flask. DMF (1.5 mL), DIPEA (10 mg, 0.076 mmol), and HATU (11 mg, 0.030 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1; DCM:MeOH=20:1, 10:1, 8:1) to obtain a bright yellow solid P53 (7 mg, 63% yield). MS: m/z [M+1]⁺ = 485.32.

### Example 54

### Preparation of Compound P54: 5-(3-Fluoro-[1,3'-diazetidinyl]-1'-yl)-2-methyl-N-(1-(1-methyl-2-oxo-1,2-dihydrobenzo[cd]indol-6-yl)cyclopropyl)ben zamide

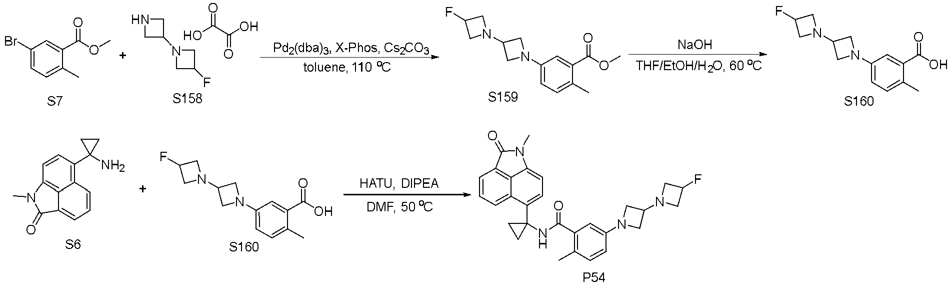

### (1) Preparation of Intermediate S159: methyl 5-(3-fluoro-[1,3'-diazetidinyl]-1'-yl)-2-methylbenzoate

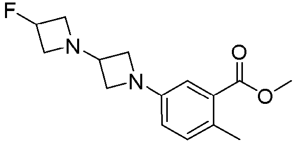

S7 (60 mg, 0.26 mmol) and S158 (68 mg, 0.31 mmol) were placed in a sealed tube. Toluene (2.0 mL), X-Phos (5 mg, 0.010 mmol), Cs₂CO₃ (339 mg, 1.04 mmol), and Pd₂(dba)₃ (5 mg, 0.0052 mmol) were added. The mixture was bubbled with N₂ for 30 seconds, then heated to 110°C and stirred overnight. After the starting materials had completely reacted, the reaction solution was cooled to room temperature. Water (6 mL) was added. The mixture was extracted three times with DCM, and the organic phases were combined. The combined organic phases were concentrated under reduced pressure to remove solvent and then purified by column chromatography (PE:EA=3:1, 1:1) to obtain a colorless oil S159 (42 mg, 58% yield). MS: m/z [M+1]⁺ = 279.21.

### (2) Preparation of Intermediate S160: 5-(3-fluoro-[1,3'-diazetidinyl]-1'-yl)-2-methylbenzoic acid

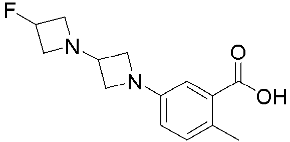

S159 (42 mg, 0.15 mmol) was placed in a single-necked flask. THF (1.0 mL) and EtOH (1.0 mL) were added, followed by the addition of a solution of NaOH (30 mg, 0.75 mmol) in H₂O (1.0 mL). The mixture was heated to 60°C and stirred for 2 hours. After the starting materials had completely reacted, the reaction solution was concentrated under reduced pressure to remove THF and EtOH, and the pH was then adjusted to 4-5 with dilute hydrochloric acid (2 N). The resulting mixture was then concentrated under reduced pressure to remove the solvent and obtain a solid mixture. The obtained solid mixture was dissolved in DCM, dried with anhydrous sodium sulfate, and filtered. The filter cake was washed with DCM. The filtrate was collected and concentrated under reduced pressure to obtain a pale yellow oil S160 (40 mg, 100% yield). MS: m/z [M+1]⁺ = 265.26.

### (3) Preparation of P54: 5-(3-fluoro-[1,3'-diazetidinyl]-1'-yl)-2-methyl-N-(1-(1-methyl-2-oxo-1,2-dihydrobenzo[cd]indol-6-yl)cyclopropyl)benz amide

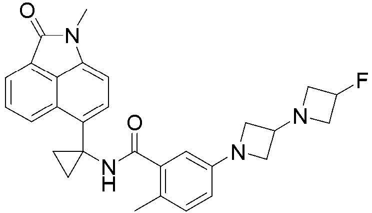

S6 (15 mg, 0.063 mmol) and S160 (20 mg, 0.075 mmol) were placed in a single-necked flask. DMF (1.5 mL), DIPEA (27 mg, 0.21 mmol), and HATU (31 mg, 0.082 mmol) were added. The mixture was heated to 50 °C and stirred for 1.0 hour. After the starting materials had completely reacted, the reaction solution was cooled to room temperature. Water was added, resulting in the precipitation of a yellow solid. The resulting mixture was extracted three times with DCM, and the organic phases were combined. The combined organic phases were concentrated under reduced pressure and then purified by column chromatography (PE:EA=1:1, DCM:MeOH=20:1) to obtain a bright yellow solid P54 (22 mg, 72% yield). MS: m/z [M+1]⁺ = 485.32.

### Test Example 1. Protein level enzyme activity test

Based on the fluorescence resonance energy transfer (FRET) method, an enzymatic assay for SARS-CoV-2 PL^{Pro} was constructed using fluorescence-labeled substrates. This assay was applied to determine the inhibitory activity of compounds against SARS-CoV-2 PL^{Pro}, with the specific steps as follows:
1. Construct a recombinant expression plasmid for the SARS-CoV-2 PL^{Pro} catalytic domain (746-1060). The recombinant expressed SARS-CoV-2 PL^{Pro} catalytic domain protein was purified and used for evaluating the inhibition activity of compounds.
2. Substrate: Z-Arg-Leu-Arg-Gly-Gly-AMC (GLPBIO, product number GA23715).
3. Buffer solution: 50 mM 4-(2-hydroxyethyl)piperazin-1-ethanesulfonic acid (HEPES), 10 mM dithiothreitol (DTT), 0.1 mM ethylene diamine tetraacetic acid (EDTA), pH 7.2.
4. 384-well plate: Greiner, product number 784076.
5. Inhibitor compounds to be tested.
6. Dissolve the compounds in DMSO to prepare stock solutions at a single concentration or at an appropriate concentration gradient.
7. Pre-mix the SARS-CoV-2 PL^{Pro} protein with the compounds to be tested and incubate for 10 minutes.
8. Add 15 µL of the mixed solution containing the SARS-CoV-2 PL^{Pro} protein and the compounds to be tested to each well of the 384-well plate.
9. Prepare 80 µM substrate working solution using the buffer solution described above.
10. Add 5 µL of the 80 µM substrate working solution to each well and incubate for 10 minutes.
11. Detection: BioTek synergy NEO2, Ex: 360 nM / Em: 460 nm.
12. Data Analysis: Analyze the data by nonlinear regression fitting using Prism to calculate the IC₅₀ values, and the results are shown in Table 1.

**Table 1 Inhibitory activity of compounds against papain-like protease**

| Test compound | PLpro IC₅₀ (µM) | Test compound | PLpro IC₅₀ (µM) |
|---|---|---|---|
| Compound P1 | ++ | Compound P28 | +++ |
| Compound P2 | ++ | Compound P29 | +++ |
| Compound P3 | +++ | Compound P30 | +++ |
| Compound P4 | ++ | Compound P31 | ++ |
| Compound P5 | +++ | Compound P32 | +++ |
| Compound P6 | +++ | Compound P33 | + |
| Compound P7 | +++ | Compound P34 | +++ |
| Compound P8 | + | Compound P35 | + |
| Compound P9 | +++ | Compound P36 | +++ |
| Compound P10 | + | Compound P37 | ++ |
| Compound P11 | +++ | Compound P38 | + |
| Compound P12 | ++ | Compound P39 | +++ |
| Compound P13 | + | Compound P40 | +++ |
| Compound P14 | +++ | Compound P41 | +++ |
| Compound P15 | +++ | Compound P42 | +++ |
| Compound P16 | +++ | Compound P43 | +++ |
| Compound P17 | +++ | Compound P44 | + |
| Compound P18 | + | Compound P45 | +++ |
| Compound P19 | + | Compound P46 | + |
| Compound P20 | + | Compound P47 | +++ |
| Compound P21 | +++ | Compound P48 | + |
| Compound P22 | ++ | Compound P49 | +++ |
| Compound P23 | + | Compound P50 | +++ |
| Compound P24 | +++ | Compound P51 | ++ |
| Compound P25 | +++ | Compound P52 | + |
| Compound P26 | +++ | Compound P53 | ++ |
| Compound P27 | +++ | Compound P54 | ++ |

| | | | |
|---|---|---|---|
| Note: +: 0.1µM < IC₅₀≤1µM; ++: 0.04µM < IC₅₀≤0.1µM; +++: IC₅₀≤ 0.04µM | | | |

### Test Example 2. Anti-viral activity evaluation in SARS-CoV-2 infected Vero-E6 cell model

1. Cell line: African green monkey kidney cell Vero E6 (ATCC, CRL-1586).
2. Virus strains: SARS-CoV-2 WT (WT-IQTC02-16#-P2-YQ), XBB.1 (XBB.1-P3-YQ), Omicron BA.5 (GDPCC-303-Omicron-BA.5-P3-YQ) (provided by Guangzhou Customs Technical Center).
3. Positive control: Ensitrelvir (Product Code HY-143216, MedChemExpress LLC).
4. Infection dose: MOI (multiplicity of infection) = 0.01.
5. One day prior to the experiment, add 250 µL of cells to each well of a 48-well plate to achieve approximately 5 × 10⁴ cells per well.
6. Perform gradient dilution (1:3) starting from a maximum concentration of 10µM, with three replicates for each concentration.
7. Add virus strain (MOI=0.01) to each well and infect the cells for 48 hours.
8. After 48 hours, extract the supernatant RNA using the QIAamp Viral RNA Mini Kit (Qiagen). Subsequently, quantify viral copy numbers via quantitative reverse transcription polymerase chain reaction (qRT-PCR). Calculate the half-maximal effective concentration (EC₅₀) based on the absolute viral copy numbers. The results are presented in Table 2.

**Table 2 Inhibitory activity of VeroE6 cells infected by virus strains**

| Test compound | SARS-Cov-2 WT EC₅₀ (µM) | SARS-Cov-2 XBB.1 EC₅₀ (µM) | SARS-Cov-2 Omicron BA.5 EC₅₀ (µM) |
|---|---|---|---|
| Compound P1 | + | + | + |
| Compound P3 | + | + | + |
| Compound P5 | +++ | ++ | + |
| Compound P7 | + | + | + |
| Compound P11 | + | ++ | + |
| Compound P16 | + | ++ | + |
| Compound P17 | +++ | ++ | + |

| | | | |
|---|---|---|---|
| Note: +: 0.1µM≤IC₅₀ ≤1µM; ++: 0.04µM < IC₅₀≤0.1µM; +++: IC₅₀≤0.04µM. | | | |

### Test Example 3. Liver Microsomal Stability Test

1. Preheat PBS (pH 7.4).
2. Preparation of intermediate stock solution for positive reference compound and those to be tested.
2.1 500 µM intermediate stock solution: Add 5 µL of 10 mM compound stock solution in DMSO and positive reference compound to 95 µL of DMSO;
2.2 1.5 µM intermediate stock solution in Liver Microsomes (0.75 mg/mL): Add 1.5 µL of 500 µM intermediate stock solution and 18.75 µL of 20 mg/mL Liver Microsomes to 479.75 µL of PBS.
3. Prepare NADPH stock solution (6 mM) by dissolving NADPH in PBS.
4. Dispense 30 µL of a 1.5 µM intermediate stock solution containing 0.75 mg/mL microsomal solution into the assay plate designated for different time points (0, 5, 15, 30 and 45 minutes).
5. Pre-incubate the plate at 37°C for 5 minutes.
6. For the 0-minute mark, before adding 15 µL of NADPH stock solution (6 mM), add 150 µL of a stop solution (acetonitrile: methanol, 1:1) containing an internal standard to the well.
7. For other time points, add 15 µL of NADPH stock solution (6 mM) to the well to initiate the reaction and start timing.
8. At 5, 15, 30 and 45 minutes, add 150 µL of a stop solution (acetonitrile: methanol, 1:1) containing an internal standard to the wells of the corresponding plate to stop the reaction.
9. After termination, shake the plate for 10 minutes at 600 rpm, and then centrifuge at 4,000 rpm for 15 minutes.
10. Transfer 80 µL of supernatant from each well to a 96-well sample plate containing 140 µL of pure water for LC/MS analysis.

**Table 3: Results of liver microsomal stability test**

| Test compound | Rat t_{1/2} (min) | Rat Clint (mL/min/kg) | Rat Eh (%) | Human t_{1/2} (min) | Human Clint (mL/min/kg) | Human Eh (%) |
|---|---|---|---|---|---|---|
| Compound P1 | 49.4 | 51.0 | 37.3 | 23 | 70 | 77 |
| Compound P3 | 95.3 | 26.2 | 23.6 | 68.3 | 23.5 | 53.2 |

| | | | | | | |
|---|---|---|---|---|---|---|
| Note: Rat represents: rat; Human represents: human; t_{1/2} represents: half-life; Clint represents: clearance rate; Eh represents: clearance proportion. Wherein, the rat liver microsomes are extracted from rat liver, while human liver microsomes are extracted from human-derived hepatocytes. | | | | | | |

### Test Example 4. Pharmacokinetic test

### Routine pharmacokinetic (PK) test in rats:

### 1. The experimental design is presented in the table below:

| Number of animals | Administration dose (mg/kg) | Administration concentration (mg/mL) | Administrati on volume (mL/kg) | Route of administration | Collect samples |
|---|---|---|---|---|---|
| Male ** | | | | | |
| 3 | 5 | 0.5 | 10 | Oral gavage* | Plasma |

| | | | | | |
|---|---|---|---|---|---|
| Note: **: Blood samples were collected from 3 rats at each time point. *: Animals in the oral administration group were fasted overnight (10-14 hours) before administration, and were given food 4 hours after administration. | | | | | |

### 2. Preparation of administration formulation:

Solvent formula: 10% DMSO, 5% ethanol, 5% Cremophor EL and 80% deionized water. Weigh 6.68 mg of the sample to be tested and place it in a suitable container. Measure 1.316 mL of DMSO, vortex for 1 minute, and sonicate for 10 minutes to obtain a 5 mg/mL clear DMSO stock solution.

Take 1 mL of 5 mg/mL DMSO stock solution, add 0.5 mL of ethanol, vortex for 1 minute, add 0.5 mL of Cremophor EL, vortex for 1 minute, and finally add 8 mL of deionized water, vortex for 1 minute to obtain a clear administration formulation with a concentration of 0.5 mg/mL. Prepare it immediately before use and store it at room temperature after preparation.

### 3. Animal selection:

Before the study, all animals suitable for the experiment were weighed. After removing a certain number of animals with high and/or low body weight, the body weight of the animals used for the experiment was within ±20% of the average body weight.

### 4. Animal feeding:

The test animals were housed in rat breeding cages (2-5 animals per cage), with a daily light/dark cycle of approximately 12 hours each. The darkness period could be intermittently interrupted as needed for research-related activities. The environmental temperature and relative humidity in the animal room were monitored daily and controlled within the range of 20-26°C and 40-70%, respectively.

### 5. Route of administration:

Oral gavage administration, with the route of administration consistent with the intended clinical route of administration.

### 6. Observation from the cage side:

During the experiment, all animals were observed at least twice daily at the cage side (which could be accompanied by sampling), with observations including incidence of disease, mortality rate, damage, and food and water supply. Animals in poor health were marked for further observation, and euthanasia was implemented if necessary.

### 7. Biological sample collection:

Oral sampling: There were a total of 8 collection time points, at 0.25, 0.5, 1, 2, 4, 6, 8 and 24 hours after administration.

Plasma: After euthanasia of the animals, blood was drawn by puncture. The samples were placed in tubes containing K2-EDTA and stored on ice until centrifugation. Within 1 hour after blood collection, the samples were centrifuged at 6800 g for 6 minutes at 2-8°C to obtain plasma samples, which were then frozen and stored at approximately -80°C.

### 8. Biological sample analysis

Analyze the compound. While analyzing the sample, utilize quality control samples to evaluate the intra-day accuracy of the analytical batch. Acceptance criteria: At least 50% of the quality control samples at each concentration level, and at least >66.7% of all quality control samples across all concentration levels, should have an accuracy within the range of 80-120% (75%-125% for tissues).

When calculating pharmacokinetic parameters, the BLQ concentration data before the time to reach peak concentration (Tmax) in the oral administration group are calculated as "0", and BLQ concentration data after Tmax are excluded from all calculations.

The non-compartmental model statistical moment method using Phoenix WinNonlin^{®} 7.0 software was employed to calculate the following main pharmacokinetic parameters: Tmax, Cmax, AUC(0-t), T1/2, etc.

### 9. Final disposal of animals

All surviving experimental animals were euthanized after the experiment (using a dedicated euthanasia box for carbon dioxide euthanasia).

### 10. Analytical method

Analytical instrument: LC-MS/MS-12 (TQ5500, Triple quad);
Pipette: Eppendorf
Oscillator: 5810R, Eppendorf
Centrifuges: 420R, 220R, Eppendorf
(If necessary, equipment from other suppliers or of other models can be used)

### Sample processing:

1) Take 30 µL of mixed standard curve samples, quality control samples, blank samples, zero concentration samples, and test samples.
2) For standard curve samples, quality control samples, zero concentration samples and test samples, add 300 µL of internal standard working solution (containing 100 ng/mL IS). For blank samples, ULOQ without IS samples (if necessary), add the same volume of acetonitrile.
3) Vortex mix for 1 minute
4) Centrifuge at 4°C for 7 minutes (18,000g)
5) Take 200 µL of the supernatant to the corresponding wells of a 96-well injection plate.
6) Inject 4µL of sample for LC-MS/MS analysis.

**Table 4 Pharmacokinetic data of compounds after oral administration in vivo**

| Name | T_{1/2} (h) | Tₘₐₓ (h) | Cₘₐₓ (ng/mL) | AUC₍₀₋ₜ₎ (h*ng/mL) | Oral dosage (mg/kg) |
|---|---|---|---|---|---|
| Compound P1 | 3 | 3 | 392.9 | 3389.5 | 5 |

Lastly, it should be noted that the above examples are merely used to illustrate the technical solutions of the present invention and are not intended to limit them. Although the present application has been described in detail with reference to preferred examples, those skilled in the art should understand that modifications can still be made to the specific embodiments of the present application or equivalent substitutions can be made to some technical features, without departing from the spirit of the technical solutions of the present application, which should all be encompassed within the scope of the technical solutions claimed by the present application.

## Claims

1. A compound represented by formula (I), or a stereoisomer thereof, a tautomer thereof, a solvate thereof, an isotopically labeled compound thereof, or a pharmaceutically acceptable salt thereof, 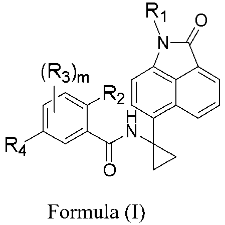 wherein,
R₁ is selected from the group consisting of hydrogen, C₁₋₆ alkyl, C₁₋₆ haloalkyl, -C(=O)-C₁₋₆ alkyl, -C(=O)-C₁₋₆ haloalkyl, -C(=O)-NH-C₁₋₆ alkyl, C₃₋₈ cycloalkyl, and 4-8 membered heterocyclyl;
R₂ is selected from the group consisting of hydrogen, halogen, nitro, -CN, -OH, -COOH, -NH₂, -O-C₁₋₆ alkyl, -NH-C₁₋₆ alkyl, -N-(C₁₋₆ alkyl)₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl, -S(=O)₂NH₂, C₁₋₆ alkyl, and C₁₋₆ haloalkyl;
R₃ is selected from the group consisting of halogen, nitro, -CN, -OH, -COOH, -NH₂, -O-C₁₋₆ alkyl, -NH-C₁₋₆ alkyl, -N-(C₁₋₆ alkyl)₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl, -S(=O)₂NH₂, C₁₋₆ alkyl, and C₁₋₆ haloalkyl;
m is 0, 1, 2, or 3;
R₄ is selected from R₅ and 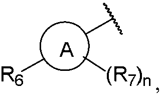 preferably 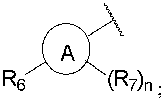
R₅ is selected from C₁₋₈ alkyl and C₁₋₈ haloalkyl, wherein the C₁₋₈ alkyl and C₁₋₈ haloalkyl are optionally substituted with one or more groups selected from the group consisting of -OH, -O-C₁₋₄ alkyl, -NH₂, -NH-C₁₋₄ alkyl, and -N(C₁₋₄ alkyl)₂;
ring A is selected from the group consisting of C₃₋₁₂ cycloalkyl, C₄₋₁₂ cycloalkenyl, 4-12 membered heterocyclyl, C₆₋₁₂ aryl, and 5-12 membered heteroaryl;
R₆ is selected from the group consisting of hydrogen, carbonyl, halogen, nitro, -CN, -OH, -COOH, -NH₂, C₁₋₆ alkyl, -O-C₁₋₆ alkyl, -NH-C₁₋₆ alkyl, -N-(C₁₋₆ alkyl)₂, -S(=O)₂NH₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl, -C(=NH)NH₂, -C(=NH)NHC(=NH)NH, 4-8 membered heterocyclyl, and -NH-(4-8 membered heterocyclyl), wherein the C₁₋₆ alkyl and 4-8 membered heterocyclyl are optionally substituted with one or more groups selected from the group consisting of halogen, nitro, =O, -CN, -OH, -COOH, -NH₂, -C₁₋₆ alkyl, -O-C₁₋₆ alkyl, -NH-C₁₋₆ alkyl, -N-(C₁₋₆ alkyl)₂, -S(=O)₂OH, -S(=O)₂NH₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl, and morpholinyl;
R₇ is selected from the group consisting of halogen, nitro, -CN, -OH, -COOH, -NH₂, -O-C₁₋₆ alkyl, -NH-C₁₋₆ alkyl, -N-(C₁₋₆ alkyl)₂, -S(=O)₂-C₁₋₆ alkyl, -C(=O)-C₁₋₆ alkyl, -C(=O)-NH-C₁₋₆ alkyl, -NH-C(=O)-C₁₋₆ alkyl, -S(=O)₂NH₂, C₁₋₆ alkyl, and C₁₋₆ haloalkyl, or two R₇ form =O;
or, two R₇, together with the connecting atom, form C₃₋₆ cycloalkyl or 4-7 membered heterocyclyl, wherein the C₃₋₆ cycloalkyl or the 4-7 membered heterocyclyl is optionally substituted with 1, 2, or 3 groups selected from -OH and C₁₋₄ alkyl;
or, R₆ and R₇ are joined to form a 4-6 membered heterocyclyl, the 4-6 membered heterocyclyl is optionally substituted with 1, 2, or 3 groups selected from -OH and C₁₋₄ alkyl; and
n is 0, 1, 2, 3, 4, 5, 6, or 7.

2. The compound according to claim 1, wherein R₁ is selected from the group consisting of hydrogen, C₁₋₄ alkyl, C₁₋₄ haloalkyl, -C(=O)-C₁₋₄ alkyl, -C(=O)-C₁₋₄ haloalkyl, C₃₋₆ cycloalkyl, and 4-6 membered heterocyclyl;
preferably, R₁ is selected from hydrogen, C₁₋₄ alkyl and C₃₋₆ cycloalkyl;
preferably, R₁ is selected from hydrogen, methyl, ethyl, n-propyl, isopropyl, cyclobutyl, cyclopentyl and cyclobutyl;
preferably, R₁ is selected from hydrogen, methyl, ethyl, isopropyl and cyclopropyl;
further preferably, R₁ is selected from hydrogen and C₁₋₄ alkyl; and
further preferably, R₁ is selected from hydrogen, methyl, ethyl and isopropyl.

3. The compound according to claim 1 or 2, wherein R₂ is selected from the group consisting of hydrogen, fluorine, chlorine, bromine, -CN, -OH, -COOH, -NH₂, -O-C₁₋₄ alkyl, -NH-C₁₋₄ alkyl, -N-(C₁₋₄ alkyl)₂, C₁₋₄ alkyl, and C₁₋₄ haloalkyl;
preferably, R₂ is selected from hydrogen, fluorine, chlorine, -CN, -OH, -COOH, -NH₂, -O-methyl, -NH-methyl, methyl, ethyl, halogenated methyl, and halogenated ethyl; and
preferably, R₂ is methyl.

4. The compound according to any one of claims 1 to 3, wherein R₃ is selected from the group consisting of halogen, -CN, -OH, -COOH, -NH₂, -O-C₁₋₄ alkyl, -NH-C₁₋₄ alkyl, -N-(C₁₋₄ alkyl)₂, C₁₋₆ alkyl, and C₁₋₆ haloalkyl;
preferably, R₃ is selected from fluorine, chlorine, bromine, -CN, -OH, -COOH, -NH₂, -O-methyl, -NH-methyl, methyl, ethyl, halogenated methyl, and halogenated ethyl; and
preferably, m is 0.

5. The compound according to any one of claims 1 to 4, wherein,
R₅ is selected from C₁₋₆ alkyl and C₁₋₆ haloalkyl, wherein the C₁₋₆ alkyl and C₁₋₆ haloalkyl are optionally substituted with 1, 2, 3, 4, or 5 groups selected from -NH₂, -NH-C₁₋₄ alkyl, and -N(C₁₋₄ alkyl)₂;
preferably, R₅ is selected from C₁₋₆ alkyl, the C₁₋₆ alkyl is optionally substituted with 1, 2, or 3 groups selected from -NH₂, -NH-methyl, and -NH(methyl)₂;
preferably, R₅ is selected from 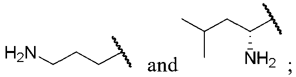
ring A is selected from C₄₋₁₀ cycloalkenyl and 4-10 membered heterocyclyl; preferably, the 4-10 membered heterocyclyl contains 1, 2, or 3 heteroatoms selected from N, O, and S;
preferably, ring A is selected from the group consisting of C₄₋₈ cycloalkenyl, 4-7 membered nitrogen-containing monocyclic heterocyclyl, 7-10 membered nitrogen-containing bridged heterocyclyl, 7-10 membered nitrogen-containing fused heterocyclyl, and 7-10 membered nitrogen-containing spiroheterocyclyl;
preferably, ring A is selected from the group consisting of 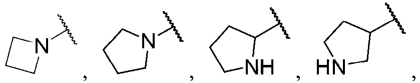 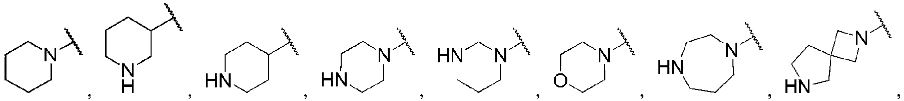 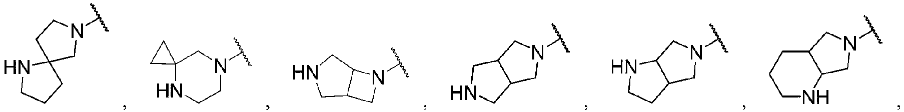 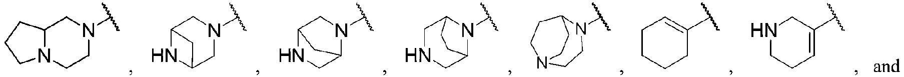 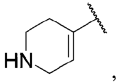 wherein 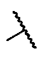 represents the connection site between ring A and phenyl in formula I;
further preferably, ring A is selected from 4-10 membered heterocyclyl;
further preferably, the 4-10 membered heterocyclyl contains 1 or 2 N atoms;
further preferably, ring A is selected from 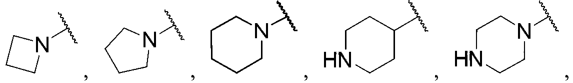 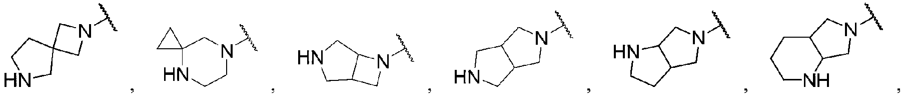 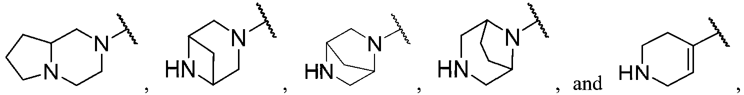 wherein 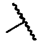 represents the connection site between ring A and phenyl in formula I;
R₆ is selected from the group consisting of hydrogen, carbonyl, halogen, nitro, -CN, -OH, -COOH, -NH₂, C₁₋₄ alkyl, -O-C₁₋₄ alkyl, -NH-C₁₋₄ alkyl, -N-(C₁₋₄ alkyl)₂, -S(=O)₂NH₂, -S(=O)₂-C₁₋₄ alkyl, -C(=O)-C₁₋₄ alkyl, -C(=O)-NH-C₁₋₄ alkyl, -NH-C(=O)-C₁₋₄ alkyl, -C(=NH)NH₂, -C(=NH)NHC(=NH)NH, 4-6 membered heterocyclyl, and -NH-(4-6 membered heterocyclyl), wherein the C₁₋₄ alkyl and 4-6 membered heterocyclyl are optionally substituted with 1, 2, 3, 4, or 5 groups selected from the group consisting of fluorine, chlorine, bromine, =O, -CN, -OH, -COOH, -NH₂, -C₁₋₄ alkyl, -O-C₁₋₄ alkyl, -NH-C₁₋₄ alkyl, -N-(C₁₋₄ alkyl)₂, -S(=O)₂OH, -S(=O)₂NH₂, -S(=O)₂-C₁₋₄ alkyl, and morpholinyl;
preferably, R₆ is selected from the group consisting of hydrogen, fluorine, chlorine, bromine, -CN, -OH, -NH₂, methyl, ethyl, propyl, butyl, -NH-methyl, -NH-ethyl, -NH-propyl, -NH-butyl, -N(methyl)₂, -N(methyl)-ethyl, -N(methyl)-propyl, -N(ethyl)₂, -N(ethyl)-propyl, -S(=O)₂NH₂, -S(=O)₂-methyl, -S(=O)₂-ethyl, -S(=O)₂-propyl, -C(=NH)NH₂, -C(=NH)NHC(=NH)NH₂, azetidinyl, pyrrolidinyl, piperidinyl, piperazinyl, morpholinyl, thiomorpholinyl, -NH-oxetanyl, -NH-tetrahydrofuranyl, and -NH-tetrahydropyranyl, wherein the methyl, ethyl, propyl, butyl, azetidinyl, pyrrolidinyl, piperidinyl, piperazinyl, morpholinyl, thiomorpholinyl, oxetanyl, tetrahydrofuranyl, and tetrahydropyranyl groups are optionally substituted with 1, 2, 3, 4, or 5 groups selected from the group consisting of fluorine, chlorine, bromine, =O, -OH, -COOH, -NH₂, methyl, ethyl, propyl, -O-methyl, -O-ethyl, -NH-methyl, -NH-ethyl, -N(methyl)₂, -N(methyl)-ethyl, -N(ethyl)₂, -S(=O)₂OH, -S(=O)₂NH₂, -S(=O)₂-methyl, and morpholinyl;
preferably, R₆ is selected from the group consisting of hydrogen, fluorine, -CN, -OH, -NH₂, methyl, ethyl, propyl, butyl, 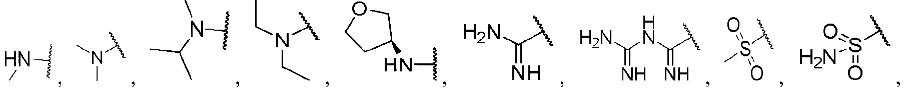 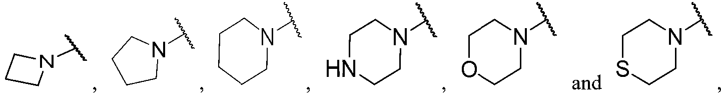 wherein the methyl, ethyl, propyl, butyl, 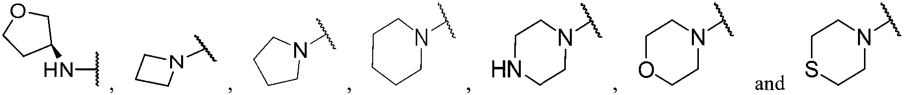 are optionally substituted with 1, 2, or 3 groups selected from the group consisting of fluorine, =O, -OH, -COOH, methyl, -N(CH₃)₂, -S(=O)₂-methyl, and morpholinyl;
preferably, R₆ is selected from the group consisting of hydrogen, fluorine, -CN, -OH, -NH₂, -CH₃, -CH₂CH₃, 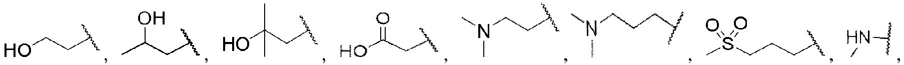 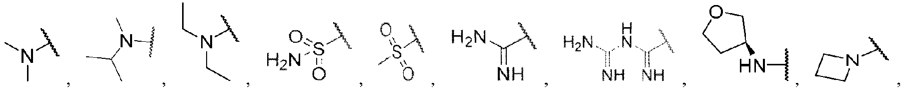 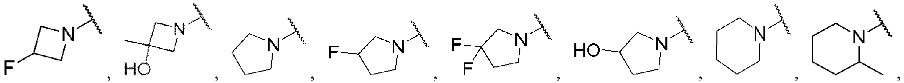 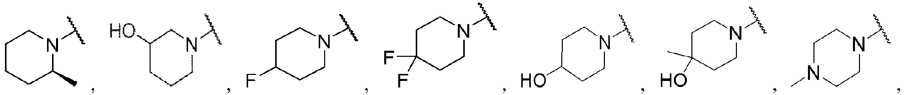 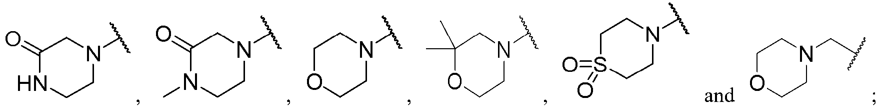
further preferably, R₆ is selected from the group consisting of hydrogen, C₁₋₄ alkyl, -N-(C₁₋₄ alkyl)₂ and 4-6 membered heterocyclyl, wherein the C₁₋₄ alkyl and 4-6 membered heterocyclyl are optionally substituted with 1, 2, or 3 groups selected from halogen, =O, -CN, -OH, -COOH, -NH₂, -N-(C₁₋₄ alkyl)₂, -S(=O)₂-methyl, C₁₋₄ alkyl and morpholinyl;
further preferably, R₆ is selected from the group consisting of hydrogen, C₁₋₄ alkyl, -N-(C₁₋₄ alkyl)₂ and 4-6 membered heterocyclyl, wherein the 4-6 membered heterocyclyl contains 1 or 2 heteroatoms selected from N, O and S, the C₁₋₄ alkyl and 4-6 membered heterocyclyl are optionally substituted with 1, 2, or 3 groups selected from the group consisting of fluorine, =O, -OH, -N(CH₃)₂, -S(=O)₂-CH₃, -CH₃ and morpholinyl;
further preferably, R₆ is selected from the group consisting of hydrogen, -CH₃, -CH₂CH₃, 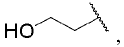 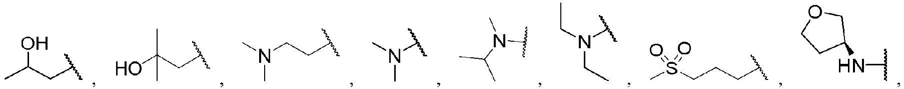 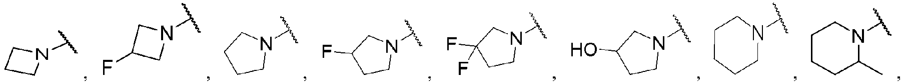 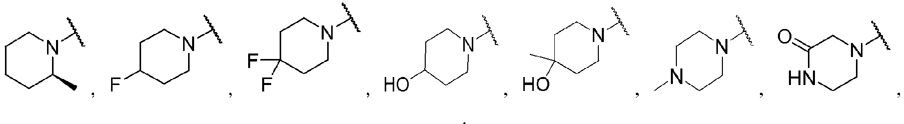 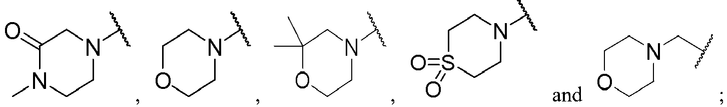
R₇ is independently selected from the group consisting of halogen, nitro, -CN, -OH, -COOH, -NH₂, -O-C₁₋₄ alkyl, -NH-C₁₋₄ alkyl, -N-(C₁₋₄ alkyl)₂, -C(=O)-C₁₋₄ alkyl, -C(=O)-NH-C₁₋₄ alkyl, -NH-C(=O)-C₁₋₄ alkyl, C₁₋₆ alkyl, and C₁₋₄ haloalkyl;
preferably, each R₇ is independently selected from the group consisting of fluorine, chlorine, bromine, -CN, -OH, -COOH, -NH₂, -O-methyl, -O-ethyl, -O-propyl, -NH-methyl, -NH-ethyl, -NH-propyl, -N(methyl)₂, -N(methyl)-ethyl, -N(methyl)-propyl, -N(ethyl)₂, methyl, ethyl, propyl, halogenated methyl, halogenated ethyl, and halogenated propyl;
preferably, each R₇ is independently selected from the group consisting of fluorine, -OH, -NH₂, -COOH, and methyl;
further preferably, each R₇ is independently selected from fluorine and methyl;
or, two R₇ form =O.

6. The compound according to any one of claims 1 to 5, wherein the compound is selected from the group consisting of compounds represented by formula (I-1), or a stereoisomer thereof, a tautomer thereof, a solvate thereof, an isotopically labeled compound thereof, or a pharmaceutically acceptable salt thereof, 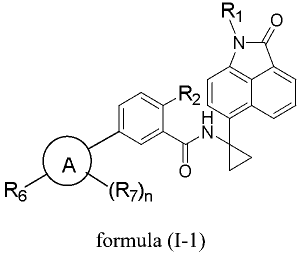
wherein, R₁, R₂, ring A, R₆, R₇ and n are defined as described in any one of claims 1 to 5;
1) preferably, the compound is selected from compounds represented by formula (I-1A), or a stereoisomer thereof, a tautomer thereof, a solvate thereof, an isotopically labeled compound thereof, or a pharmaceutically acceptable salt thereof, 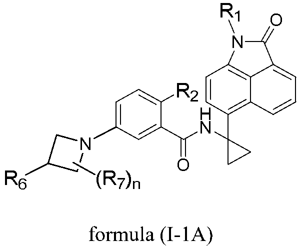
wherein, R₁, R₂, R₆, R₇ and n are defined as described in any one of claims 1 to 5;
preferably, R₁ is selected from methyl and ethyl;
preferably, R₆ is selected from the group consisting of -NH-C₁₋₄ alkyl, -N-(C₁₋₄ alkyl)₂, 4-6 membered heterocyclyl and -NH-(4-6 membered heterocyclyl), wherein the -NH-C₁₋₄ alkyl, -N-(C₁₋₄ alkyl)₂, 4-6 membered heterocyclyl and -NH-(4-6 membered heterocyclyl) are optionally substituted with 1, 2, or 3 groups selected from the group consisting of fluorine, chlorine, =O, -OH, -NH₂, methyl and -N(CH₃)₂;
preferably, R₆ is selected from the group consisting of -NH-methyl, -NH-ethyl, -NH-propyl, -NH-butyl, -N(methyl)₂, -N(methyl)-ethyl, -N(methyl)-propyl, -N(ethyl)₂, -N(ethyl)-propyl, azetidinyl, pyrrolidinyl, piperidinyl, piperazinyl, morpholinyl, thiomorpholinyl, -NH-oxetanyl, -NH-tetrahydrofuranyl and -NH-tetrahydropyranyl, wherein the methyl, ethyl, propyl, butyl, azetidinyl, pyrrolidinyl, piperidinyl, piperazinyl, morpholinyl, thiomorpholinyl, oxetanyl, tetrahydrofuranyl, and tetrahydropyranyl are optionally substituted with 1, 2, or 3 groups selected from the group consisting of fluorine, chlorine, =O, -OH, -NH₂ and methyl;
preferably, R₆ is selected from the group consisting of 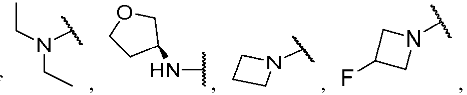 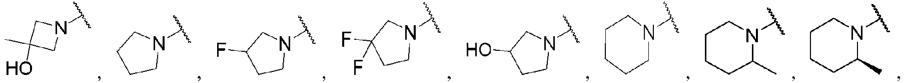 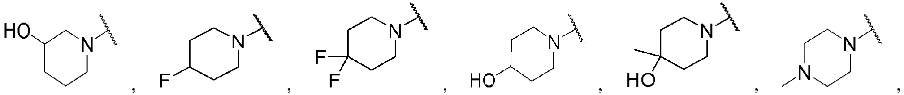 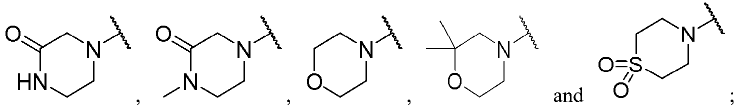
or, R₆ and R₇ are joined to form a pyrrolidinyl, wherein the pyrrolidinyl is optionally substituted with one methyl;
preferably, n is 0, 1, 2, or 3;
further preferably, R₆ is selected from 4-6 membered heterocyclyl, wherein the 4-6 membered heterocyclyl are optionally substituted with 1, 2, or 3 groups selected from the group consisting of halogen, =O, -OH and C₁₋₄ alkyl;
further preferably, R₆ is selected from 4-6 membered heterocyclyl, wherein the 4-6 membered heterocyclyl are monocyclic ring, the 4-6 membered heterocyclyl contain 1 or 2 heteroatoms selected from N, O and S, and the 4-6 membered heterocyclyl are optionally substituted with 1, 2, or 3 groups selected from the group consisting of fluorine, =O, -OH and methyl;
further preferably, R₆ is selected from the group consisting of 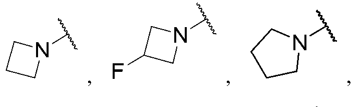 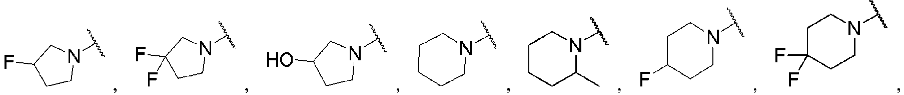 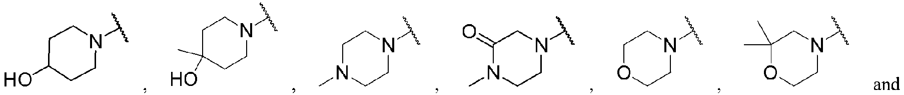 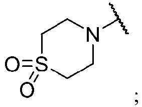
preferably, the compound is selected from compounds represented by formula (I-1A-1), or a stereoisomer thereof, a tautomer thereof, a solvate thereof, an isotopically labeled compound thereof, or a pharmaceutically acceptable salt thereof, 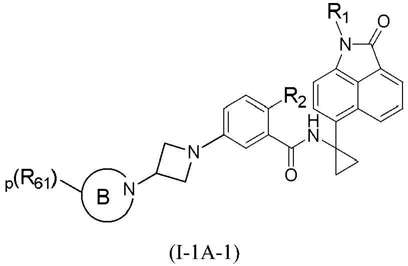
wherein,
R₁ and R₂ are defined as described in any one of claims 1 to 5;
ring B is selected from 4-6 membered heterocyclyl,
R₆₁ is selected from the group consisting of halogen, =O, -CN, -OH, -COOH, -NH₂, -C₁₋₄ alkyl, -NH-C₁₋₄ alkyl and -N-(C₁₋₄ alkyl)₂,
p is 0, 1, 2, or 3;
preferably, R₁ is selected from methyl and ethyl;
preferably, ring B is selected from the group consisting of azetidinyl, pyrrolidinyl, piperidinyl, piperazinyl, morpholinyl and thiomorpholinyl;
preferably, R₆₁ is selected from the group consisting of fluorine, chlorine, bromine, =O, -CN, -OH, -COOH, -NH₂, methyl, ethyl and propyl, preferably, R₆₁ is selected from fluorine, =O, -OH and methyl;
preferably, the compound is selected from compounds represented by formula (I-1A-2), or a stereoisomer thereof, a tautomer thereof, a solvate thereof, an isotopically labeled compound thereof, or a pharmaceutically acceptable salt thereof, 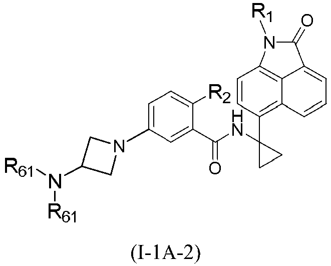
wherein,
R₁ and R₂ are defined as described in any one of claims 1 to 5,
R₆₁ is selected from the group consisting of hydrogen, C₁₋₄ alkyl and 4-6 membered heterocyclyl;
preferably, R₁ is methyl;
preferably, R₆₁ is selected from the group consisting of hydrogen, methyl, ethyl, propyl, butyl, oxetanyl, tetrahydrofuranyl and tetrahydropyranyl; preferably, R₆₁ is selected from hydrogen, -CH₂CH₃ and 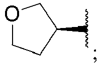
2) or preferably, the compound is selected from compounds represented by formula (I-1B), or a stereoisomer thereof, a tautomer thereof, a solvate thereof, an isotopically labeled compound thereof, or a pharmaceutically acceptable salt thereof, 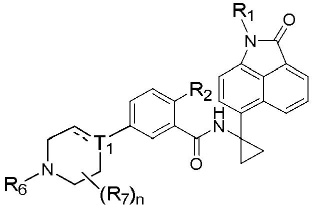 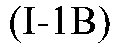
wherein,
T₁ is C, CH or N;
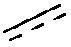 is a single bond or a double bond;
R₁, R₂, R₆, R₇ and n are defined as described in any one of claims 1 to 5;
preferably, T₁ is N, 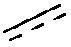 is a single bond;
preferably, R₆ is selected from the group consisting of hydrogen, -CN, -NH₂, C₁₋₄ alkyl, -C(=NH)NH₂, -C(=NH)NHC(=NH)NH, -S(=O)₂-C₁₋₄ alkyl and -S(=O)₂NH₂, wherein the C₁₋₄ alkyl is optionally substituted with 1, 2, or 3 groups selected from the group consisting of halogen, -OH, -COOH, methyl, -NH(CH₃), -N(CH₃)₂, -N(CH₃)(CH₂CH₃), -N(CH₂CH₃)₂ and -S(=O)₂-CH₃;
preferably, R₆ is selected from the group consisting of hydrogen, -CN, -NH₂, methyl, ethyl, propyl, butyl, -C(=NH)NH₂, -C(=NH)NHC(=NH)NH, -S(=O)₂-C₁₋₄ alkyl and -S(=O)₂NH₂, wherein the methyl, ethyl, propyl, and butyl groups are optionally substituted with 1, 2, or 3 groups selected from the group consisting of fluorine, -OH, -COOH, methyl, -N(CH₃)₂ and -S(=O)₂-CH₃;
preferably, R₆ is selected from the group consisting of hydrogen, -CN, -NH₂, -CH₃, -CH₂CH₃, 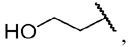 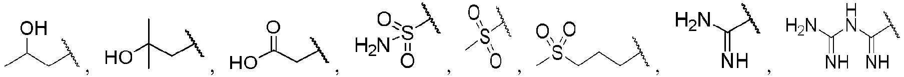 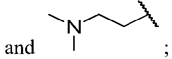
preferably, each R₇ is independently selected from -COOH and C₁₋₄ alkyl;
preferably, each R₇ is independently selected from -COOH and methyl;
or, two R₇ form =O;
or, two R₇, together with the connected atoms, form cyclopropyl, azetidinyl, pyrrolidinyl, piperidinyl, or homopiperidinyl,
or, R₆ and R₇ are joined to form a pyrrolidinyl, wherein the pyrrolidinyl is optionally substituted with one OH; preferably, n is 0, 1, 2, 3, or 4;
further preferably, R₆ is selected from hydrogen and C₁₋₄ alkyl, wherein the C₁₋₄ alkyl is optionally substituted with 1, 2, or 3 groups selected from the group consisting of -OH, -NH(CH₃), -N(CH₃)₂ and -S(=O)₂-CH₃;
further preferably, R₆ is selected from the group consisting of hydrogen, -CH₃, -CH₂CH₃, 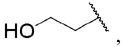 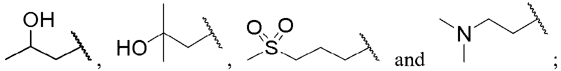
further preferably, each R₇ is independently selected from C₁₋₄ alkyl;
further preferably, each R₇ is an independent methyl;
or, two R₇, together with the connected atoms, form cyclopropyl, azetidinyl, pyrrolidinyl, piperidinyl, or homopiperidinyl,
or, R₆ and R₇ are joined to form a pyrrolidinyl; and
further preferably, n is 0, 1, or 2;
3) or preferably, the compound is selected from compounds represented by formula (I-1C), or a stereoisomer thereof, a tautomer thereof, a solvate thereof, an isotopically labeled compound thereof, or a pharmaceutically acceptable salt thereof, 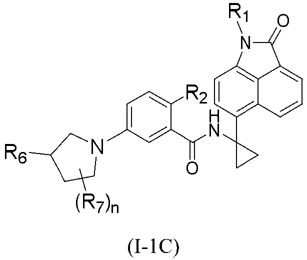
wherein,
R₁, R₂, R₆, R₇ and n are defined as described in any one of claims 1 to 5;
preferably, R₆ is selected from the group consisting of hydrogen, C₁₋₄ alkyl, -N-(C₁₋₄ alkyl)₂ and 4-6 membered heterocyclyl, wherein the C₁₋₄ alkyl and 4-6 membered heterocyclyl are optionally substituted with 1, 2, or 3 groups selected from halogen, -OH, -COOH and methyl;
preferably, R₆ is selected from the group consisting of hydrogen, -CH₃, 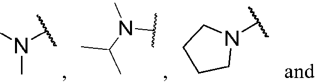 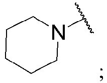
or, R₆ and R₇ are joined to form a 5-6 membered heterocyclyl, wherein the 5-6 membered heterocyclyl is optionally substituted with one OH or methyl, preferably, R₆ and R₇ are joined to form a pyrrolidinyl or a piperidinyl, wherein the pyrrolidinyl or piperidinyl is optionally substituted with one methyl; and
preferably, n is 0, 1, or 2.

7. The compound according to any one of claims 1 to 6, wherein the compound is selected from the group consisting of:
| | | |
|---|---|---|
| 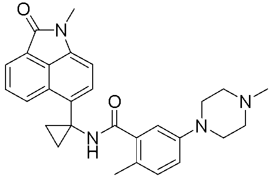 | 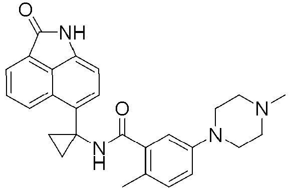 | 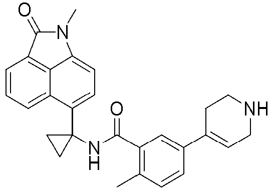 |
| P1 | P2 | P3 |
| 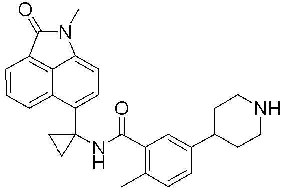 | 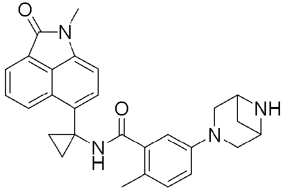 | 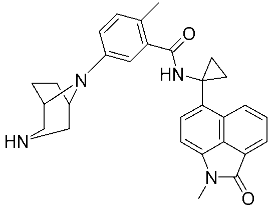 |
| P4 | P5 | P6 |
| 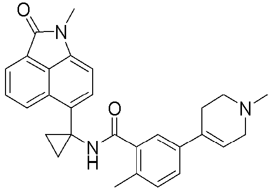 | 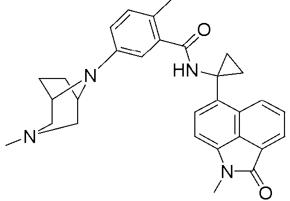 | 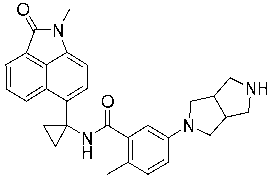 |
| P7 | P8 | P9 |
| 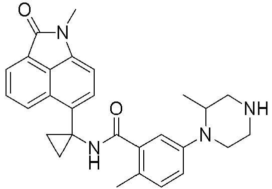 | 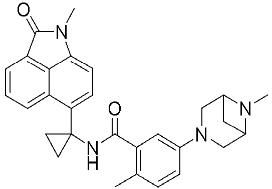 | 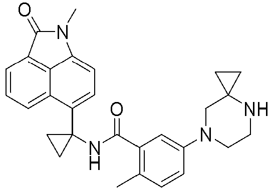 |
| P10 | P11 | P12 |
| 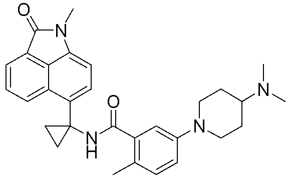 | 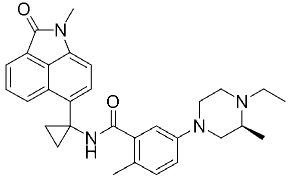 | 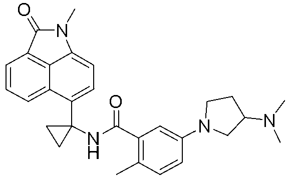 |
| P13 | P14 | P15 |
| 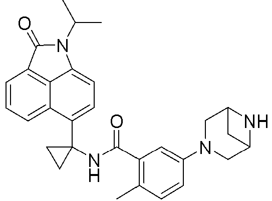 | 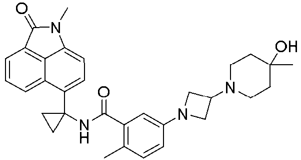 | 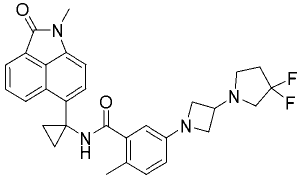 |
| P16 | P17 | P18 |
| 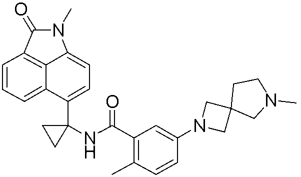 | 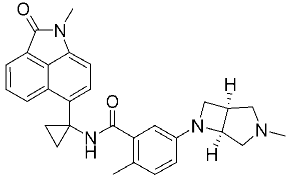 | 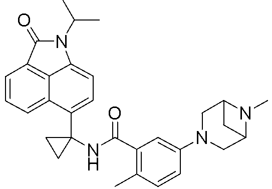 |
| P19 | P20 | P21 |
| 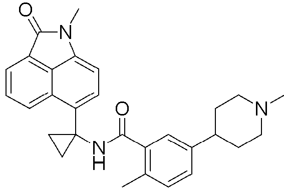 | 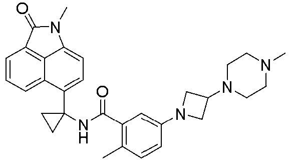 | 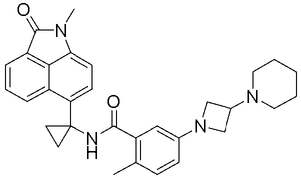 |
| P22 | P23 | P24 |
| 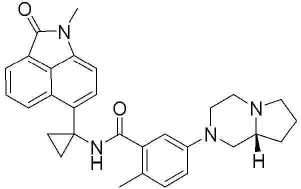 | 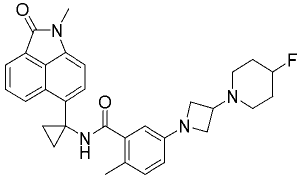 | 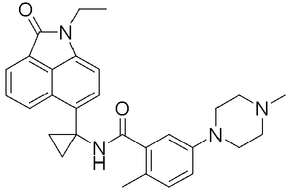 |
| P25 | P26 | P27 |
| 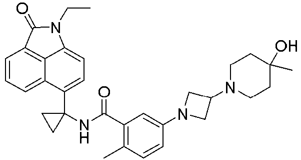 | 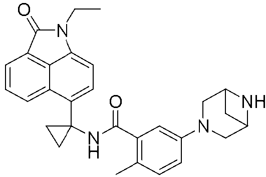 | 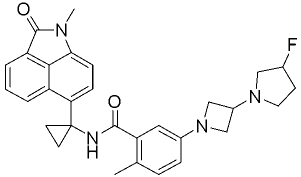 |
| P28 | P29 | P30 |
| 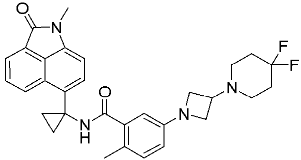 | 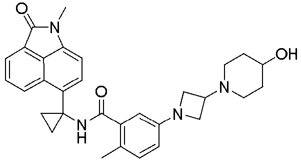 | 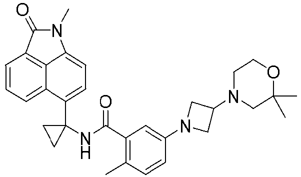 |
| P31 | P32 | P33 |
| 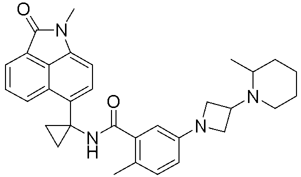 | 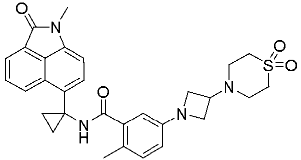 | 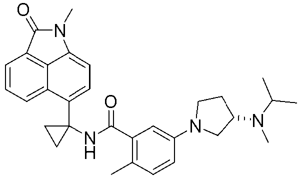 |
| P34 | P35 | P36 |
| 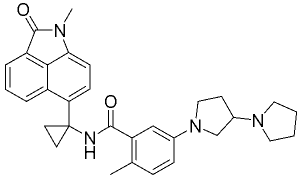 | 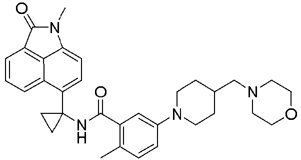 | 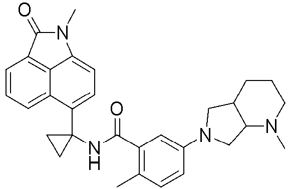 |
| P37 | P38 | P39 |
| 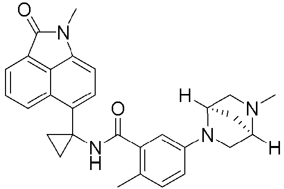 | 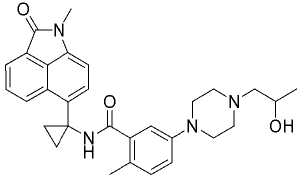 | 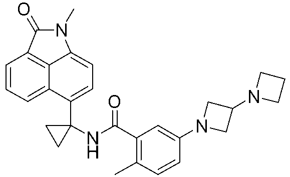 |
| P40 | P41 | P42 |
| 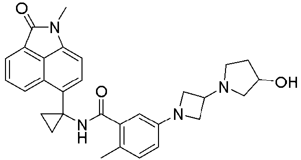 | 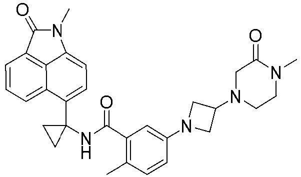 | 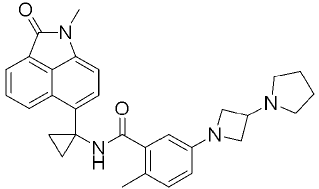 |
| P43 | P44 | P45 |
| 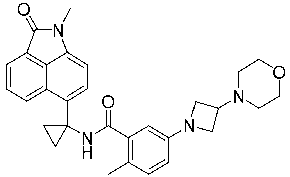 | 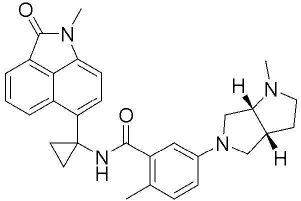 | 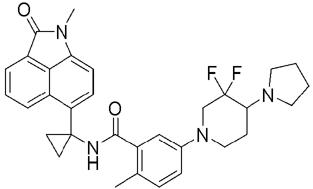 |
| P46 | P47 | P48 |
| 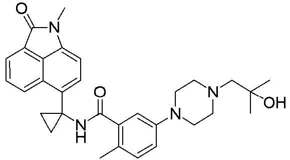 | 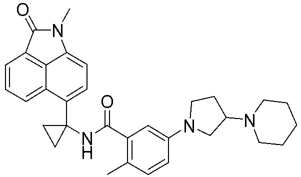 | 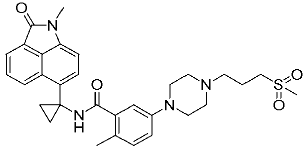 |
| P49 | P50 | P51 |
| 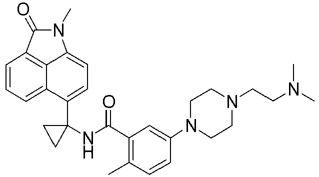 | 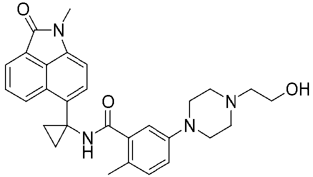 | 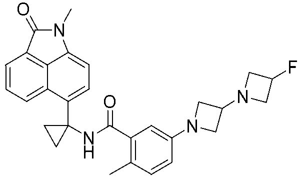 |
| P52 | P53 | P54 |
| 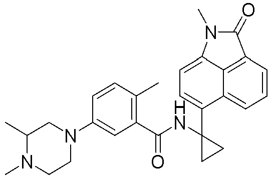 | 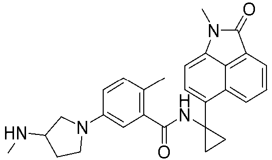 | 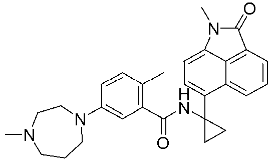 |
| P55 | P56 | P57 |
| 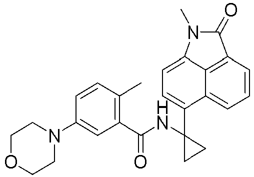 | 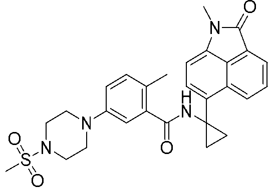 | 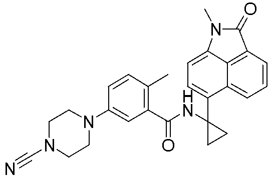 |
| P58 | P59 | P60 |
| 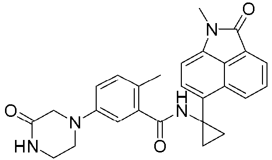 | 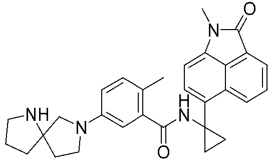 | 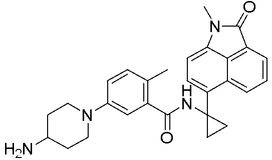 |
| P61 | P62 | P63 |
| 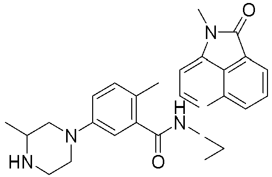 | 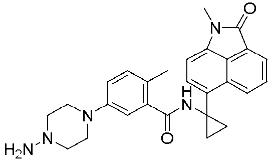 | 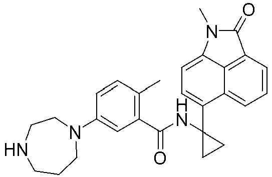 |
| P64 | P65 | P66 |
| 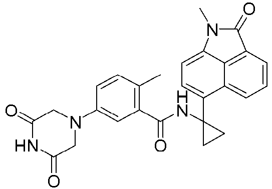 | 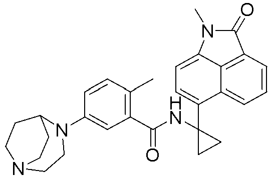 | 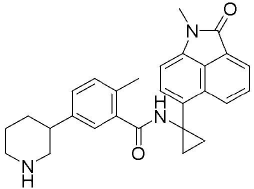 |
| P67 | P68 | P69 |
| 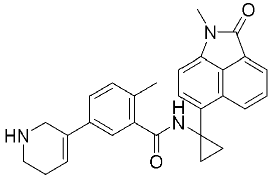 | 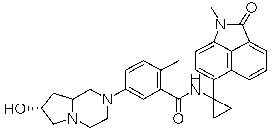 | 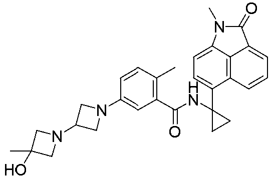 |
| P70 | P71 | P72 |
| 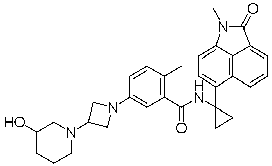 | 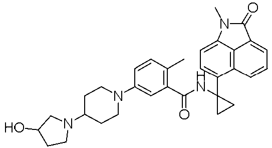 | 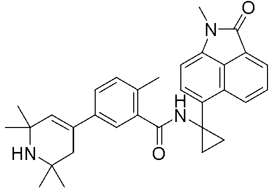 |
| P73 | P74 | P75 |
| 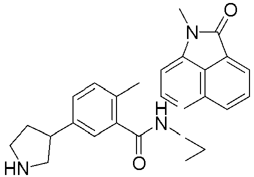 | 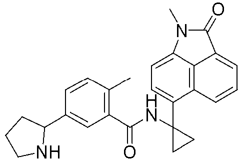 | 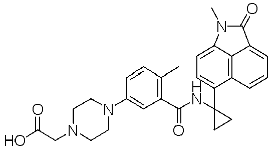 |
| P76 | P77 | P78 |
| 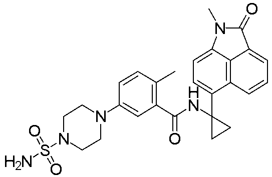 | 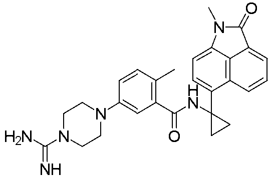 | 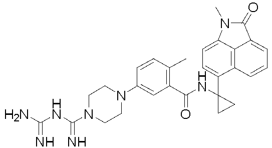 |
| P79 | P80 | P81 |
| 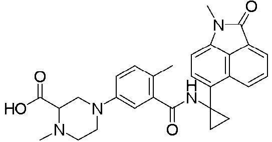 | 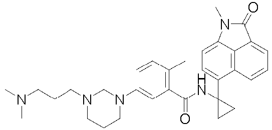 | 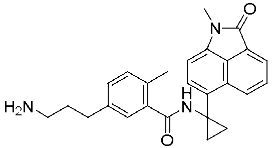 |
| P82 | P83 | P84 |
| 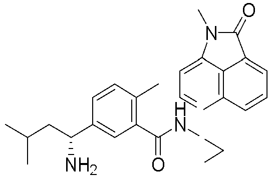 | 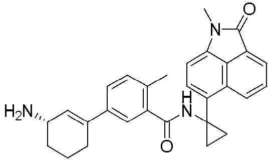 | 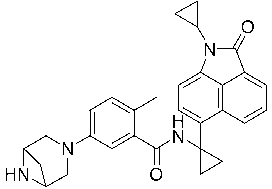 |
| P85 | P86 | P87 |
| 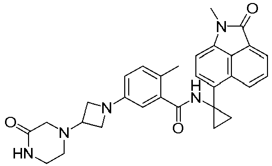 | 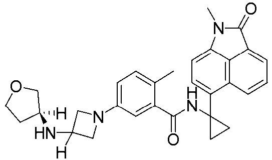 | 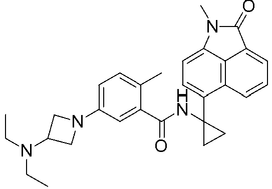 |
| P88 | P89 | P90 |

8. A pharmaceutical composition, comprising at least one compound according to any one of claims 1 to 7, and one or more pharmaceutical carriers and/or excipients.

9. Use of the compound according to any one of claims 1 to 7, or the pharmaceutical composition according to claim 8, in the preparation of a medicament for the treatment and/or prevention of a disease or condition, or for the alleviation of the severity of the disease or condition, wherein the disease or condition is caused by a coronavirus, and wherein the medicament comprises a human medicament and a veterinary medicament.

10. The use according to claim 9, wherein the disease or condition is selected from the group consisting of respiratory diseases (include simple infections like fever, cough, and sore throat, pneumonia, acute respiratory infection, severe acute respiratory infection (SARI), hypoxic respiratory failure and acute respiratory distress syndrome, COVID-19, severe acute respiratory syndrome (SARS), Middle East respiratory syndrome (MERS)), sepsis, septic shock and feline infectious peritonitis.
